# EUROPEAN PATENT APPLICATION

(11) **EP 4 023 245 A1**
(43) Date of publication of application: **06.07.2022**
(21) Application number: 20856636.4
(22) Date of filing: 13.05.2020
(51) Int. Cl.: A61K 39/395, A61P 35/00, A61P 43/00, C07K 16/46, A61K 47/26, C12N 1/21, C12N 15/13, A61K 31/7016, A61K 35/745

(54) **BIFIDOBACTERIUM SPP. EXPRESSING AND SECRETING DIABODY-TYPE BSAB**

(30) Priority: 28.08.2019 JP 2019155792; 17.02.2020 JP 2020023955
(71) Applicant: Azusapharma Sciences, Inc., Seattle, WA 98109 (US); Tohoku University, Sendai-shi, Miyagi 980-8577 (JP)
(72) Inventor: SHIMATANI, Yuko, Matsumoto-shi, Nagano 390-8621 (JP); KOBAYASHI, Satoshi, Matsumoto-shi, Nagano 390-8621 (JP); SHIOYA, Koichiro, Matsumoto-shi, Nagano 390-8621 (JP); KATAOKA, Shiro, Matsumoto-shi, Nagano 390-8621 (JP); SEKI, Yuji, Matsumoto-shi, Nagano 390-8621 (JP); MATSUMURA, Tomio, Matsumoto-shi, Nagano 390-8621 (JP); KANARI, Yasuyoshi, Matsumoto-shi, Nagano 390-8621 (JP); UMETSU, Mitsuo, Sendai-shi, Miyagi 980-8577 (JP); NAKAZAWA, Hikaru, Sendai-shi, Miyagi 980-8577 (JP)
(74) Representative: D Young & Co LLP
(86) International application number: PCT/JP2020/019075
(87) International publication number: WO 2021/038975

(57) **Abstract**

An object of the present invention is to provide a bacterium of the genus *Bifidobacterium* exerting an anti-tumor effect against solid cancers sustainably and specifically. Produced is a bacterium of the genus *Bifidobacterium* expressing a first polypeptide comprising a light-chain variable region (VL) that binds to human CD3 and a heavy-chain variable region (VH) that binds to a human tumor cell surface antigen; and a second polypeptide comprising VL that binds to a human tumor cell surface antigen and VH that binds to human CD3 and secreting a diabody-type bispecific antibody composed of the first polypeptide and the second polypeptide.

## Description

### Technical Field

The present invention relates to a bacterium of the genus *Bifidobacterium* (which may be hereinafter referred to as *Bifidobacterium* or B bacterium) expressing/secreting a diabody-type bispecific antibody (BsAb: bispecific monoclonal antibody), which is useful for immunotherapy of solid tumors, and to pharmaceutical uses thereof.

### Background Art

The BsAb (which may be referred to as bifunctional antibody) is an artificial divalent antibody that binds to two different antigens and is thus different from a natural divalent antibody that binds to the same antigens. Among BsAbs, an antibody that binds to an antigen (tumor marker) on a tumor cell and CD3, which is an antigen (T cell marker) on a T cell (i.e., T-BsAb), activates the T cell into a cytotoxic T cell by crosslinking the tumor cell with the T cell, to exert an anti-tumor effect. Since the mid-1980s, many T-BsAbs have been researched and developed as anti-tumor agents. For example, catumaxomab is a full-length T-BsAb that binds to EpCAM, which is a tumor marker, and CD3, which is a T cell marker, and contains a constant region and is approved as an antibody drug for treating malignant ascites. Further, blinatumomab is a low-molecular weight T-BsAb that binds to CD19, which is a tumor marker, and CD3, which is a T cell marker, and contains no constant regions and is approved as an antibody drug for treating acute lymphoblastic leukemia (ALL).

When a T-BsAb is systemically administered to treat a solid tumor, there are still problems to be solved. For example, it has been pointed out that full-length T-BsAbs have a large molecular size and therefore have a limited permeability into tissues of solid tumors, and may cause cytokine release syndrome (CRS) even if the dose is reduced. Meanwhile, low-molecular weight T-BsAb have a problem in stability and a disadvantage of having a short half-life in blood (Non-patent Document 1).

A diabody-type BsAb, which is one of low-molecular weight BsAbs, consists of a first polypeptide having a light-chain variable region (VL) derived from a first antibody (VL1) and a heavy-chain variable region (VH) derived from a second antibody (VH2) on the same peptide chain and a second polypeptide having VL derived from the second antibody (VL2) and VH derived from the first antibody (VH1) on the same polypeptide chain. As a method for producing a diabody-type BsAb, a method using Escherichia coli is known (Patent Document 1 and Non-patent Document 2) . Further, many reports say that a diabody-type T-BsAb that binds to EGFR (Epidermal Growth Factor Receptor), which is a tumor cell surface antigen, and CD3, which is a T cell marker, has an anti-tumor activity (Patent Documents 2 to 4 and Non-patent Document 3).

Meanwhile, attention has been given to a method using anaerobic bacteria such as B bacteria as a gene transport carrier in treatment of solid tumors. When systemically administered, B bacteria specifically engraft with hypoxic sites such as solid tumors. Therefore, when B bacteria transformed with a gene encoding a protein having an activity of converting an anti-tumor protein or an anti-tumor substance precursor into an anti-tumor substance are systemically administered, the gene is specifically expressed locally in the tumor. Therefore, such systemic administration enables a drug having unavoidable side effects to be delivered at high concentration sustainably and locally to the tumor. Currently, clinical trials are underway in the United States for B bacteria expressing cytosine deaminase, which is an enzyme that converts 5-fluorocytosine (5-FC) into 5-fluorouracil (5-FU) (Patent Documents 5 to 10).

### Prior Art Documents

### Patent Documents

Patent Document 1: U.S. Patent No. 6492123
Patent Document 2: Japanese Patent No. 3803790
Patent Document 3: International Publication No. WO 2010/109924
Patent Document 4: International Publication No. WO 2015/146438
Patent Document 5: Japanese Patent No. 3642755
Patent Document 6: International Publication No. WO 2009/128272
Patent Document 7: International Publication No. WO 2009/128275
Patent Document 8: International Publication No. WO 2011/093465
Patent Document 9: International Publication No. WO 2015/166640
Patent Document 10: International Publication No. WO 2016/088376

### Non-patent Documents

Non-patent Document 1: Nat Rev Drug Discov. 2019 Jun 7.doi:10.1038/s41573-019-0028-1
Non-patent Document 2: Proc Natl Acad Sci U S A. 1993 Jul15;90 (14):6444-8
Non-patent Document 3: Sci Rep. 2017 Jun 6;7 (1):2862. doi:10.1038/s41598-017-03101-4

### Summary of the Invention

### Object to be Solved by the Invention

It is an object of the present invention to provide a bacterium of the genus *Bifidobacterium* that exerts an anti-tumor effect sustainably and specifically against solid cancers, and an agent for preventing or treating solid tumors containing the bacterium of the genus Bifidobacterium.

### Means to Solve the Object

As a result of dedicated studies in order to solve the aforementioned problems, the present inventors have found that a *Bifidobacterium* expressing/secreting a diabody-type BsAb that binds to both human CD3 and a human tumor cell surface antigen has a cytotoxic activity against solid cancers such as colorectal cancer, colon cancer, and cholangiocarcinoma, and an anti-tumor effect against solid tumors. Further, they have confirmed that the cytotoxic activity and the anti-tumor effect are enhanced by substituting glutamine (Q) at Kabat position 38 in the light-chain variable region (VL) that binds to human CD3 with glutamic acid (E), substituting Q at Kabat position 39 in the heavy-chain variable region (VH) that binds to the human tumor cell surface antigen with E, substituting Q at Kabat position 38 in the VL that binds to a human tumor cell surface antigen with lysine (K), and substituting Q at Kabat position 39 in the VH that binds to human CD3 with K, in the diabody-type BsAb. The present invention has been accomplished based on these findings.

That is, the present invention is as follows.
[1] A bacterium of the genus *Bifidobacterium* expressing: a first polypeptide comprising a light-chain variable region (VL) that binds to human CD3 and a heavy-chain variable region (VH) that binds to a human tumor cell surface antigen; and a second polypeptide comprising VL that binds to a human tumor cell surface antigen and VH that binds to human CD3, and secreting a diabody-type bispecific antibody composed of the first polypeptide and the second polypeptide.
[2] The bacterium of the genus *Bifidobacterium* according to [1] above, wherein the first polypeptide contains the VL that binds to human CD3 and the VH that binds to the human tumor cell surface antigen in this order from the amino terminus to the carboxyl terminus, and the second polypeptide contains the VL that binds to the human tumor cell surface antigen and the VH that binds to human CD3 in this order from the amino terminus to the carboxyl terminus.
[3] The bacterium of the genus *Bifidobacterium* according to [1] or [2] above, wherein a linker peptide each consisting of 4 to 9 amino acids is connected between the VL and the VH of the first polypeptide and between the VL and the VH of the second polypeptide, respectively.
[4] The bacterium of the genus *Bifidobacterium* according to any one of [1] to [3] above, wherein a secretion signal peptide-linker peptide conjugate containing any of amino acid sequences (i) to (iii) below is connected to the amino terminus of each of the first polypeptide and the second polypeptide:
   (i) an amino acid sequence having a sequence identity of at least 80% with the amino acid sequence shown in SEQ ID No: 10;
   (ii) an amino acid sequence having a sequence identity of at least 80% with the amino acid sequence shown in SEQ ID No: 13; and
   (iii) an amino acid sequence having a sequence identity of at least 80% with the amino acid sequence shown in SEQ ID No: 18.
[5] The bacterium of the genus *Bifidobacterium* according to any one of [1] to [4] above, wherein
   the amino acid residue at Kabat position 38 in the VL of the first polypeptide is glutamic acid, the amino acid residue at Kabat position 39 in the VH of the first polypeptide is glutamic acid, the amino acid residue at Kabat position 38 in the VL of the second polypeptide is lysine, and the amino acid residue at Kabat position 39 in the VH of the second polypeptide is lysine; or
   the amino acid residue at Kabat position 38 in the VL of the first polypeptide is lysine, the amino acid residue at Kabat position 39 in the VH of the first polypeptide is lysine, the amino acid residue at Kabat position 38 in the VL of the second polypeptide is glutamic acid, and the amino acid residue at Kabat position 39 in the VH of the second polypeptide is glutamic acid.
[6] The bacterium of the genus *Bifidobacterium* according to any one of [1] to [5] above, wherein the human tumor cell surface antigen is an antigen belonging to a human EGFR (Epidermal Growth Factor Receptor) family, a human EpCAM (Epithelial cell adhesion molecule), or a human PSMA (Prostate Specific Membrane Antigen).
[7] The bacterium of the genus *Bifidobacterium* according to any one of [1] to [6] above, wherein the antigen belonging to a human EGFR family is a human EGFR, a human HER2, or a human HER3.
[8] The bacterium of the genus *Bifidobacterium* according to any one of [1] to [7] above, wherein the first polypeptide and the second polypeptide are expressed polycistronically.
[9] The bacterium of the genus *Bifidobacterium* according to any one of [1] to [8] above, wherein the bacterium is *Bifidobacterium longum.*
[10] An agent for preventing or treating solid tumors containing the bacterium of the genus *Bifidobacterium* according to any one of [1] to [9] above as an active component.
[11] The agent according to [10] above, to be used in combination with an agent for promoting engraftment and/or growth of bacterium of the genus *Bifidobacterium* in solid tumors.
[12] The agent according to [11] above, wherein the agent for promoting engraftment and/or growth of bacterium of the genus *Bifidobacterium* in solid tumors is maltose.

Further, examples of other embodiments of the present invention can include: a method for preventing or treating solid tumors, comprising a step of administering an agent for preventing or treating solid tumors containing the bacterium of the genus *Bifidobacterium* of the present invention as an active component to a subject in need of prevention or treatment of solid tumors; the bacterium of the genus *Bifidobacterium* of the present invention to be used as an agent for preventing or treating solid tumors; the bacterium of the genus *Bifidobacterium* of the present invention for use in prevention or treatment of solid tumors; and use of the bacterium of the genus *Bifidobacterium* of the present invention for manufacturing an agent for preventing or treating solid tumors.

### Effect of the Invention

The bacterium of the genus *Bifidobacterium* of the present invention has a cancer cytotoxic activity against solid cancers and an anti-tumor effect against solid tumors and is therefore effective in prevention or treatment of solid tumors. It is inferred that, since the bacterium of the genus *Bifidobacterium* of the present invention does not grow in a normal tissue but grows only in a tumor tissue in an anaerobic environment and expresses/secretes a diabody-type BsAb that binds specifically to both CD3 and a tumor cell surface antigen, a human T cell expressing CD3 on its surface is recruited to the vicinity of the solid tumor expressing the tumor cell surface antigen on its surface via the diabody-type BsAb and activated into a cytotoxic human T cell, as a result of which the cancer cytotoxic activity and the anti-tumor effect described above are exerted. Further, since the diabody-type BsAb is secreted specifically and sustainably in the tumor tissue, effects such as long-lasting efficacy and reduction of the risk of unintended human T cell activation in the normal tissue can be expected.

### Brief Description of Drawings

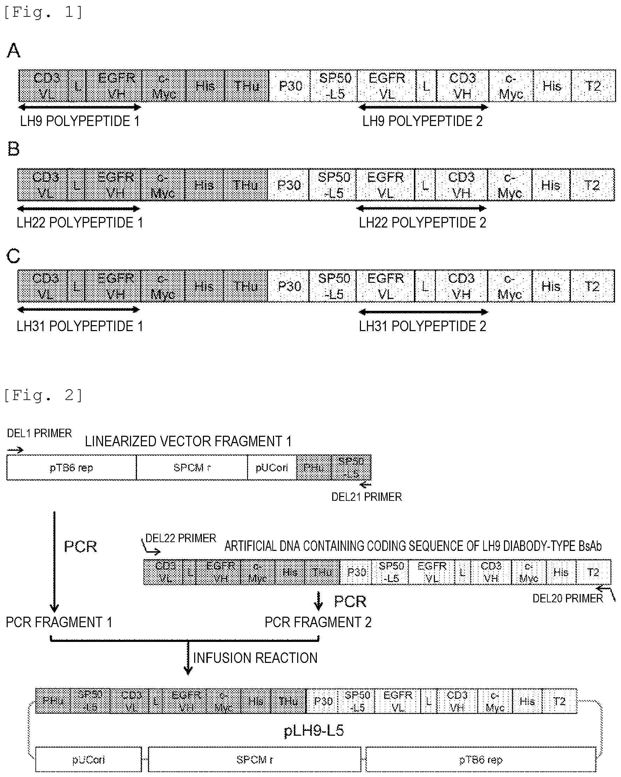

[Figure 1] Figure 1 includes schematic diagrams of three types of artificial DNAs containing the coding sequences of EGFR×CD3 diabody-type BsAbs (artificial DNA containing the coding sequence of LH9 diabody-type BsAb [Figure 1A], artificial DNA containing the coding sequence of LH22 diabody-type BsAb [Figure 1B], and artificial DNA containing the coding sequence of LH31 diabody-type BsAb [Figure 1C]). The "CD3 VL" in the figure indicates a nucleotide sequence encoding VL derived from anti-human CD3 antibody, the "L" in the figure indicates a nucleotide sequence encoding a linker peptide (AGGGGS), the "EGFR VH" in the figure indicates a nucleotide sequence encoding VH derived from anti-human EGFR antibody, the "c-Myc" in the figure indicates a nucleotide sequence encoding a c-Myc tag, the "His" in the figure indicates a nucleotide sequence encoding a His tag, the "THu" in the figure indicates a Hu terminator for B bacteria, the "P30" in the figure indicates a P30 promoter for B bacteria, the "SP50-L5" in the figure indicates a nucleotide sequence encoding a connecting peptide between a secretion signal peptide (SP50) and a linker peptide (L5), the "EGFR VL" in the figure indicates a nucleotide sequence encoding VL derived from anti-human EGFR antibody, the "CD3 VH" in the figure indicates a nucleotide sequence encoding VH derived from anti-human CD3 antibody, and the "T2" in the figure indicates a T2 terminator for B bacteria.
[Figure 2] Figure 2 is a schematic diagram of a method for producing plasmid pLH9-L5. The "PHu" in the figure indicates a Hu promoter for B bacteria (PHu), and the "SPCMr" in the figure indicates a spectinomycin resistance gene.
[Figure 3] Figure 3 is a schematic diagram of a method for producing plasmid pLH22-L5-F1cm.
[Figure 4] Figure 4 is a schematic diagram of a method for producing plasmid pLH22-L5cm.
[Figure 5] Figure 5 is a schematic diagram of a method for producing plasmid pLH31-L5-F1.
[Figure 6] Figure 6 is a schematic diagram of a method for producing plasmid pLH31-L5.
[Figure 7] Figure 7 is a diagram showing the results of analyzing culture supernatants of four types of *Bifidobacterium* transformants (FLM-9H strain [lane 2], FOM-22H strain [lane 3], DUM-31H strain [lane 4], and BE shuttle strain [lane 5]) by the Western blotting method using anti-His tag antibody. Lane 1 indicates a size marker.
[Figure 8] Figure 8 is a graph showing the results (n = 2) of analyzing three types of EGFR×CD3 diabody-type BsAbs (LH9 diabody-type BsAb ["FLM-9H" in the figure], LH22 diabody-type BsAb ["FOM-22H" in the figure], and LH31 diabody-type BsAb ["DUM-31H" in the figure]) for binding property to human EGFR by the ELISA method.
[Figure 9] Figure 9 is a graph showing the results (n = 2) of analyzing the three types of EGFR×CD3 diabody-type BsAbs for binding property to human CD3 by the ELISA method.
[Figure 10] Figure 10 is a graph showing the results (n = 3) of analyzing the three types of EGFR×CD3 diabody-type BsAbs for injury activity on HCT116 cells using cell viability as an index.
[Figure 11] Figure 11 is a schematic diagram of a method for producing plasmids pLH31-L2-F1 and pLH31-L2-F2.
[Figure 12] Figure 12 is a schematic diagram of a method for producing plasmid pLH31-L2.
[Figure 13] Figure 13 is a schematic diagram of a method for producing plasmid p126.
[Figure 14] Figure 14 is a diagram showing the results of analyzing culture supernatants of two types of *Bifidobacterium* transformants (DUM-126H strain [lane 2] and BE shuttle strain [lane 3]) by the Western blotting method using anti-His tag antibody. Lane 1 indicates a size marker.
[Figure 15] Figure 15 is a graph showing the results (n = 2) of analyzing EGFR×CD3 diabody-type BsAbs purified from three types of *Bifidobacterium* transformants (DUP-143 strain, DUP-153 strain, and DUM-126H strain) and a HER2×CD3 diabody-type BsAb purified from one type of *Bifidobacterium* transformant (CUM-36-1H strain) for binding property to human CD3 and human EGFR by the ELISA method.
[Figure 16] Figure 16 is a graph showing the results (n = 3) of analyzing culture supernatants of two types of *Bifidobacterium* transformants (DUM-126H strain and BE shuttle strain) for injury activity on HCT116 cells using cell viability as an index.
[Figure 17] Figure 17 is a graph showing the results (average and standard deviation when n = 14 or 15) of analyzing mice of three types of groups (PBS group, BE shuttle strain group, and DUM-126H strain group) for tumor volume. In the figure, the symbols "**" and "***" respectively indicate that there are statistically significant differences (p < 0.01 and p < 0.001) based on the one way ANOVA and Tukey's multiple comparison test.
[Figure 18] Figure 18 is a schematic diagram of a method for producing plasmid p129.
[Figure 19] Figure 19 is a schematic diagram of a method for producing plasmid p130.
[Figure 20] Figure 20 is a schematic diagram of a method for producing plasmid p130TL.
[Figure 21] Figure 21 is a schematic diagram of a method for producing plasmid pLH31-L5-F2-C.
[Figure 22] Figure 22 is a schematic diagram of a method for producing plasmid p133.
[Figure 23] Figure 23 is a schematic diagram of a method for producing plasmid p143-F2TL.
[Figure 24] Figure 24 is a schematic diagram of a method for producing plasmid p143TL.
[Figure 25] Figure 25 is a schematic diagram of a method for producing plasmid p143TL4.
[Figure 26] Figure 26 is a schematic diagram of a method for producing plasmid p143TLB6a.
[Figure 27] Figure 27 is a schematic diagram of a method for producing plasmid p152.
[Figure 28] Figure 28 is a schematic diagram of a method for producing plasmid p153-F2TL.
[Figure 29] Figure 29 is a schematic diagram of a method for producing plasmid p153TL.
[Figure 30] Figure 30 is a schematic diagram of a method for producing plasmid p153TL4.
[Figure 31] Figure 31 is a schematic diagram of a method for producing plasmid p153TLB6a.
[Figure 32] Figure 32 is a diagram showing the results (n = 3) of SDS-PAGE analyzing mutant LH31 diabody-type BsAbs derived from DUP-143 strain (lanes 2 to 4) and mutant LH31 diabody-type BsAbs derived from DUP-153 strain (lanes 5 to 7). In the figure, the "band 1" indicates a band derived from mutant LH31 polypeptide 1 in the mutant LH31 diabody-type BsAbs, and the "band 2" indicates a band derived from mutant LH31 polypeptide 2 in the mutant LH31 diabody-type BsAbs. Lane 1 indicates a size marker.
[Figure 33] Figure 33 is a graph showing the results (n = 3) of analyzing culture supernatants of three types of *Bifidobacterium* transformants (DUP-143 strain, DUP-153 strain, and BE shuttle strain) for injury activity on three types of cancer cells (TFK-1 cells [Figure 33A], HCT116 cells [Figure 33B], and RKO cells [Figure 33C]) using cell viability as an index.
[Figure 34] Figure 34 is a graph showing the results (average and standard deviation when n = 12) of analyzing mice of four types of groups (BE shuttle strain group, DUM-126H strain group, DUP-143 strain-administered group, and DUP-153 strain-administered group) for tumor volume. In the figure, the symbol "*" indicates that there is a statistically significant difference (p < 0.05) based on the one way ANOVA and Tukey's multiple comparison test.

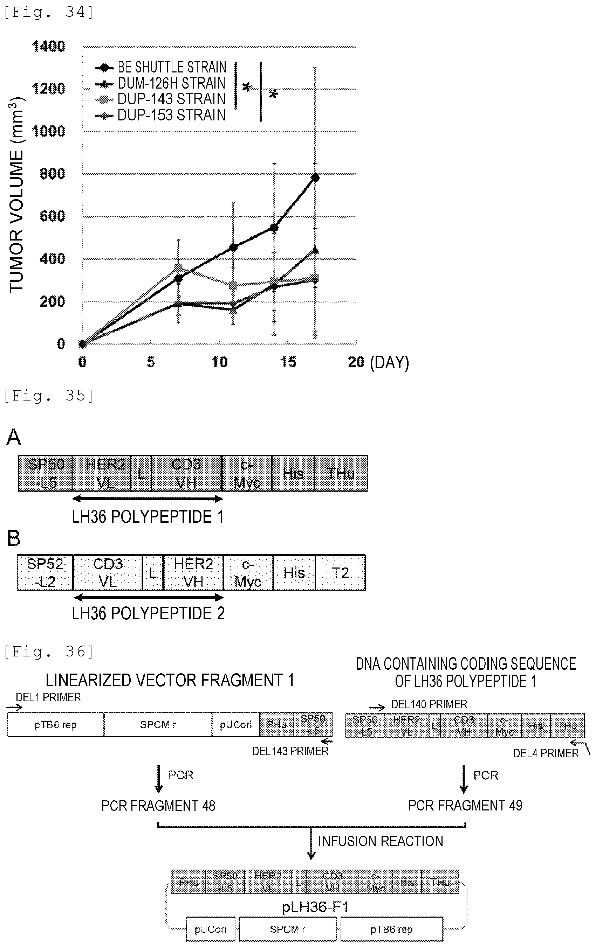

[Figure 35] Figure 35 is a schematic diagram of two types of artificial DNAs containing the coding sequences of polypeptides constituting HER2×CD3 diabody-type BsAb (an artificial DNA containing the coding sequence of LH36 polypeptide 1 [Figure 35A] and an artificial DNA containing the coding sequence of LH36 polypeptide 2 [Figure 35B]). The "HER2 VL" in the figure indicates a nucleotide sequence encoding VL derived from anti-HER2 antibody, the "HER2 VH" in the figure indicates a nucleotide sequence encoding VH derived from anti-HER2 antibody, the "SP52-L2" in the figure indicates a nucleotide sequence encoding a connecting peptide between a secretion signal peptide (SP52) and a linker peptide (L2).
[Figure 36] Figure 36 is a schematic diagram of a method for producing plasmid pLH36-F1.
[Figure 37] Figure 37 is a schematic diagram of a method for producing plasmid pLH36-1_F2.
[Figure 38] Figure 38 is a schematic diagram of a method for producing plasmid pLH36-2_F2.
[Figure 39] Figure 39 is a schematic diagram of a method for producing plasmid pLH36-1.
[Figure 40] Figure 40 is a schematic diagram of a method for producing plasmid pLH36-2.
[Figure 41] Figure 41 is a diagram showing the results of analyzing culture supernatants of three types of *Bifidobacterium* transformants (CUM-36-1H strain [lane 2], CUM-36-2H strain [lane 3], and BE shuttle strain [lane 4]) by the Western blotting method using anti-His tag antibody. Lane 1 indicates a size marker.
[Figure 42] Figure 42 is a graph showing the results (n = 2) of analyzing HER2×CD3 diabody-type BsAbs purified from two types of *Bifidobacterium* transformants (CUM-36-1H strain and CUM-36-2H strain) and EGFR×CD3 diabody-type BsAbs purified from three types of *Bifidobacterium* transformants (FLM-9H strain, FOM-22H strain, and DUM-31H strain) for binding property to human CD3 and HER2 by the ELISA method.
[Figure 43] Figure 43 is a graph showing the results (n = 3) of analyzing culture supernatants of three types of *Bifidobacterium* transformants (CUM-36-1H strain, CUM-36-2H strain, and BE shuttle strain) for injury activity on TFK-1 cells using cell viability as an index.
[Figure 44] Figure 44 is a schematic diagram of a method for producing plasmid pEUM-H_F1.
[Figure 45] Figure 45 is a schematic diagram of a method for producing plasmid pEUM-H_F2.
[Figure 46] Figure 46 is a schematic diagram of a method for producing plasmid pEUM-H.
[Figure 47] Figure 47 is a diagram showing the results of analyzing culture supernatants of two types of *Bifidobacterium* transformants (EUM-H strain [lane 2] and BE shuttle strain [lane 3]) by the Western blotting method using anti-His tag antibody. Lane 1 indicates a size marker.
[Figure 48] Figure 48 is a graph showing the results (n = 2) of analyzing EpCAM×CD3 diabody-type BsAb purified from *Bifidobacterium* transformant (EUM-H strain) for binding property to human CD3 and human EpCAM by the ELISA method.
[Figure 49] Figure 49 is a graph showing the results (n = 3) of analyzing EpCAM×CD3 diabody-type BsAb purified from *Bifidobacterium* transformants (EUM-H strain) for injury activity on HCT116 cells using cell viability as an index.
[Figure 50] Figure 50 is a schematic diagram of a method for producing plasmid pLH-PUM-H_F1.
[Figure 51] Figure 51 is a schematic diagram of a method for producing plasmid pLH-PUM-H_F2.
[Figure 52] Figure 52 is a schematic diagram of a method for producing plasmid pHL-PUM-H_F1.
[Figure 53] Figure 53 is a schematic diagram of a method for producing plasmid pHL-PUM-H_F2.
[Figure 54] Figure 54 is a schematic diagram of a method for producing plasmid pLH-PUM-H.
[Figure 55] Figure 55 is a schematic diagram of a method for producing plasmid pHL-PUM-H.
[Figure 56] Figure 56 is a diagram showing the results of analyzing culture supernatants of three types of *Bifidobacterium* transformants (LH-PUM-H strain [lane 2], HL-PUM-H strain [lane 3], and BE shuttle strain [lane 4]) by the Western blotting method using anti-His tag antibody. Lane 1 indicates a size marker.
[Figure 57] Figure 57 is a graph showing the results (n = 3) of analyzing PSMA×CD3 diabody-type BsAbs purified from two types of *Bifidobacterium* transformants (LH-PUM-H strain and HL-PUM-H strain) for injury activity on 22Rv1 cells using cell viability as an index.
[Figure 58] Figure 58 is a graph showing the results (n = 2) of analyzing EGFR×CD3 diabody-type BsAb purified from *Bifidobacterium* transformant DUP-143 strain for binding property to human HER3 by the ELISA method.
[Figure 59-1] Figure 59-1 is a graph showing typical examples of the results (n = 3 wells) of analyzing EGFR×CD3 diabody-type BsAb purified from *Bifidobacterium* transformant DUP-143 strain for injury activity on one type of human epidermoid cancer cell line (A-431[Figure 59-1A]) and one type of human non-small cell lung cancer cell line (NCI-H1975[Figure 59-1B]) using cell injury rate as an index.
[Figure 59-2] Figure 59-2 is a graph showing typical examples of the results (n = 3 wells) of analyzing EGFR×CD3 diabody-type BsAb purified from *Bifidobacterium* transformant DUP-143 strain for injury activity on four types of human colon cancer cell lines (HCT116 [Figure 59-2A], HT-29 [Figure 59-2B], RKO [Figure 59-2C], and SW620 [Figure 59-2D]) using cell injury rate as an index.
[Figure 60] Figure 60 is a schematic diagram of a method for producing plasmid p195.
[Figure 61] Figure 61 is a schematic diagram of a method for producing plasmid p196.
[Figure 62] Figure 62 is a schematic diagram of a method for producing plasmid p199.
[Figure 63] Figure 63 is a diagram showing the results of analyzing a culture supernatant purified product of *Bifidobacterium* transformant (DUP-199HAF strain [lane 2]) by the SDS-PAGE method. Lane 1 indicates a size marker.

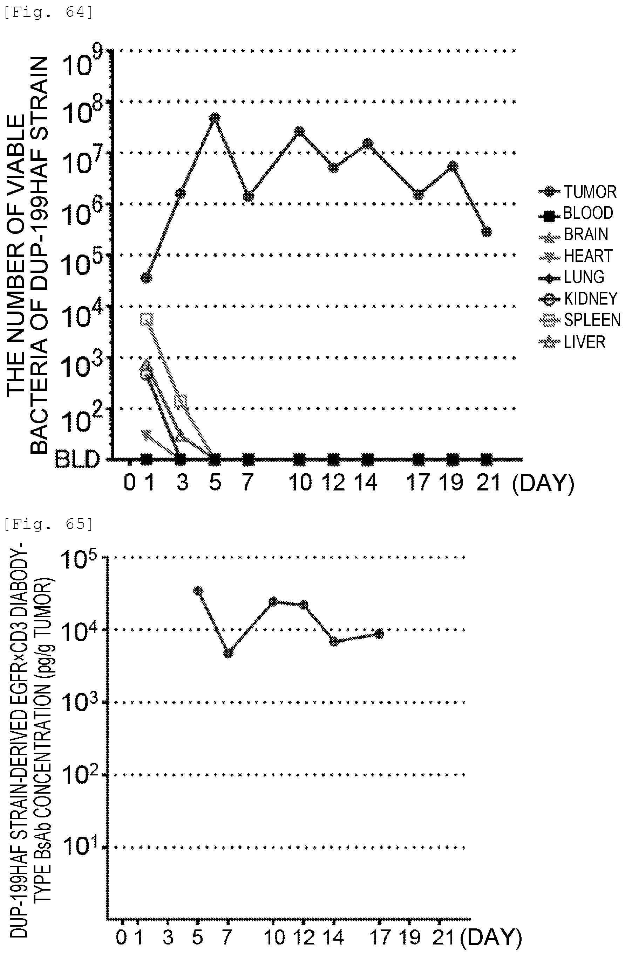

[Figure 64] Figure 64 is a graph showing the results (central value when n = 5) of analyzing the number of viable bacteria of DUP-199HAF strain in each of the tumor, the non-tumor tissues (brain, heart, lung, kidney, spleen, and liver), and the blood on day 1, day 3, day 5, day 7, day 10, day 12, day 14, day 17, day 19, and day 21 after administration of the DUP-199HAF strain into the tail veins of cancer-bearing SCID mice. In the figure, the "BLD" indicates below the detection limit. The "number of viable bacteria in DUP-199HAF strain" on the vertical axis indicates, for the tumor or the non-tumor tissues, cfu of the DUP-199HAF strain per 1 g of the tumor or the non-tumor tissues and indicates, for blood, cfu per 1 mL of blood.
[Figure 65] Figure 65 is a graph showing the results (average when n = 5) of measuring the concentration of the EGFR×CD3 diabody-type BsAb derived from DUP-199HAF strain in the tumor on day 1, day 3, day 5, day 7, day 10, day 12, day 14, day 17, day 19, and day 21 after administration of the DUP-199HAF strain into the tail veins of cancer-bearing SCID mice.
[Figure 66] Figure 66 is a graph showing the results (average and standard deviation when n = 3 or 4) of analyzing the mice of groups 1 to 6 for tumor volume. In the figure, the symbol "*" indicates that there is a statistically significant difference (p < 0.05) based on the one way ANOVA and Tukey's multiple comparison test.
[Figure 67] Figure 67 is a graph showing the results (average and standard deviation when n = 3 or 4) of measuring the mice of groups 1 to 6 for body weight.

### Mode of Carrying Out the Invention

The bacterium of the genus *Bifidobacterium* of the present invention is a bacterium of the genus *Bifidobacterium* expressing: a first polypeptide (which may be hereinafter referred to as "first polypeptide of the present invention") containing a light-chain variable region (VL) that binds to human CD3 (which may be hereinafter referred to as "CD3 VL") and a heavy-chain variable region (VH) that binds to a human tumor cell surface antigen (which may be hereinafter referred to as "tumor VH"); and a second polypeptide (which may be hereinafter referred to as "the second polypeptide of the present invention") containing VL that binds to a human tumor cell surface antigen (which may be hereinafter referred to as "tumor VL") and VH that binds to human CD3 (which may be hereinafter referred to as "CD3 VH"), and secreting a diabody-type bispecific antibody composed of the first polypeptide of the present invention and the second polypeptide of the present invention (which may be hereinafter referred to as "diabody-type BsAb of the present invention"), more specifically, a bacterium of the genus *Bifidobacterium* having a polynucleotide (which may be hereinafter referred to as "first polynucleotide of the present invention") having a nucleotide sequence encoding the first polypeptide of the present invention and a polynucleotide (which may be hereinafter referred to as "second polynucleotide of the present invention") having a nucleotide sequence encoding the second polypeptide of the present invention. In the bacterium of the genus *Bifidobacterium* of the present invention, the first polynucleotide of the present invention and the second polynucleotide of the present invention may be integrated on the chromosome of the bacterium of the genus *Bifidobacterium* of the present invention or on an autonomously replicable vector present outside the chromosome of the bacterium of the genus *Bifidobacterium* of the present invention. In view of convenience, a bacterium of the genus *Bifidobacterium* in which the first polynucleotide of the present invention and the second polynucleotide of the present invention are integrated on an autonomously replicable vector present outside the chromosome of the bacterium of the genus *Bifidobacterium* of the present invention (i.e., a bacterium of the genus *Bifidobacterium* holding an autonomously replicable vector [which may be hereinafter referred to as "vector of the present invention"] containing the first polynucleotide of the present invention and the second polynucleotide of the present invention) is preferable. In general, a promoter is operably connected onto the upstream of the first polynucleotide of the present invention and the second polynucleotide of the present invention, or both of the upstream of the first polynucleotide of the present invention and the upstream of the second polynucleotide of the present invention. Here, the term "upstream" means a direction opposite to the direction in which mRNA transcription proceeds (downstream).

In this description, the "promoter" means a region to which RNA polymerase (preferably, RNA polymerase and basic transcription factor) binds and in which mRNA transcription encoded by the gene located downstream thereof starts. The promoter generally includes the transcription start site (TSS) .

The agent for preventing or treating solid tumors of the present invention is a formulation specified for use "for preventing or treating solid tumors" and containing the bacterium of the genus *Bifidobacterium* of the present invention as an active component (which may be hereinafter referred to as "prophylactic/therapeutic agent of the present invention"). Here, to prevent solid tumors includes to prevent worsening of symptoms of solid tumors other than prevention of onset of solid tumors. The prophylactic/therapeutic agent of the present invention may be used alone as a pharmaceutical product (formulation) or may be further mixed with an additive and used in the form of a composition (pharmaceutical composition). In this description, solid tumors include both solid malignant tumors and solid benign tumors, and solid tumors are preferably solid malignant tumors (i.e., solid cancers).

Examples of the solid cancers can include colorectal cancer, head and neck cancer, breast cancer, lung cancer (e.g., non-small cell lung cancer), esophageal cancer, gastric cancer, liver cancer, gallbladder cancer, cholangiocarcinoma, pancreatic cancer, pancreatic islets cell cancer, choriocarcinoma, colon cancer, renal cell cancer, adrenal cortex cancer, bladder cancer, testis cancer, prostate cancer, testicular cancer, ovarian cancer, uterine cancer, thyroid cancer, squamous-cell carcinoma (epidermoid cancer), skin cancer, brain tumor, malignant carcinoid tumor, osteosarcoma, soft tissue sarcoma, neuroblastoma, Wilms tumor, retinoblastoma, and melanoma.

In this description, the "diabody-type bispecific antibody" means an antibody (i.e., the diabody-type BsAb of the present invention) which binds specifically to both human CD3 and a human tumor cell surface antigen and in which "CD3 VL" in the first polypeptide of the present invention and "CD3 VH" in the second polypeptide of the present invention are non-covalently bound (e.g., via an ionic bond, a hydrogen bond, or van der Waals force; hereinafter, the same applies), and "tumor VL" in the second polypeptide of the present invention and "tumor VH" in the first polypeptide of the present invention are non-covalently bound, to constitute a heterodimer of the first polypeptide of the present invention and the second polypeptide of the present invention.

Specifically, examples of the combination of the first polypeptide of the present invention and the second polypeptide of the present invention constituting the diabody-type BsAb of the present invention can include a combination of the first polypeptide of the present invention containing "CD3 VL" and "tumor VH" in the order from the amino (N) terminus toward the carboxyl (C) terminus and the second polypeptide of the present invention containing "tumor VL" and "CD3 VH" in the order from the N-terminus toward the C-terminus; and a combination of the first polypeptide of the present invention containing "tumor VH" and "CD3 VL" in the order from the N-terminus toward the C-terminus and the second polypeptide of the present invention containing "CD3 VH" and "tumor VL" in the order from the N-terminus toward the C-terminus. Among these, the combination of the first polypeptide of the present invention containing "CD3 VL" and "tumor VH" in the order from the N-terminus toward the C-terminus and the second polypeptide of the present invention containing "tumor VL" and "CD3 VH" in the order from the N-terminus toward the C-terminus can be mentioned as a suitable example since its effect has been demonstrated in Examples, which will be described below.

In the case where the first polypeptide of the present invention contains "CD3 VL" and "tumor VH" in the order from the N-terminus toward the C-terminus, one or more of a secretion signal peptide, a linker peptide, and their conjugate (a conjugate of the C-terminus of the secretion signal peptide and the N-terminus of the linker peptide; hereinafter, the same applies), and a protein tag may be connected or not connected at one, two, or three sites selected from the N-terminus of "CD3 VL", the site between "CD3 VL" and "tumor VH" (i.e., a site that is the C-terminus of "CD3 VL" and the N-terminus of "tumor VH"), and the C-terminus of "tumor VH" in the first polypeptide of the present invention. Further, in the case where the first polypeptide of the present invention contains "tumor VH" and "CD3 VL" in the order from the N-terminus toward the C-terminus, one or more of a secretion signal peptide, a linker peptide, and their conjugate, and a protein tag may be connected or not connected at one, two, or three sites selected from the N-terminus of "tumor VH", the site between "tumor VH" and "CD3 VL" (i.e., a site that is the C-terminus of "tumor VH" and the N-terminus of "CD3 VL"), and the C-terminus of "CD3 VL" in the first polypeptide of the present invention.

Further, in the case where the second polypeptide of the present invention contains "tumor VL" and "CD3 VH" in the order from the N-terminus toward the C-terminus, one or more of a secretion signal peptide, a linker peptide, and their conjugate, and a protein tag may be connected or not connected at one, two, or three sites selected from the N-terminus of "tumor VL", the site between "tumor VL" and "CD3 VH" (i.e., a site that is the C-terminus of "tumor VL" and the N-terminus of "CD3 VH"), and the C-terminus of "CD3 VH" in the second polypeptide of the present invention. Further, in the case where the second polypeptide of the present invention contains "CD3 VH" and "tumor VL" in the order from the N-terminus toward the C-terminus, one or more of a secretion signal peptide, a linker peptide, and their conjugate, and a protein tag may be connected or not connected at one, two, or three sites selected from the N-terminus of "CD3 VH", the site between "CD3 VH" and "tumor VL" (i.e., a site that is the C-terminus of "CD3 VH" and the N-terminus of "tumor VL"), and the C-terminus of "tumor VL" in the second polypeptide of the present invention.

Examples of the linker peptide may include a linker peptide that does not inhibit the formation of the diabody-type bispecific antibody and the functions thereof (i.e., the binding ability to human CD3 and a human tumor cell surface antigen). The linker peptide, for example, has a length in the range of 1 to 30 amino acids, preferably 1 to 20 amino acids, more preferably 2 to 15 amino acids, further preferably 3 to 10 amino acids, most preferably about 6 to 7 amino acids (such as 4 to 9 amino acids and 5 to 8 amino acids), in the case of using the linker peptide for the connection between VL and VH. Further, the linker peptide, for example, has a length in the range of 1 to 30 amino acids, preferably 1 to 20 amino acids, more preferably 1 to 15 amino acids, further preferably 1 to 10 amino acids, most preferably 1 to 6 amino acids, in the case of using the linker peptide for the connection between the secretion signal peptide and VL or VH.

Since its effect has been demonstrated in Examples, which will be described below, the first polypeptide of the present invention in which a linker peptide consisting of 4 to 9 amino acids is connected at the site between "CD3 VL" and "tumor VH" can be mentioned as a suitable example. Further, the second polypeptide of the present invention in which a linker peptide consisting of 4 to 9 amino acids is connected each at the N-terminus of "tumor VL" and the site between "tumor VL" and "CD3 VH" can be mentioned as a suitable example since its effect has been demonstrated in Examples, which will be described below.

The secretion signal peptide is preferably connected to the linker peptide to form a secretion signal peptide-linker peptide conjugate in view of expression/secretion efficiency of heterologous polypeptides. Examples of the secretion signal peptide-linker peptide conjugate can include the secretion signal peptide-linker peptide conjugate disclosed in International Publication No. WO 2016/088376, specifically, those containing (i) to (iii) below:
(i) an amino acid sequence having a sequence identity of at least 80% with the amino acid sequence shown in SEQ ID No: 10;
(ii) an amino acid sequence having a sequence identity of at least 80% with the amino acid sequence shown in SEQ ID No: 13; and
(iii) an amino acid sequence having a sequence identity of at least 80% with the amino acid sequence shown in SEQ ID No: 18.

Examples of the protein tag can include a c-Myc tag, a histidine (His) tag, a HA tag, and a FLAG tag.

The origin, type, class, form, and the like of the VL and the VH in the diabody-type BsAb of the present invention are not particularly limited. Examples thereof include VL and VH derived from humans; VL and VH derived from nonhuman animals such as mice and rats; VL and VH derived from polyclonal antibodies, VL and VH derived from oligoclonal antibodies (mixtures of several to dozens of antibodies), VL and VH derived from monoclonal antibodies; chimeric VL and VH, humanized VL and VH in which a part of the VL and VH (e.g., variable regions excluding complementarity determining regions [CDRs]) is substituted with a region derived from a different species.

Examples of the "CD3 VL" and "CD3 VH" can include VL and VH derived from anti-human CD3 antibody with a known amino acid sequence, for example, VL and VH derived from the anti-human CD3 antibody disclosed in Japanese unexamined Patent Application Publication (Translation of PCT Application) No. 2009-520734; VL and VH derived from the anti-human CD3 antibody disclosed in Japanese unexamined Patent Application Publication (Translation of PCT Application) No. 2008-503449; VL and VH derived from the anti-human CD3 antibody disclosed in Japanese unexamined Patent Application Publication (Translation of PCT Application) No. 2013-508391; VL and VH derived from the anti-human CD3 antibody disclosed in Japanese unexamined Patent Application Publication (Translation of PCT Application) No. 2017-504314; VL and VH derived from the anti-human CD3 antibody disclosed in Japanese unexamined Patent Application Publication (Translation of PCT Application) No. 2018-535972; and VL and VH derived from the anti-human CD3 antibody disclosed in International Publication No. WO 2015/146437, specifically, VL consisting of an amino acid sequence having a sequence identity of at least 80% with amino acid residues 1 to 107 of SEQ ID No: 1 and VH consisting of an amino acid sequence having a sequence identity of at least 80% with amino acid residues 115 to 233 of SEQ ID No: 2; VL consisting of an amino acid sequence having a sequence identity of at least 80% with amino acid residues 1 to 107 of SEQ ID No: 3 and VH consisting of an amino acid sequence having a sequence identity of at least 80% with amino acid residues 115 to 233 of SEQ ID No: 4; and VL consisting of an amino acid sequence having a sequence identity of at least 80% with amino acid residues 1 to 108 of SEQ ID No: 5 and VH consisting of an amino acid sequence having a sequence identity of at least 80% with amino acid residues 115 to 236 of SEQ ID No: 6. Based on the amino acid sequences of these VL and VH, numbering by kabat (see the literature "kabat, E.A. et al., (1991) NIH Publication No. 91-3242, sequences of proteins of immunological interest") is performed, and those having CDR1 to CDR3 of the heavy (H) chain and the light (L) chain, that is, Kabat positions 31 to 35 corresponding to the H-chain CDR1, Kabat positions 50 to 65 corresponding to the H-chain CDR2, and Kabat positions 95 to 102 corresponding to the H-chain CDR3 and Kabat positions 24 to 34 corresponding to the L-chain CDR1, Kabat positions 50 to 56 corresponding to the L-chain CDR2, and Kabat positions 89 to 97 corresponding to the L-chain CDR3, in which variable regions other than the H-chain and L-chain CDR1 to CDR3 (specifically, framework regions [FRs]) are modified (humanized) into human-derived variable regions may be used as "CD3 VL" and "CD3 VH".

The human tumor cell surface antigen may be an antigen present on the tumor cell surface, and examples thereof can include human B7-H3, human CEA (carcinoembryonic antigen), human DLL3, an antigen belonging to the human EGFR (Epidermal Growth Factor Receptor) family (e.g., HER [Human Epidermal Growth Factor Receptor] 1 [which may be referred to also as human EGFR and human ErB1], HER2, HER3, and HER4), human EpCAM (Epithelial cell adhesion molecule), human GPC3 (Glypican-3), human GPA33 (glycoprotein A33), human MUC16, human P-type cadherin, human PSMA (Prostate Specific Membrane Antigen), human SSTR2, and human PD-L1. Since their effects have been demonstrated in Examples, an antigen belonging to the human EGFR family, human EpCAM, and human PSMA are preferable, and suitable examples of the antigen belonging to the human EGFR family can include human EGFR, human HER2, and human HER3.

Examples of the "tumor VL" and "tumor VH" can include VL and VH derived from an anti-human tumor cell surface antigen antibody with a known amino acid sequence, for example, VL and VH derived from the anti-human B7-H3 antibody disclosed in International Publication No. WO 2012/147713; VL and VH derived from the anti-human CEA antibody disclosed in Japanese unexamined Patent Application Publication (Translation of PCT Application) No. 2009-520734; VL and VH derived from the anti-human DLL3 antibody disclosed in International Publication No. WO 2011/093097; VL and VH derived from the anti-human EGFR antibody disclosed in International Publication No. WO 2011/062112; VL and VH derived from the anti-HER2 antibody disclosed in Japanese unexamined Patent Application Publication No. 2012-158534; VL and VH derived from the anti-HER3 antibody disclosed in Japanese unexamined Patent Application Publication (Translation of PCT Application) No. 2013-514793; VL and VH derived from the anti-human EpCAM antibody disclosed in Japanese unexamined Patent Application Publication (Translation of PCT Application) No. 2010-523096; and VL and VH derived from the anti-human MUC16 antibody disclosed in Japanese unexamined Patent Application Publication (Translation of PCT Application) No. 2013-529061, specifically, VL derived from anti-human EGFR antibody and consisting of an amino acid sequence having a sequence identity of at least 80% with amino acid residues 1 to 108 of SEQ ID No: 2, and VH derived from anti-human EGFR antibody and consisting of an amino acid sequence having a sequence identity of at least 80% with amino acid residues 114 to 235 of SEQ ID No: 1; VL derived from anti-human EGFR antibody and consisting of an amino acid sequence having a sequence identity of at least 80% with amino acid residues 1 to 108 of SEQ ID No: 4, and VH derived from anti-EGFR antibody and consisting of an amino acid sequence having a sequence identity of at least 80% with amino acid residues 114 to 234 of SEQ ID No: 3; VL derived from anti-HER2 antibody and consisting of an amino acid sequence having a sequence identity of at least 80% with amino acid residues 1 to 108 of SEQ ID No: 14, and VH derived from anti-HER2 antibody and consisting of an amino acid sequence having a sequence identity of at least 80% with amino acid residues 115 to 233 of SEQ ID No: 15; VH derived from anti-human EpCAM antibody and consisting of an amino acid sequence having a sequence identity of at least 80% with amino acid residues 115 to 234 of SEQ ID No: 82, and VL derived from anti-human EpCAM antibody and consisting of an amino acid sequence having a sequence identity of at least 80% with amino acid residues 1 to 113 of SEQ ID No: 83; and VH derived from anti-human PSMA antibody and consisting of an amino acid sequence having a sequence identity of at least 80% with amino acid residues 115 to 235 of SEQ ID No: 86, and VL derived from anti-human PSMA antibody and consisting of an amino acid sequence having a sequence identity of at least 80% with amino acid residues 1 to 107 of SEQ ID No: 87. Based on the amino acid sequences of these VL and VH, numbering by Kabat is performed, and those having CDR1 to CDR3 of the H chain and the L chain, that is, Kabat positions 31 to 35 corresponding to the H-chain CDR1, Kabat positions 50 to 65 corresponding to the H-chain CDR2, and Kabat positions 95 to 102 corresponding to the H-chain CDR3 and Kabat positions 24 to 34 corresponding to the L-chain CDR1, Kabat positions 50 to 56 corresponding to the L-chain CDR2, and Kabat positions 89 to 97 corresponding to the L-chain CDR3, in which variable regions other than the H-chain and L-chain CDR1 to CDR3 (specifically, FRs) are modified (humanized) into human-derived variable regions may be used as "tumor VL" and "tumor VH".

The non-covalent bond between the first polypeptide of the present invention and the second polypeptide of the present invention such as the non-covalent bond between "CD3 VL" in the first polypeptide of the present invention and "CD3 VH" in the second polypeptide of the present invention, and the non-covalent bond between "tumor VH" in the first polypeptide of the present invention and "tumor VL" in the second polypeptide of the present invention is promoted/stabilized by substituting the amino acid residues where "CD3 VL" and "CD3 VH" are adjacent to each other and/or the amino acid residues where "tumor VL" and "tumor VH" are adjacent to each other respectively with amino acid residues having opposite electric charges, thereby enabling the cytotoxic activity and the anti-tumor effect to be further enhanced. Examples of the amino acid residues to be substituted can include regions other than complementarity determining regions (CDRs) in the VL and VH, for example, the amino acid residues in the framework region (FR2) between CDR1 and CDR2, more specifically, examples of the combination of the amino acid residues to be substituted in the VL and the amino acid residues to be substituted in the VH can include combination of glutamine (Q) at Kabat position 38 on VL (VL Q38) and Q at Kabat position 39 on VH (VH Q39); and combination of proline (P) at Kabat position 44 on VL (VL P44) and leucine (L) at Kabat position 45 on VH (VH L45). Since all of these amino acid residues are neutral amino acids and are highly conserved in humans and mice, a combination of amino acid residues respectively having opposite electric charges can be obtained by substituting one of the amino acid residues in each combination with positively charged amino acids (e.g., lysine [K], arginine [R], and histidine [H]; hereinafter, the same applies) and substituting the other of the amino acid residues with negatively charged amino acids (e.g., glutamic acid [E] and aspartic acid [D]; hereinafter, the same applies). More specifically, in consideration of suppressing the association between VH and VL on the same polypeptide, it is preferable that (1) the amino acid residue at Kabat position 38 in "CD3 VL" of the first polypeptide of the present invention is glutamic acid, the amino acid residue at Kabat position 39 in "tumor VH" of the first polypeptide of the present invention is glutamic acid, the amino acid residue at Kabat position 38 in "tumor VL" of the second polypeptide of the present invention is lysine, and the amino acid residue at Kabat position 39 in "CD3 VH" of the second polypeptide of the present invention is lysine; and (2) the amino acid residue at Kabat position 38 in "CD3 VL" of the first polypeptide of the present invention is lysine, the amino acid residue at Kabat position 39 in "tumor VH" of the first polypeptide of the present invention is lysine, the amino acid residue at Kabat position 38 in "tumor VL" of the second polypeptide of the present invention is glutamic acid, and the amino acid residue at Kabat position 39 in "CD3 VH" of the second polypeptide of the present invention is glutamic acid.

In this description, "having a sequence identity of at least 80% with an amino acid sequence" means that the proportion of amino acids that are the same as in the amino acid sequence to be compared is 80% or more, preferably 85% or more, more preferably 88% or more, further preferably 90% or more, furthermore preferably 93% or more, particularly preferably 95% or more, particularly more preferably 98% or more, most preferably 99%. The identity of the amino acid sequence can be determined using known programs such as ClustalW, GENETYX, BLAST and the like.

The method for producing the vector of the present invention and the method for producing the bacterium of the genus *Bifidobacterium* of the present invention using the vector of the present invention can be carried out according to methods disclosed in commercially available experimental certificates, for example, Gene Manual, Kodansha Ltd., Yasuyuki Takagi, Procedure for Experiment in Genetic Engineering, Kodansha Ltd., Molecular/Cloning [Cold Spring Harbor Laboratory (1982)], Molecular/Cloning, second edition [Cold Spring Harbor Laboratory (1989)], Methods in Enzymology 194 (1991), and Experimental Medicine -separate volume-: Gene Experiment Method by Yeast, YODOSHA CO., LTD. (1994).

The promoter in the vector of the present invention may be any promoter that functions in bacterium of the genus Bifidobacterium, and examples thereof can include Hu promoter that is a promoter involved in the expression of a gene encoding histone-like DNA-binding protein derived from *Bifidobacterium longum;* P30 promoter (J. Microbiology, 2012, 638-643); P54 promoter that is a promoter involved in the expression of a gene encoding Elongation Factor Tu protein (J. Bacteriology, 2005, 5799-5808, J. Microbiology, 2012, 638-643); a promoter of *Bifidobacterium* breve-derived Gap gene (Biotechnol. Lett. 2008 30: 1983-1988); a promoter of *Bifidobacterium* longum-derived AmyB gene (Biotechnol. Lett. 2006 28: 163-168); 16SrRNA promoter (Biotechnol. Lett. 2008 30: 165-172); a promoter of GAPDH (pr-BL1363) gene (Appl Environ Microbiol. 200672 (11): 7401-7405); PRPL promoter Cancer Gene Ther. 200714: 151-157); a promoter of p572 (β-glycosidase from B. animalis subsp lactis) gene (J Microbiol Biotechnol. 2012 Dec; 22 (12): 1714-23); p919 (rplM promoter) (J Microbiol. 2012 Aug; 50 (4): 638-43); and p895 (rplR promoter) (JMicrobiol. 2012 Aug; 50 (4): 638-43).

The vector of the present invention contains a plasmid replication unit that functions in bacterium of the genus *Bifidobacterium* for autonomous replication. Examples of the replication unit can include replication units of pTB6 (Biosci Biotechnol Biochem. 2005 Feb; 69 (2): 422-5), pMB1 (Lett Appl Microbiol. 1990 Oct;11 (4):220-3), pTB4 (Structural analysis and application of plasmid pTB4 derived from *Bifidobacterium longum,* General presentation, Poster publication program, The Molecular Biology Society of Japan, 1994), pFI2576 (J Microbiol Biotechnol. 2009 Apr; 19 (4): 403-8), pCIBAO (Appl Environ Microbiol. 2007 Dec; 73 (24): 7858-66), pBC1 (Plasmid. 2007 Mar; 57 (2): 165-74), pDOJH10S (Appl Environ Microbiol. 2006 Jan; 72 (1): 527-35), and PKJ50 (Microbiology 1999 Mar; 145 (Pt): 585-92), and Rep4 (polynucleotide consisting of the nucleotide sequence of SEQ ID No: 12, the replication unit of pFI2576).

The vector of the present invention preferably contains drug resistance genes to be used for cloning of the vector of the present invention; and screening of the bacterium of the genus *Bifidobacterium* of the present invention containing the vector of the present invention, and a nucleotide sequence that terminates transcription of the first polynucleotide of the present invention and the second polynucleotide of the present invention (i.e., terminator).

The terminator is not particularly limited as long as it is a terminator that functions in a bacterium of the genus *Bifidobacterium* Specifically, examples thereof can include Hu terminator derived from *Bifidobacterium longum;* d0013 terminator that is a terminator of lactate dehydrogenase gene derived from *Bifidobacterium longum;* T572 terminator derived from *Bifidobacterium* animalis (J. Microbiol Biotechnol. 2012 Dec; 22 (12):1714-23); BBa_B0015 (T2) terminator that is an artificially designed terminator; and T2 terminator (International Publication No. WO 2016/088376).

Examples of the drug resistance gene can include spectinomycin resistance gene, chloramphenicol resistance gene, erythromycin resistance gene, and ampicillin resistance gene.

The vector of the present invention may contain the Escherichia coli origin of replication (e.g., pUCori, pBR322ori), or the Escherichia coli origin of replication may be removed after cloning and before introduction into a bacterium of the genus Bifidobacterium.

In the bacterium of the genus *Bifidobacterium* of the present invention, the first polypeptide of the present invention and the second polypeptide of the present invention may be monocistronically expressed (i.e., translated from one mRNA into one of the first polypeptide of the present invention or the second polypeptide of the present invention) or polycistronically expressed (i.e., translated from one mRNA into both the first polypeptide of the present invention and the second polypeptide of the present invention), but they are preferably polycistronically expressed for efficiently expressing the first polypeptide of the present invention and the second polypeptide of the present invention at 1:1. The bacterium of the genus *Bifidobacterium* of the present invention in which the first polypeptide of the present invention and the second polypeptide of the present invention are polycistronically expressed can be produced by producing the vector of the present invention containing no terminator and no promoter but containing a ribosome-binding site (RBS) between the first polynucleotide of the present invention and the second polynucleotide of the present invention and introducing the vector of the present invention into a bacterium of the genus Bifidobacterium. Examples of the RBS can include a polynucleotide consisting of 52 nucleotide residues upstream of the translation start codon of Hup gene derived from *Bifidobacterium longum* 105-A strain and a polynucleotide consisting of 54 nucleotide residues upstream of the translation start codon of 30S ribosomal protein S16 gene derived from *Bifidobacterium longum* 105-A strain.

Examples of the method for introducing the vector of the present invention into a bacterium of the genus *Bifidobacterium* can include the electroporation method.

Examples of the bacterium of the genus *Bifidobacterium* of the present invention can include *Bifidobacterium longum (B. longum), Bifidobacterium breve (B. breve), Bifidobacterium*/*adolescentis (B. adolescentis), Bifidobacterium bifidum (B. bifidum), Bifidobacterium*/*pseudolongum (B.pseudolongum), Bifidobacterium*/*thermophirum (B. thermophirum)*, *Bifidobacterium infantis (B.infantis), Bifidobacterium animalis (B. animalis), Bifidobacterium*/*angulatum (B. angulatum), Bifidobacterium*/*asteroides (B. asteroides), Bifidobacterium*/*boum (B. boum), Bifidobacterium*/*catenulatum (B. catenulatum), Bifidobacterium*/*choerinum (B. choerinum), Bifidobacterium*/*coryneforme (B. coryneforme), Bifidobacterium*/*cuniculi (B. cuniculi), Bifidobacterium*/*denticolens (B. denticolens), Bifidobacterium*/*dentium (B. dentium), Bifidobacterium*/*gallicum (B. gallicum), Bifidobacterium*/*gallinarum (B. gallinarum), Bifidobacterium globosum (B. globosum), Bifidobacterium*/*indicum (B. indicum), Bifidobacterium*/*inopinatum (B. inopinatum), Bifidobacterium*/*lactis (B. lactis), Bifidobacterium*/*lactentis (B. lactentis), Bifidobacterium*/*magnum (B. magnum), Bifidobacterium*/*merycicum (B. merycicum), Bifidobacterium*/*minimum (B. minimum), Bifidobacterium*/*mongolia enns (B. Mongolia Enns), Bifidobacterium*/*parvulorum (B. parvulorum), Bifidobacterium*/*pseudocatenulatum (B. pseudocatenulatum), Bifidobacterium*/*psychraerophilum (B. psychraerophilum)*, *Bifidobacterium*/*pullorum (B. pullorum)*, *Bifidobacterium*/*ruminale (B. ruminale), Bifidobacterium*/*ruminantium (B. ruminantium), Bifidobacterium*/*saeculare (B. saeculare), Bifidobacterium*/*scardovii (B. scardovii), Bifidobacterium*/*subtile (B. subtile), Bifidobacterium*/*suis (B. suis), and Bifidobacterium*/*thermacidophilum (B. thermacidophilum)* Among these, *Bifidobacterium longum, Bifidobacterium breve, Bifidobacterium*/*adolescentis, Bifidobacterium bifidum, and Bifidobacterium infantis,* which are known to be resident in the human intestine regardless of age, are preferable, and *Bifidobacterium longum* can be mentioned as a suitable example. All of these bacteria can be easily obtained as commercial products or from depository institutions.

Further, the strains of each species are not specifically limited, and examples thereof can include *Bifidobacterium longum* 105-A strain, *Bifidobacterium longum* aE-194b strain, *Bifidobacterium longum* bs-601 strain, *Bifidobacterium longum* M101-2 strain, and *Bifidobacterium longum* ATCC-15707 strain, for example, in the case of *Bifidobacterium longum.* Among them, *Bifidobacterium longum* 105-A strain can be mentioned as a suitable example. Further, examples thereof can include *Bifidobacterium breve* standard strain (JCM1192), *Bifidobacterium breve* aS-1 strain, and *Bifidobacterium breve* I-53-8W strain, for example, in the case of *Bifidobacterium breve.* Among them, *Bifidobacterium breve* standard strain and *Bifidobacterium breve* aS-1 strain can be mentioned as suitable examples. Further examples thereof can include *Bifidobacterium infantis* standard strain (JCM1222), *Bifidobacterium infantis* 1-10-5 strain, for example, in the case of *Bifidobacterium infantis.* Further, examples thereof can include *Bifidobacterium*/*lactentis* standard strain (JCM1210), for example, in the case of *Bifidobacterium*/*lactentis.* Further, examples thereof can include *Bifidobacterium bifidum* ATCC-11863 strain, for example, in the case of *Bifidobacterium bifidum.*

The prophylactic/therapeutic agent of the present invention is roughly classified into liquid type and non-liquid type. The prophylactic/therapeutic agent of the present invention of liquid type can be manufactured by purifying the culture solution of the bacterium of the genus *Bifidobacterium* of the present invention, adding suitable saline or fluid replacement or a pharmaceutical additive, as needed, and filling an ampoule, a vial container or the like with the mixture. Meanwhile, the prophylactic/therapeutic agent of the present invention of non-liquid type can be manufactured by adding a suitable protectant to the prophylactic/therapeutic agent of the present invention and filling an ampoule, a vial container or the like with the mixture, followed by freezing or freeze drying. Both oral administration and parenteral administration are possible as the method for administering the pharmaceutical composition of the present invention, but parenteral administration is preferable, and examples thereof can include intravenous administration and local administration.

The prophylactic/therapeutic agent of the present invention may further contain an additive such as a pharmaceutically acceptable general carrier, a binder, a stabilizer, an excipient, a diluent, a pH buffer, a disintegrant, an isotonic agent, an additive, a coating, a solubilizer, a lubricant, a solubilizing agent, a lubricant, a flavoring agent, a sweetener, a solvent, a gelling agent, and a nutritional supplement. Examples of the additive can specifically include water, saline, animal fat and oil, vegetable oil, lactose, starch, gelatin, crystalline cellulose, gum, talc, magnesium stearate, hydroxypropyl cellulose, polyalkylene glycol, polyvinyl alcohol, and glycerin.

The prophylactic/therapeutic agent of the present invention is preferably used in combination with an agent for promoting engraftment and/or growth of a bacterium of the genus *Bifidobacterium* in solid tumors, in order to promote the engraftment and growth of bacterium of the genus *Bifidobacterium* in solid tumors. Examples of the promoter can include saccharides such as arabinose, xylose, galactose, glucose, maltose, lactose, melibiose, melezitose, raffinose, and lactulose. Among these, maltose can be mentioned as a suitable example since its effect has been demonstrated in Examples, which will be described below.

The dose of the bacterium of the genus *Bifidobacterium* of the present invention is not particularly limited as long as it is an amount enough for the bacterium of the genus *Bifidobacterium* of the present invention to grow in the solid tumor sites and enough for the diabody-type BsAb of the present invention to be expressed/secreted in an effective therapeutic amount. The dose can be appropriately selected depending on the volume of the tumor, the body weight, age, and gender of the subject to be administered and can be appropriately increased or decreased depending on the degree of improvement. For example, in the case of intravenous administration, it is preferable to dispense an injectable formulation at a concentration as low as possible in multiple times or dilute the injectable formulation with a suitable fluid replacement for continuous injection, since it is required to reduce the risk of embolization due to bacterial mass or the like. For example, in the case of an adult, the bacterial cells of the bacterium of the genus *Bifidobacterium* of the present invention are administered at 10⁴ to 10¹² cfu (Colony Forming Unit) per 1 kg of the body weight once to multiple times a day for one to several days continuously or at appropriate intervals. More specifically, a formulation containing 10⁴ to 10¹⁰ cfu/mL of the bacterial cells of the bacterium of the genus *Bifidobacterium* of the present invention is administered in an amount of 1 to 1000 mL per adult directly or continuously after dilution with a suitable fluid replacement once to multiple times a day for one to several days. Further, in the case of local administration directly to the disease tissue, it is desirable to administer a high-concentration injection to a plurality of sites of the disease tissue, since it is required for bacteria to engraft and grow in the entire disease tissue as much as possible. For example, in the case of adult, the bacterial cells of the bacterium of the genus *Bifidobacterium* of the present invention are administered at 10⁴ to 10¹² cfu per 1 kg of the body weight once to multiple times a day for one to multiple days, as needed, continuously or at appropriate intervals. More specifically, a formulation containing 10⁴ to 10¹⁰ cfu/mL of the bacterial cells of the bacterium of the genus *Bifidobacterium* of the present invention is administered in an amount of 0.1 to 100 mL per adult directly several times a day continuously for one to multiple days, as needed.

Hereinafter, the present invention will be described further specifically by way of examples, but the technical scope of the present invention is not limited to these examples. In the following examples, the cultured cells were cultured under the conditions of 37°C and 5%CO₂.

### Examples

### [Example 1] Production of Bifidobacteria (FLM-9H strain, FOM-22H strain, and DUM-31H strain) expressing/secreting EGFR×CD3 diabody-type BsAb

*Bifidobacteria* expressing/secreting human EGFR×human CD3 diabody-type BsAb were produced according to the methods described in sections [1-1] to [1-5] below.

1-1: Synthesis of DNA containing coding sequences of EGFR×CD3 diabody-type BsAbs

In order to produce *Bifidobacteria* expressing/secreting three types of human EGFR×human CD3 diabody-type BsAbs, that is, a diabody-type BsAb composed of LH9 polypeptide 1 consisting of the amino acid sequence of SEQ ID No: 1 (see Table 1) and LH9 polypeptide 2 consisting of the amino acid sequence of SEQ ID No: 2 (see Table 2) (which may be hereinafter referred to as "LH9 diabody-type BsAb"); a diabody-type BsAb composed of LH22 polypeptide 1 consisting of the amino acid sequence of SEQ ID No: 3 (see Table 3) and LH22 polypeptide 2 consisting of the amino acid sequence of SEQ ID No: 4 (see Table 4) (which may be hereinafter referred to as "LH22 diabody-type BsAb"); and a diabody-type BsAb composed of LH31 polypeptide 1 consisting of the amino acid sequence of SEQ ID No: 5 (see Table 5) and LH31 polypeptide 2 consisting of the amino acid sequence of SEQ ID No: 6 (see Table 6) (which may be hereinafter referred to as "LH31 diabody-type BsAb"), three types of DNAs (an artificial DNA consisting of the nucleotide sequence of SEQ ID No: 7 and containing the coding sequence of LH9 diabody-type BsAb [see Figure 1A], an artificial DNA consisting of the nucleotide sequence of SEQ ID No: 8 and containing the coding sequence of LH22 diabody-type BsAb [see Figure 1B], and an artificial DNA consisting of the nucleotide sequence of SEQ ID No: 9 and containing the coding sequence of LH31 diabody-type BsAb [see Figure 1C]) were outsourced to GenScript Japan Inc. and synthesized. The polypeptides 1 and 2 each contains a secretion signal peptide SP50 (amino acid residues 1 to 56 of SEQ ID No: 10)-L5 linker peptide (amino acid residues 57 to 61 of SEQ ID No: 10) conjugate (SP50-L5; the amino acid sequence of SEQ ID No: 10) at the N-terminus, and the polypeptides 1 and 2 each contains a c-Myc (amino acid residues 4 to 13 of SEQ ID No: 11)-His (amino acid residues 23 to 28 of SEQ ID No: 11) fusion tag (the amino acid sequence of SEQ ID No: 11) at the C-terminus (see Figures 1 to 6).

**[Table 1]**

| |
|---|
| LH9 polypeptide 1 consisting of amino acid sequence of SEQ ID No: 1 |
| DIVLTQSPATLSLSPGERATLSCRASQSVSYMNWYQQKPGKAPKRWIYDTSKVASGVPARFS GSGSGTDYSLTINSLEAEDAATYYCQQWSSNPLTFGGGTKVEIKAAGGGGSQVQLQESGPG LVKPSQTLSLTCTVSGGSISSGDYYWSWIRQPPGKGLEWIGYIYYSGSTDYNPSLKSRVTMS VDTSKNQFSLKVNSVTAADTAVYYCARVSIFGVGTFDYWGQGTLVTVSSA |

The part surrounded by a square in the table indicates VL derived from anti-human CD3 antibody, the part surrounded by a double square indicates VH derived from anti-human EGFR antibody, and the peptide (AGGGGS) between the two indicates a linker peptide.

**[Table 2]**

| |
|---|
| LH9 polypeptide 2 consisting of amino acid sequence of SEQ ID No: 2 |
| DIVMTQSPATLSLSPGERATLSCRASQSVSSYLAWYQQKPGQAPRLLIYDASNRATGIPARFS GSGSGTDFTLTISSLEPEDFAVYYCHQYGSTPLTFGGGTKAEIKAAGGGGSDVQLVQSGAEV KKPGASVKVSCKASGYTFTRYTMHWVRQAPGQGLEWIGYINPSRGYTNYADSVKGRFTITT DKSTSTAYMELSSLRSEDTATYYCARYYDDHYCLDYWGQGTTVTVSS |

The part shown by an underline in the table indicates VL derived from anti-human EGFR antibody, the part shown by a double underline indicates VH derived from anti-human CD3 antibody, and the peptide (AGGGGS) between the two indicates a linker peptide.

**[Table 3]**

| |
|---|
| LH22 polypeptide 1 consisting of amino acid sequence of SEQ ID No: 3 |
| DIQMTQSPSSLSASVGDRVTITCSASSSVSYMNWYQQTPGKAPKRWIYDTSKLASGVPSRFS GSGSGTDYTFTISSLQPEDIATYYCQQWSSNPFTFGQGTKLQITAAGGGGS QVQLQESGPGL VKPSQTLSLTCTVSGGSISSGDYYWSWIRQPPGKGLEWIGYIYYSGSTDYNPSLKSRVTMSV DTSKNQFSLKVNSVTAADTAVYYCARVSIFGVGTFDYWGQGTLVTVSS |

The part surrounded by a square in the table indicates VL derived from anti-human CD3 antibody, the part surrounded by a double square indicates VH derived from anti-human EGFR antibody, and the peptide (AGGGGS) between the two indicates a linker peptide.

**[Table 4]**

| |
|---|
| LH22 polypeptide 2 consisting of amino acid sequence of SEQ ID No: 4 |
| DIVMTQSPATLSLSPGERATLSCRASQSVSSYLAWYQQKPGQAPRLLIYDASNRATGIPARFS GSGSGTDFTLTISSLEPEDFAVYYCHQYGSTPLTFGGGTKAEIKAAGGGGSQVQLVQSGGGV VQPGRSLRLSCKASGYTFTRYTMHWVRQAPGKGLEWIGYINPSRGYTNYNQKVKDRFTISR DNSKNTAFLQMDSLRPEDTGVYFCARYYDDHYSLDYWGQGTPVTVSS |

The part shown by an underline in the table indicates VL derived from anti-human EGFR antibody, the part shown by a double underline indicates VH derived from anti-human CD3 antibody, and the peptide (AGGGGS) between the two indicates a linker peptide.

**[Table 5]**

| |
|---|
| LH31 polypeptide 1 consisting of amino acid sequence of SEQ ID No: 5 |
| DIQMTQSPSSLSASVGDRVTITCRASQDIRNYLNWYQQKPGKAPKLLIYYTSRLESGVPSRFS GSGSGTDYTLTISSLQPEDFATYYCQQGNTLPWTFGQGTKVEIKAAGGGGS EVQLVESGGG LVQPGGSLRLSCAASGFTLSGDWIHWVRQAPGKGLEWLGEISAAGGYTDYADSVKGRFTIS ADTSKNTAYLQMNSLRAEDTAVYYCARESRVSFEAAMDYWGQGTLVTVSSA |

The part surrounded by a square in the table indicates VL derived from anti-human CD3 antibody, the part surrounded by a double square indicates VH derived from anti-human EGFR antibody, and the peptide (AGGGGS) between the two indicates a linker peptide.

**[Table 6]**

| |
|---|
| LH31 polypeptide 2 consisting of amino acid sequence of SEQ ID No: 6 |
| DIQMTQSPSSLSASVGDRVTITCRASQDLATDVAWYQQKPGKAPKLLIYSASFLYSGVPSRFS GSGSGTDFTLTISSLQPEDFATYYCQQSEPEPYTFGQGTKVEIKAAGGGGSEVQLVESGGGL VQPGGSLRLSCAASGYSFTGYTMNWVRQAPGKGLEWVALINPYKGVSTYNQKFKDRFTISV DKSKNTAYLQMNSLRAEDTAVYYCARSGYYGDSDWYFDVWGQGTLVTVSS |

The part shown by an underline in the table indicates VL derived from anti-human EGFR antibody, the part shown by a double underline indicates VH derived from anti-human CD3 antibody, and the peptide (AGGGGS) between the two indicates a linker peptide.

### 1-2: Production of plasmid pLH9-L5

In order to produce *Bifidobacterium* expressing/secreting LH9 diabody-type BsAb in B bacterium, plasmid pLH9-L5 was produced according to the procedure shown in Figure 2.

### 1-2-1: PCR reaction

Using 50 pg/µL of two types of template DNAs (artificial DNAs respectively containing linearized vector fragment 1 [see Figure 2] and the coding sequence of LH9 diabody-type BsAb), 0.2 µM of two types of primer sets configured to amplify the template DNA (a set of DEL1 primer and DEL21 primer, and a set of DEL22 primer and DEL20 primer; see Figure 2 and Table 7), and a PrimeSTAR HS (Premix) kit (manufactured by Takara Bio Inc.), a PCR reaction (30 cycles with 10 seconds at 98°C, 5 seconds at 65°C, and an elongation reaction at 72°C [for about 1 minute per 1000 base pairs] taken as one cycle) was performed, to produce PCR fragment 1 and PCR fragment 2.

### 1-2-2: Infusion reaction

The PCR fragment 1 and the PCR fragment 2 were connected using In-Fusion (R) HD Cloning Kit (manufactured by Takara Bio Inc.). Specifically, the PCR fragment 1 (vector fragment) and the PCR fragment 2 (insert fragment) were added to a microtube at a molar ratio of 1:1, then 2 µL of 5xIn-Fusion HD Enzyme premix and 1 µL of CloningEnhancer were added thereto, and the volume of the reaction solution was adjusted to 10 µL. This was kept warm at 37°C for 15 minutes and then further kept warm at 50°C for 15 minutes. For other procedures, an infusion reaction solution was prepared according to the product manual of the kit.

### 1-2-3: Production of Escherichia coli transformants and purification of plasmid pLH9-L5

Using 1 µL of the infusion reaction solution and Escherichia coli HST16CR competent cells (manufactured by Takara Bio Inc.), Escherichia coli transformants carrying the plasmid pLH9-L5 were produced according to the product manual. A suspension containing Escherichia coli transformants was applied to an LB agar medium containing 75 µg/mL spectinomycin, followed by overnight static culture at 37°C and then overnight shaking culture of Escherichia coli colonies formed on the agar medium with an LB liquid medium containing 75 µg/mL spectinomycin at 37°C, and then the plasmid pLH9-L5 was isolated/purified using QIAprep Spin Miniprep Kit (manufactured by QIAGEN K.K.).

### 1-3: Production of plasmid pLH22-L5cm

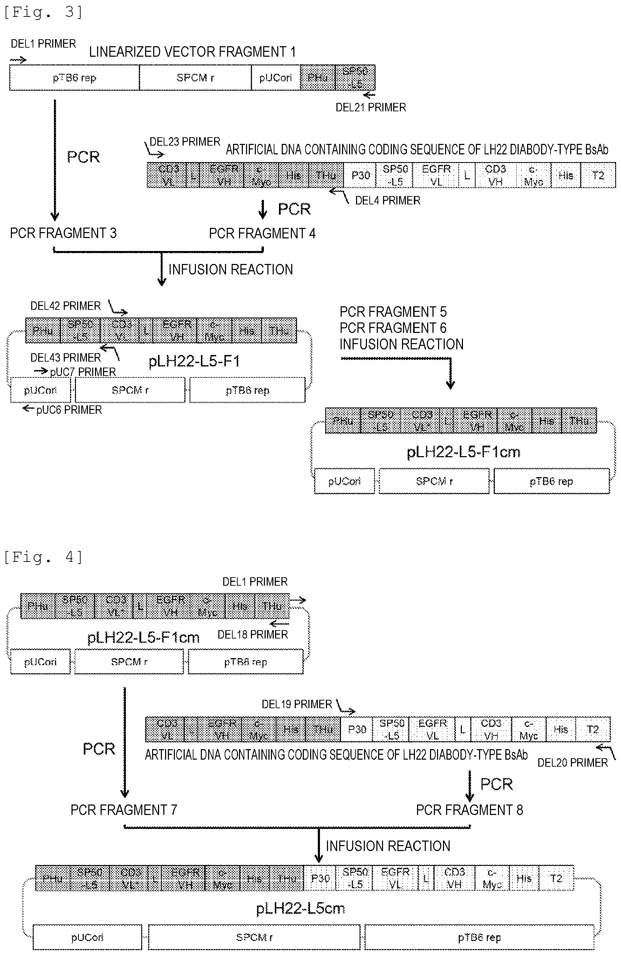

In order to produce *Bifidobacterium* expressing/secreting LH22 diabody-type BsAb in B bacterium, plasmid pLH22-L5cm was produced according to the procedure shown in Figures 3 and 4. Specifically, PCR was first performed using the linearized vector fragment 1 as a template and a primer set of DEL1 primer and DEL21 primer, to produce PCR fragment 3, and PCR was performed using an artificial DNA containing the coding sequence of the LH22 diabody-type BsAb as a template and a primer set of DEL23 primer and DEL4 primer, to produce PCR fragment 4. Thereafter, the infusion reaction was performed using PCR fragments 3 and 4, and Escherichia coli transformants carrying plasmid pLH22-L5-F1 were produced according to the method described in section "1-2-3" above. Thereafter, the plasmid pLH22-L5-F1 was isolated/purified (see Figure 3 and Table 7). Thereafter, since a region highly homological to the nucleotide sequence encoding LH22 polypeptide 2 was present in the nucleotide sequence encoding VL derived from anti-human CD3 antibody of the plasmid pLH22-L5-F1, a silent mutation (substitution of nucleotide residues without changing the VL amino acid sequence derived from anti-human CD3 antibody) was introduced. Specifically, PCR was performed using the plasmid pLH22-L5-F1 as a template and a primer set for introduction of mutation of DEL43 primer and pUC6 primer and a primer set for introduction of mutation of DEL42 primer and pUC7 primer, to produce PCR fragments 5 and 6. Thereafter, the infusion reaction was performed using these fragments, and Escherichia coli transformants carrying plasmid pLH22-L5-F1cm was produced according to the method described in section "1-2-3" above. Thereafter, the plasmid pLH22-L5-F1cm was isolated/purified (see Figure 3 and Table 7). Then, PCR was performed using the plasmid pLH22-L5-F1cm as a template and a primer set of DEL1 primer and DEL18 primer, to produce PCR fragment 7, and PCR was performed using an artificial DNA containing the coding sequence of the LH22 diabody-type BsAb as a template and a primer set of DEL19 primer and DEL20 primer, to produce PCR fragment 8. Thereafter, the infusion reaction was performed using PCR fragments 7 and 8, and Escherichia coli transformants carrying the plasmid pLH22-L5cm was produced according to the method described in section "1-2-3" above. Then, the plasmid pLH22-L5cm was isolated/purified (see Figure 4 and Table 7). The PCR reaction and the infusion reaction were performed respectively according to the methods described in sections "1-2-1" and "1-2-2" above.

1-4: Production of plasmid pLH31-L5

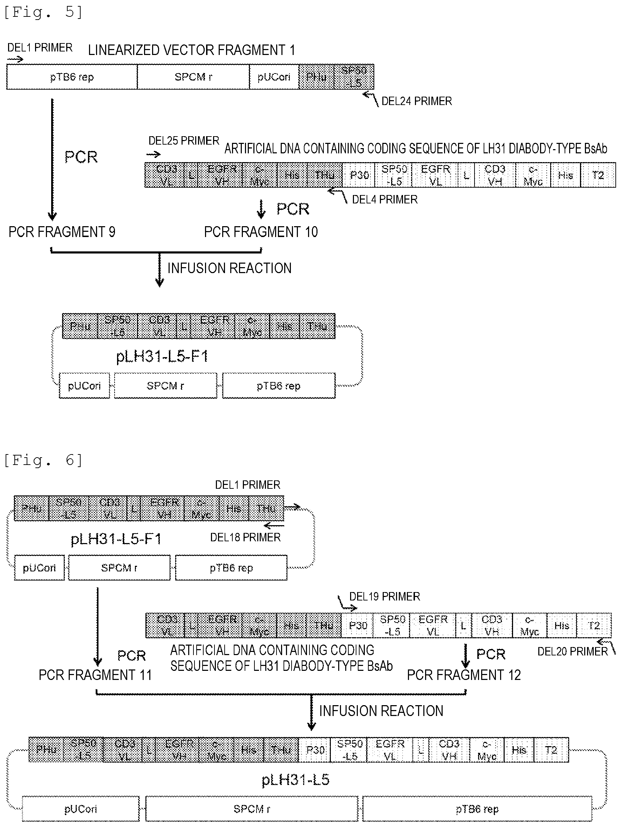

In order to produce *Bifidobacterium* expressing/secreting the LH31 diabody-type BsAb in B bacterium, plasmid pLH31-L5 was produced according to the procedures shown in Figures 5 and 6. Specifically, PCR was first performed using a linearized vector fragment 1 as a template and a primer set of DEL1 primer and DEL24 primer, to produce PCR fragment 9, and PCR was performed using an artificial DNA containing the coding sequence of the LH31 diabody-type BsAb as a template and a primer set of DEL25 primer and DEL4 primer, to produce PCR fragment 10. Thereafter, the infusion reaction was performed using PCR fragments 9 and 10, to produce Escherichia coli transformants carrying plasmid pLH31-L5-F1 according to the method described in section "1-2-3" above. Thereafter, the plasmid pLH31-L5-F1 was isolated/purified (see Figure 5 and Table 7). Then, PCR was performed using the plasmid pLH31-L5-F1 as a template and a primer set of DEL1 primer and DEL18 primer, to produce PCR fragment 11, and PCR was performed using an artificial DNA containing the coding sequence of the LH31 diabody-type BsAb as a template and a primer set of DEL19 primer and DEL20 primer, to produce PCR fragment 12. Thereafter, the infusion reaction was performed using PCR fragments 11 and 12, to produce Escherichia coli transformants carrying the plasmid pLH31-L5 according to the method described in section "1-2-3" above. Thereafter, the plasmid pLH31-L5 was isolated/purified (see Figure 6 and Table 7) . The PCR reaction and the infusion reaction were performed respectively according to the methods described in sections "1-2-1" and "1-2-2" above.

**[Table 7]**

| Primer name | Primer sequence (5'->3') |
|---|---|
| DEL1 primer | ACTAGTcctccaggacctc |
| DEL2 primer | GGCATACGCCGTATTCGC |
| DEL4 primer | tcctggaggACTAGTCCGGAATAATACGGTTCG |
| DEL5 primer | GCTAGGATCCgtagaaaagatcaaaggatc |
| DEL6 primer | tctacGGATCCTAGCCGGCATTTTCC-CGATACATTCCC |
| DEL7 primer | GGCGTAGGCGTGTTGGC |
| DEL9 primer | tcctgcaggACTAGTTATAAACGCACAAAGGCCCACCC |
| DEL18 primer | CCGGAATAATACGGTTGG |
| DEL19 primer | ACCGTATTATTGCGGTAGCCCGCATTTTCGCCATACATTCCCC |
| DEL20 primer | tcctggaggACTAGTTATAAACGCAGAAAGGCCCACCCG |
| DEL21 primer | GGGCGTCAACGTCGCGGCATACG |
| DEL22 primer | GCGACGTTGACGCCCGACATCGTGCTGACCCAG |
| DEL23 primer | GCGACGTTGACGCCCGACATCCAGATGACCCAGTCCCCGTC |
| DEL24 primer | AGGCGGACACCGAGGACGCGGCACTCCGTCATCTGCATGTCGGGCGTCAA CGTCGCGGCATACG CCTCCCTGTCCGCCTCCGTGGGCGACCGTGTGACCATCACCTGCCGTGC CTCCCAGGACATCCG |
| DEL25 primer | CCTCCCTGTCCGCCTCCGTGGGCGACCGTGTGACCATCACCTGCCGTGC CTCCCAGGACATCCG |
| DEL42 primer | gCGGCagCGGCagCGGCACgGACTACACCTTCACCATC |
| DEL43 primer | CGctGCCGctGCCGctGAAgCGcGACGGCACGCCGGAGGCCAG |
| DEL63 primer | TGAACTGGTACCAGgAGAAGCCGGGCAAGGCCCCGAAGC |
| DEL64 primer | CCTcACGCACCCAGTGAATCCAGTCG |
| DEL65 primer | ACTGGGTGGGTgAGGCCCCGGGCAACGGCCTC |
| DEL66 primer | cCTGGTACCAGTTCAGGTAGTTGC |
| DEL67 primer | GTGGCCTGGTACCAGaAGAAGCCGGGCAAGGCCCCGAAGC |
| DEL68 primer | CCTtACGCACCCAGTTGATGGTGTAGCCGGTGAAGGAGTAGC |
| DEL69 primer | ACTGGGTGCGTaAGGCCCCGGGCAAGGGCCTGGAG |
| DEL73 primer | AATACGGCGTATGCCGCGACGGACATCCAGATGACCCAGTCCC |
| DEL74 primer | AACACGGCCTAGGCCGCCACCGACATCCAGATGACCCAGTCCC |
| DEL82 primer | CTGGTACCAGGGCACGTCGGTGGCCAGGTCCTGGGAG |
| DEL140 primer | GTATGCCGCGACGTTGACGCC |
| DEL143 primer | AACGTCGCGGCATACGCCGTATTCGC |
| DEL240 primer | GACATCCAGATGACCCAGTCCC |
| DEL306 primer | CCTCATCTGGATGTCatcggcGGCCATTGCGGTCGGCGCAAGG |
| DEL307 primer | GGTCATCTGGATGTCggtggctgaatcggcGGC |
| DEL314-2 primer | GACATCCAGATGACCCAGTCCCCGTC |
| DEL319 primer | accgtattattccggCGTCTATTTTCATACCCCCTTCG |
| DEL321 primer | GTCGACtcgattttcgttcgtgaatacatg |
| DEL322 primer | ACTAGTTATAAACGCAGAAAGGCCCACCCC |
| DEL323 primer | GCGTTTATAACTAGTCGAAGGGCGCTGGAGGTGCTG |
| DEL324 primer | gaaaatcgaGTCGACGTGCTGGAGCAACAACCCGAGTG |
| DEL330 primer | GGTCATCTGGATGTCGGTGGCGGCGTAGGC |
| DEL331 primer | CCGGACTAGTCGAAGC-GCGCTGGAGGT |
| DEL380 primer | GGATCCgtagaaaagatcaaaggatcttcttgagatcctttttttctgc gcgtaatc |
| DEL381 primer | AGCAGAAGGTCATTAC-GCGGCGGCGGAGGACACGGTCAC |
| DEL382 primer | TAATGACCTTCTGCTCGTAGCGATTACTTCGAGC |
| DEL383 primer | ACAACATGGACTAAGCAAAAGTGC |
| DEL384 primer | CTTAGTCCATGTTGTgtagaaaagatcaaaggatcttcttgagatcc |
| DEL385 primer | TAATGACCAGGCATCAAATAAAACGAAAGGCTCAGTCG |
| DEL386 primer | GATGCCTGGTCATTAGGCGGCGGCGGAGGACACGGTCAC |
| DEL391 primer | GCCCCACATGGCAAACCGAGAACCCGCAC |
| DEL392 primer | TTTGCCATGTGGGGCgtagaaaagatcaaaggatcttcttgagatcc |
| DEL394 primer | GCCGCCGCCTAATGAACAACATGGACTAAGCAAAAGTGC |
| DEL395 primer | TCATTAGGCGGCGGCGGAGGACACGGTCAC |
| DEL396 primer | ACTAGTCGAAGGGCGCTGGAGG |
| DEL397 primer | CGCCCTTCGACTAGTTATAAACGCAGAAAGGCCCACCC |
| DEL400 primer | GCCGCCGCCTAATGAGCCCCACATGGCAAACC |
| GA88 primer | cttcgACTAGTccggaataatacgg |
| Hu ins F1 | ttgatcttttctacCGTCTATTTTCATACCCC |
| Hu8 primer | cttttctacGGATCCCGTCTATTTTCATACCCC |
| P30_R1 primer | aatggctctccttgtaatgctagg |
| PC2 primer | ccggaataatacggttggac |
| pUC_ori_Vec_R4 | gtagaaaagatcaaaggatcttcttgagatcctttttttctgcgcg |
| pUC6 primer | gctgtagGtatctcagttcggtgtagg |
| pUC7 primer | tgagataCctacagcgtgagctatgagaaagcgc |
| SP52-ins_F2 primer | acaaggagagccattATGAGTTTCCATGTATCCGCG |
| Phu_R1 primer | aaagcatccttcttgggtcag |
| SP50-ins_F1 primer | caagaaggatgctttatgatcgtggcctacccg |
| DEL300 primer | cgacgtgccggactacgccTAATGACCTTCTGCTCGTAGCGATTACTTC GAGC |
| DEL301 primer | tagtccggcacgtcgtacgggtaGGCGGCGGCGGAGGACACGGTCACC |
| DEL302 primer | aggacgacgacgacaagTAATGACCAGGCATCAAATAAAACGAAAGGCT CAGTCG |
| DEL303 primer | tgtcgtcgtcgtccttgtagtcGGAGCCGCGCATGCCGCCGCCCAGG |
| DEL346 primer | GCTTGTCCCCTGACCCAAGAAGGATGCTTTATGAGTTTCCATGTATCCG CGCAATCG |
| DEL421 primer | GGTCAGGGGACAAGCACTTTTGCTTAGTCCATGTTGTTCATTAggcgta gtccggcacgtcgtacg |

### 1-5: Production of FLM-9H strain, FOM-22H strain, and DUM-31H strain

The three types of plasmids isolated/purified (pLH9-L5, pLH22-L5cm, and pLH31-L5) were transformed into *Bifidobacterium longum* 105-A strains (sold by Yasumasa Kano, former Professor at Kyoto Pharmaceutical University) using an electroporation system (Gene Pulser II, manufactured by Bio-Rad Laboratories, Inc.). Specifically, an electric shock (at 2 kV, 25 µF, and 200 Ω) was performed, and a mixed solution of 800 µL of an IMR liquid medium and 50 µL of a vitamin C-added solution (a solution containing 350 mg/mL ascorbic acid, 20 mg/mL L-cysteine hydrochloride monohydrate, and 110 mg/mL sodium carbonate) was immediately added to a cuvette (2 mm gap), and the mixture was collected in a sterilized 2-mL microtube. Thereafter, the tube with its cover loosened was put into a closed container together with an oxygen scavenger/a carbon dioxide generator (AnaeroPack (R)/Kenki, manufactured by MITSUBISHI GAS CHEMICAL COMPANY, INC.) and kept warm in an incubator set at 37°C for 3 hours. Each bacterial suspension after being kept warm was applied to an IMR agar medium containing 75 µg/mL spectinomycin. These plates were put into a closed container together with the oxygen scavenger/the carbon dioxide generator, followed by culture in an incubator set at 37°C for 2 days. The colonies formed on the spectinomycin-containing IMR agar medium were caught, drawn on a BL-bS agar medium containing 75 µg/mL spectinomycin, and then put into a closed container together with an oxygen scavenger/a carbon dioxide generator, followed by culture in an incubator set at 37°C for one day, to obtain three types of *Bifidobacterium* transformants, that is, *Bifidobacterium longum* 105-A strain carrying the plasmid pLH9-L5 (FLM-9H strain), *Bifidobacterium longum* 105-A strain carrying the plasmid pLH22-L5cm (FOM-22H strain), and *Bifidobacterium longum* 105-A strain carrying the plasmid pLH31-L5 (DUM-31H strain).

### [Example 2] Evaluation of expression/secretion of FLM-9H strain, FOM-22H strain, and DUM-31H strain (WB)

It was confirmed that the three types of *Bifidobacterium* transformants (FLM-9H strain, FOM-22H strain, and DUM-31H strain) expressed and secreted the EGFR×CD3 diabody-type BsAb, according to the methods described in sections [2-1] and [2-2] below.

### 2-1: Preparation of SDS-sample

The three types of *Bifidobacterium* transformants were inoculated on 10 mL of an MRS liquid medium containing 75 µg/mL spectinomycin and 100 µL of vitamin C-added solution (manufactured by Becton, Dickinson and Company), followed by anaerobic culture at 37°C for 24 hours, to prepare a pre-culture solution. Thereafter, 100 µL of a vitamin C-added solution and spectinomycin were added to 20 mL of a medium obtained by mixing a DMEM liquid medium (Cat No. 11885-084, manufactured by Life Technologies Corporation) and an MRS liquid medium at a ratio of 9:1 to 75 µg/mL, and 100 µL of the pre-culture solution was inoculated thereon, followed by anaerobic culture at 37°C for 18 hours, to prepare the present culture solution. After centrifuging the present culture solution, the culture supernatant was collected, and 10 µL of 5× sample loading buffer (Pierce Lane Marker Reducing Sample Buffer, manufactured by Thermo Fisher SCIENTIFIC K.K.) was mixed with 40 µL of the culture supernatant, followed by heat treatment at 95°C for 3 minutes, to prepare a sample for electrophoresis. The same operation was performed on *Bifidobacterium longum* 105-A strain carrying BE shuttle vector (BE shuttle strain), which was used as a negative control.

### 2-2: Western blotting method (WB)

The sample for electrophoresis was electrophoresed on a Mini-PROTEAN (R) TGX gel (4 to 20%) (manufactured by Bio-Rad Laboratories, Inc.), the gel after electrophoresis was transferred to PVDF membrane (Trans-Blot Turbo Mini Format, manufactured by Bio-Rad Laboratories, Inc.) using Trans-Blot Turbo (manufactured by Bio-Rad Laboratories, Inc.). The PVDF membrane after transcription was blocked using a blocking solution (Blocking One, manufactured by NACALAI TESQUE, INC.), followed by a primary antibody reaction treatment using mouse anti-His tag antibody (manufactured by GenScript Japan Inc.) and a secondary antibody reaction treatment using ECL-peroxidase-labeled anti-mouse antibody (manufactured by GE Healthcare Japan Corporation), to emit light using Western Lightning Ultra (manufactured by PerkinElmer, Inc.). This was analyzed using an imaging analyzer (myECL Imager, manufactured by Thermo Fisher SCIENTIFIC K.K.).

### (Result)

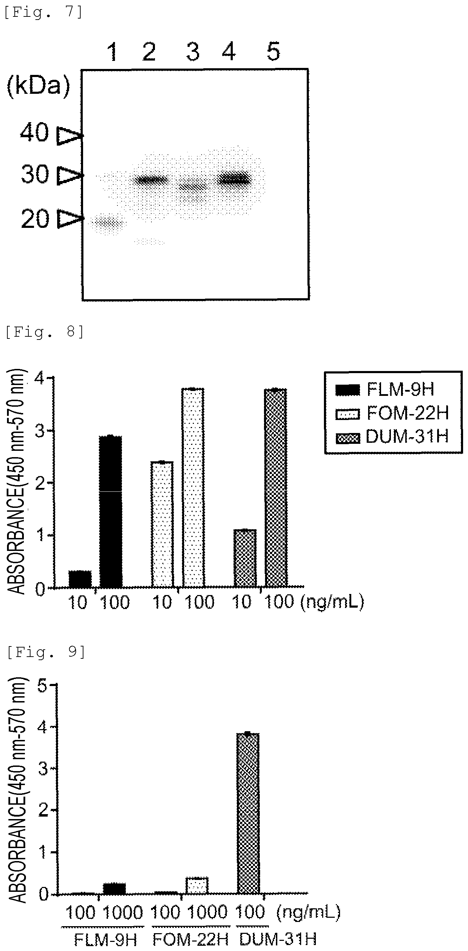

In all the culture supernatants of the three types of *Bifidobacterium* transformants, two bands derived from the polypeptides 1 and 2 constituting EGFR×CD3 diabody-type BsAb were observed around 30 kDa (see Figure 7). These results indicate that all of the three types of *Bifidobacterium* transformants (FLM-9H strain, FOM-22H strain, and DUM-31H strain) express and secrete EGFR×CD3 diabody-type BsAbs (i.e., LH9 diabody-type BsAb, LH22 diabody-type BsAb, and LH31 diabody-type BsAb, respectively).

### [Example 3] Evaluation of binding activity of EGFR×CD3 diabody-type BsAbs derived from FLM-9H strain, FOM-22H strain, and DUM-31H strain to EGFR/CD3

It was confirmed that the three types of EGFR×CD3 diabody-type BsAbs (LH9 diabody-type BsAb, LH22 diabody-type BsAb, and LH31 diabody-type BsAb) bound to EGFR and CD3, according to the methods described in sections [3-1] and [3-2] below.

### 3-1: Purification of EGFR×CD3 diabody-type BsAb

The three types of *Bifidobacterium* transformants were cultured according to the method described in Example 2, to prepare 50 mL of the present culture solution. The present culture solution prepared was centrifuged, and ammonium sulfate was added to 80% saturation little by little to under stirring the culture supernatant obtained, followed by overnight stirring at 4°C for salting out. Then, after centrifugation and collection of the precipitate, the protein (i.e., EGFR×CD3 diabody-type BsAb) was purified by a histidine tag fusion protein purification kit (TALON Methal Affinity Resins, manufactured by Takara Bio Inc.) and concentrated by ultrafiltration (Amicon Ultra-0.5, nominal fraction molecular weight: 10 KDa, manufactured by Merck KGaA). The EGFR×CD3 diabody-type BsAbs were calculated based on the absorbance at 280 nm (A280 value) measured by a spectrophotometer (NanoDrop2000c, manufactured by Thermo Fisher SCIENTIFIC K.K.), and the absorbance coefficients of the diabody-type BsAbs (LH9 diabody-type BsAb Abs 0.1%: 1.914, LH22 diabody-type BsAb Abs 0.1%: 1.851, and LH31 diabody-type BsAb Abs 0.1%: 1.793) were adjusted to 10 (ng/mL), 100 (ng/mL), and 1000 (ng/mL). The A280 value of Abs 0.1% was calculated using the ProtParam tool (https://web.expasy.org/protparam/) of the gene analysis site ExPASY.

### 3-2: ELISA method

### 3-2-1: Reaction treatment between antigen and antibody

25 µL of each of a solution containing 2.5 µg/mL human EGFR recombinant protein (manufactured by R&D Systems, Inc.) and a solution containing 5 µg/mL human CD3E/CD3D heterodimer recombinant protein (manufactured by ACROBiosystems) was dispensed into each well of a 96-well plate, followed by standing overnight at 4°C, for immobilization. The solution after immobilization was removed, and each well was washed with 200 µL of PBS (pH 7.4) containing 0.05% Tween20 (MP Biomedical) (which may be hereinafter referred to as "PBS-T") 3 times. Thereafter, 200 µL of a blocking solution (Blocking One, manufactured by NACALAI TESQUE, INC.) diluted 5-fold with distilled water was dispensed, followed by standing at 25°C for 2 hours, for blocking treatment. The blocking solution in each well was removed, and the well was washed with 200 µL of PBS-T 3 times. A solution containing 10 (ng/mL), 100 (ng/mL), or 1000 (ng/mL) EGFR×CD3 diabody-type BsAb was prepared using a signal enhancer HIKARIA solution containing 1% BSA (manufactured by NACALAI TESQUE, INC.), and 25 µL of the solution was dispensed into each well after blocking, followed by standing at 25°C for 2 hours, for the reaction treatment between the antibody (EGFR×CD3 diabody-type BsAb) and the antigen (immobilized human EGFR recombinant protein and immobilized human CD3E/CD3D heterodimer recombinant protein). Thereafter, the solution in each well was removed, and the well was washed with 200 µL of PBS-T 3 times.

### 3-2-2: Detection of antibody

25 µL of 0.1 µg/mL biotin-labeled anti-His tag antibody (manufactured by MEDICAL & BIOLOGICAL LABORATORIES CO., LTD.) was dispensed into each well subjected to the reaction treatment between the antigen and the antibody, followed by standing at 25°C for 1 hour. The solution in each well was removed, and the well was washed with 200 µL of PBS-T 3 times. VECTASTAIN Elite ABC reagent (avidin-biotin-labeled HRP [Horseradish Peroxidase]) (manufactured by Vector Laboratories) was adjusted to 4 µL/mL using a signal enhancer HIKARI B solution (manufactured by NACALAI TESQUE, INC.) containing 1% BSA, and then 25 µL thereof was dispensed into each well, followed by standing at 25°C for 30 minutes. Thereafter, the solution in each well was removed, and the well was washed with 200 µL of PBS-T 3 times. After mixing equal amounts of Color Solution A and Color Solution B (solutions containing TMB [3,3', 5,5'-Tetramethylbenzidine] as a substrate of HRP) (manufactured by R&D Systems, Inc.) as detection reagents, 50 µL thereof was dispensed into each well, and light was blocked, followed by standing at room temperature for 20 minutes. Thereafter, 25 µL of Stop Solution (manufactured by R&D Systems, Inc.) was added thereto, to stop the color reaction. The absorbance at 450 nm as the absorption wavelength of TMB-derived pigments was measured, and the value corrected with the absorbance at 570 nm (negative control) (average ± standard deviation, see the vertical axes of Figures 8 and 9) was calculated.

### (Result)

It was indicated that the corrected value of absorbance increased depending on the concentrations of the three types of EGFR×CD3 diabody-type BsAbs, even in the case of using any of the immobilized human EGFR recombinant protein and the human CD3E/CD3D heterodimer recombinant protein (see Figures 8 and 9). These results indicate that the three types of EGFR×CD3 diabody-type BsAbs (LH9 diabody-type BsAb, LH22 diabody-type BsAb, and LH31 diabody-type BsAb) expressed/secreted by the *Bifidobacterium* transformants bind specifically to both human EGFR and human CD3.

### [Example 4] Evaluation of cytotoxic activity of EGFR×CD3 diabody-type BsAbs purified from culture supernatants of FLM-9H strain, FOM-22H strain, and DUM-31H strain

It was confirmed that the three types of EGFR×CD3 diabody-type BsAbs had cytotoxic activity, according to the methods described in sections [4-1] and [4-2] below.

### 4-1: Preparation of T-LAKs

T-LAKs (activated T lymphocytes) used for cytotoxic activity measurement were produced from human peripheral blood mononuclear cells (PBMC) . Specifically, 10 µg/mL anti-human CD3 antibody (manufactured by BioLegend, Inc.) was immobilized at 37°C in a 25 cm² flask and washed with PBS (-), and then human PBMCs suspended in an RPMI-1640 medium containing 10% deactivated FBS were seeded with medium exchange in the presence of 140 U/mL IL-2 every 3 to 4 days, followed by culture for 2 weeks, to prepare T-LAKs. T-LAKs were stored in a liquid nitrogen gas phase and suspended in an RPMI-1640 medium containing 10% deactivated FBS when used for cytotoxic activity measurement, followed by culture for one week in the presence of 140 U/mL IL-2.

### 4-2: Method for measuring cytotoxic activity

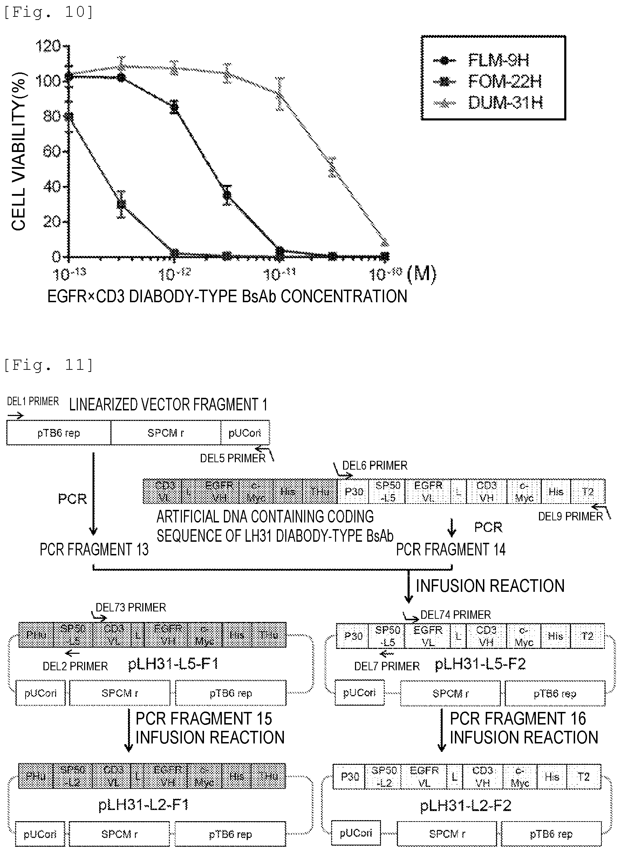

HCT116 cells as a human colon cancer cell line at 2×10⁴ cells/100 µL/well (obtained from ATCC [American Type Culture. Collection]) were seeded on a 96-well plate. 24 hours later, T-LAKs were mixed to a T-LAK:HCT116 cell ratio of 5:1. At the same time, the three types of EGFR×CD3 diabody-type BsAbs purified according to the method described in section "3-1" above serially diluted to concentrations of 100 fM (10⁻¹³ M) to 100 pM (10⁻¹⁰ M) were added thereto and adjusted so that the amount of medium was 100 µL in total, followed by culture for 24 hours. Thereafter, each well was washed with PBS (-) 3 times, and 120 µL of an RPMI-1640 medium containing CellTiter 96 (R) AQueous One Solution Cell Proliferation Assay (manufactured by Promega) was added thereto, followed by incubation at 37°C and 5%CO₂ for 10 minutes. Thereafter, the absorbance at 490 nm was measured, and the absorbance at 660 nm was measured as a negative control wavelength. The viability of HCT116 cells (average ± standard deviation, see the vertical axis of Figure 10) was calculated with the measured value of Blank well taken as a cell viability of 0% and the measured value of a well on which T-LAK-free HCT116 cells were seeded taken as a cell viability of 100%.

### (Result)

It was indicated that the viability of HCT116 cells as a human colon cancer cell line decreased depending on the concentrations of the three types of EGFR×CD3 diabody-type BsAbs (see Figure 10) . These results indicate that the three types of EGFR×CD3 diabody-type BsAbs (LH9 diabody-type BsAb, LH22 diabody-type BsAb, and LH31 diabody-type BsAb) expressed/secreted by the *Bifidobacterium* transformants have cytotoxic activity on the cancer cells. The EC₅₀ values of the EGFR×CD3 diabody-type BsAbs (i.e., the concentrations of the EGFR×CD3 diabody-type BsAbs that reduced the viability of HCT116 cells to 50%) were 2.2 pM in the case of LH9 diabody-type BsAb, 0.25 pM in the case of LH22 diabody-type BsAb, and 32 pM in the case of LH31 diabody-type BsAb.

### [Example 5] Production of Bifidobacterium (DUM-126H strain) expressing/secreting EGFR×CD3 diabody-type BsAb

Separately from the *Bifidobacterium* transformants produced in Example 1, a *Bifidobacterium* expressing/secreting human EGFR×human CD3 diabody-type BsAb was produced according to the methods described in sections [5-1] to [5-2] below.

### 5-1: Production of plasmid p126

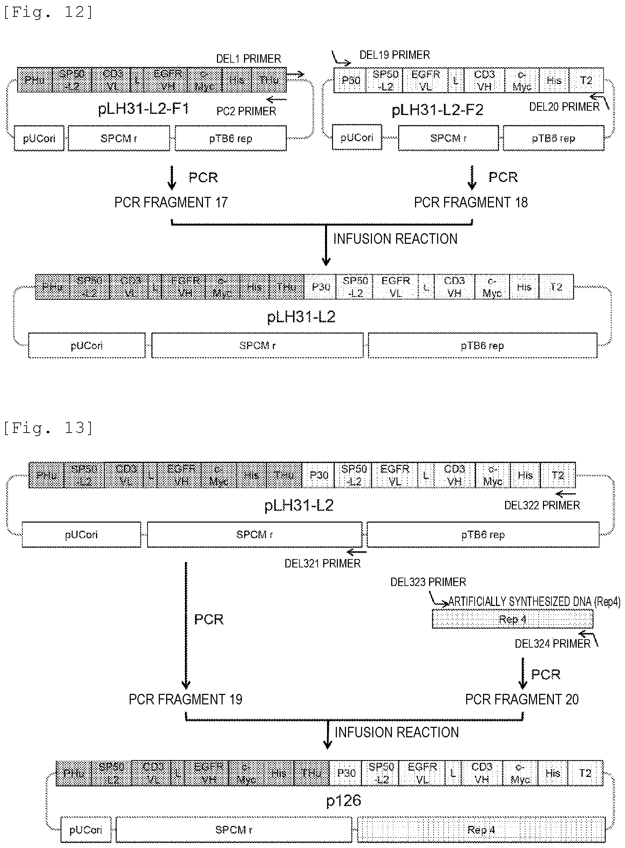

A plasmid (plasmid p126) in which pTB6 as a plasmid replication unit in the *Bifidobacterium* was changed into Rep4 (the nucleotide sequence of SEQ ID No: 12) in the plasmid pLH31-L5 produced in Example 1 (see Figure 6) and which expressed LH31 polypeptide 1 and LH31 polypeptide 2 with SP50-L2 (in which the length of the linker peptide in SP50-L5 was shortened from 5 amino acid residues to 2 amino acid residues; the amino acid sequence of SEQ ID No: 13) added at the N-terminus instead of LH31 polypeptide 1 and LH31 polypeptide 2 with SP50-L5 added at the N-terminus (see Figure 13) was produced according to the procedures shown in Figures 11 to 13 by the same method as in Example 1. Table 7 shows the primer sets used for PCR.

### 5-2: Production of DUM-126H strain

The transformation into *Bifidobacterium longum* 105-A strain using the plasmid p126 was performed by the same method as in Example 1, to obtain *Bifidobacterium longum* 105-A strain carrying plasmid p126 (DUM-126H strain).

### [Example 6] Evaluation (WB) of expression/secretion of DUM-126H strain

In order to confirm that *Bifidobacterium* transformants (DUM-126H strain) expressed and secreted the EGFR×CD3 diabody-type BsAb, Western blotting method using the DUM-126H strain culture supernatant was performed according to the method described in Example 2.

### (Result)

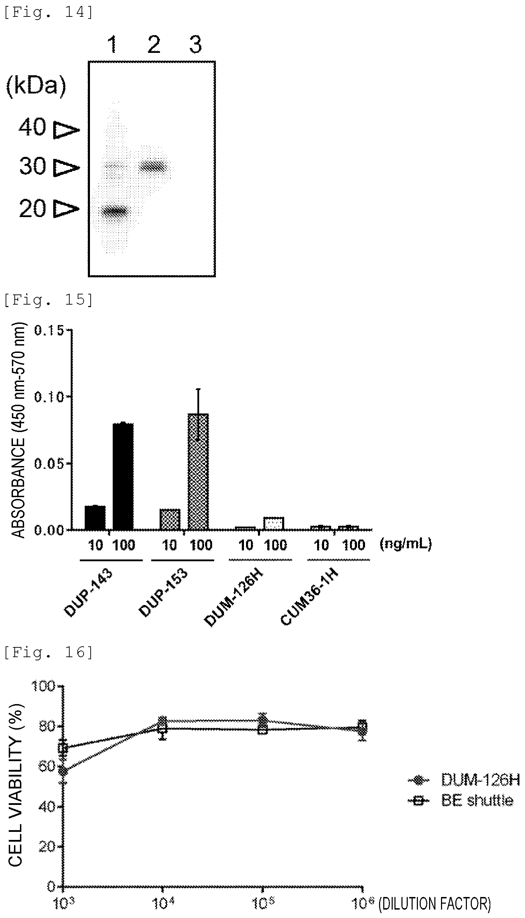

Two bands derived from LH31 polypeptides 1 and 2 constituting the EGFR×CD3 diabody-type BsAb were observed around 30 kDa in the culture supernatant of DUM-126H strain (see Figure 14) . This result indicates that *Bifidobacterium* transformants (DUM-126H strain) express and secrete the EGFR×CD3 diabody-type BsAb (i.e., LH31 diabody-type BsAb).

### [Example 7] Evaluation of binding activity of EGFR×CD3 diabody-type BsAb derived from DUM-126H strain to CD3

It was confirmed that the EGFR×CD3 diabody-type BsAb secreted by the *Bifidobacterium* transformants (DUM-126H strain) specifically bound to both human EGFR and human CD3, according to the methods described in sections [7-1] and [7-2] below.

### 7-1: Purification of EGFR×CD3 diabody-type BsAb

The EGFR×CD3 diabody-type BsAb secreted by the *Bifidobacterium* transformants (DUM-126H strain) was purified from the culture supernatant of DUM-126H strain according to a method using a protein L resin having a high affinity with κ-light chain (Piercetm Protein L Agarose, manufactured by Thermo Fisher Scientific K.K., cat No. 20510) instead of the purification kit of the histidine tag fusion protein in the method described in section "3-1" of Example 3. Further, CUM-36-1H strain-derived HER2×CD3 diabody-type BsAb purified in Example 21, which will be described below, was used as a negative control.

### 7-2: ELISA method

### 7-2-1: Reaction treatment between antigen and antibody

The reaction treatment between the antibody (LH31 diabody-type BsAb) and the antigen (immobilized human CD3E/CD3D heterodimer recombinant protein) was performed according to the method described in section "3-2-1" of Example 3.

### 7-2-2: Detection of antibody

25 µL of a signal enhancer HIKARI B solution (manufactured by NACALAI TESQUE, INC.) containing 0.1 µg/mL recombinant biotinylated human EGFR (manufactured by ACROBiosystems) was dispensed into each well subjected to the reaction treatment between the antigen and the antibody, followed by standing at 25°C for 1 hour. The solution in each well was removed, and the well was washed with 200 µL of PBS-T 3 times. VECTASTAIN Elite ABC reagent (avidin-biotin-labeled HRP) (manufactured by Vector Laboratories) was adjusted to 4 µL/mL using a signal enhancer HIKARI B solution (manufactured by NACALAI TESQUE, INC.) containing 1% BSA, and then 25 µL thereof was dispensed into each well, followed by standing at 25°C for 30 minutes. Thereafter, the solution in each well was removed, and the well was washed with 200 µL of PBS-T 3 times. After mixing equal amounts of Color Solution A and Color Solution B (solutions containing TMB as a substrate of HRP) (manufactured by R&D Systems, Inc.) as detection reagents, 50 µL thereof was dispensed into each well, and light was blocked, followed by standing at room temperature for 20 minutes. Thereafter, 25 µL of Stop Solution (manufactured by R&D Systems, Inc.) was added thereto, to stop the color reaction. The absorbance at 450 nm as the absorption wavelength of TMB-derived pigments was measured, and the value corrected with the absorbance at 570 nm (negative control) (average ± standard deviation, see the vertical axis of Figure 15) was calculated.

### (Result)

It was indicated that the corrected value of absorbance increased depending on the concentration of DUM-126H strain-derived EGFR×CD3 diabody-type BsAb (see "DUM-126H" in Figure 15). Meanwhile, no increase was observed in corrected value of absorbance in CUM-36-1H strain-derived HER2×CD3 diabody-type BsAb (see "CUM36-1H" in Figure 15). These results indicate that the EGFR×CD3 diabody-type BsAb secreted by the *Bifidobacterium* transformants (DUM-126H strain) (i.e., LH31 diabody-type BsAb) specifically binds to both human CD3 and human EGFR.

### [Example 8] Amount of EGFR×CD3 diabody-type BsAb in DUM-126H strain culture supernatant

The amount of EGFR×CD3 diabody-type BsAb in the culture supernatant of *Bifidobacterium* transformants (DUM-126H strain) was measured according to the methods described in sections [8-1] and [8-2] below.

### 8-1: Reaction treatment between antigen and antibody

25 µL of 2.5 µg/mL human CD3E/CD3D heterodimer recombinant protein (manufactured by ACROBiosystems) was dispensed into each well of a 96-well plate, followed by standing overnight at 4°C, for immobilization. Each well was washed with 200 µL of PBS-T 3 times. Thereafter, 200 µL of a blocking solution (Blocking One, manufactured by NACALAI TESQUE, INC.) diluted 5-fold with distilled water was dispensed, followed by standing at 25°C for 2 hours, for blocking treatment. The blocking solution in each well was removed, and the well was washed with 200 µL of PBS-T 3 times. The culture supernatants of DUM-126H strain prepared in Example 6 and DUP-153 strain-derived GFR×CD3 diabody-type BsAb with a known concentration prepared in Example 13, which will be described below, were each diluted 500-fold with a signal enhancer HIKARIA solution (manufactured by NACALAI TESQUE, INC.) containing 1% BSA, and 25 µL of the solution was dispensed into each well after blocking, followed by standing at 25°C for 2 hours, for the reaction treatment between the antibody (EGFR×CD3 diabody-type BsAb) and the antigen (immobilized human CD3E/CD3D heterodimer recombinant protein). Thereafter, the solution in each well was removed, and the well was washed with 200 µL of PBS-T 3 times.

### 8-2: Detection of antibody

25 µL of a signal enhancer HIKARI B solution (manufactured by NACALAI TESQUE, INC.) containing 0.05 µg/mL recombinant biotinylated protein L (manufactured by Thermo Fisher SCIENTIFIC K.K.) was dispensed into each well subjected to the reaction treatment between the antigen and the antibody, followed by standing at 25°C for 1 hour. The solution in each well was removed, and the well was washed with 200 µL of PBS-T 3 times. VECTASTAIN Elite ABC reagent (avidin-biotin-labeled HRP) (manufactured by Vector Laboratories) was adjusted to 4 µL/mL using a signal enhancer HIKARI B solution (manufactured by NACALAI TESQUE, INC.) containing 1% BSA, and then 25 µL thereof was dispensed into each well, followed by standing at 25°C for 30 minutes. Thereafter, the solution in each well was removed, and the well was washed with 200 µL of PBS-T 3 times. After mixing equal amounts of Color Solution A and Color Solution B (solutions containing TMB as a substrate of HRP) (manufactured by R&D Systems, Inc.) as detection reagents, 50 µL thereof was dispensed into each well, and light was blocked, followed by standing at room temperature for 20 minutes. Thereafter, 25 µL of Stop Solution (manufactured by R&D Systems, Inc.) was added thereto, to stop the color reaction. The absorbance at 450 nm as the absorption wavelength of TMB-derived pigments and the absorbance at 570 nm as the negative control were measured, to plot a calibration curve based on the DUP-153 strain-derived GFR×CD3 diabody-type BsAb with a known concentration. Thereafter, the concentration of the EGFR×CD3 diabody-type BsAb in the culture supernatant of *Bifidobacterium* transformants (DUM-126H strain) was quantified.

### (Result)

It was confirmed that the concentration of the EGFR×CD3 diabody-type BsAb (i.e., LH31 diabody-type BsAb) in the culture supernatant of DUM-126H strain was 909 ± 43 (ng/mL) .

### [Example 9] Cytotoxic activity of EGFR×CD3 diabody-type BsAb purified from DUM-126H strain culture supernatant

The EGFR×CD3 diabody-type BsAb expressed/secreted by *Bifidobacterium* transformants (DUM-126H strain) was purified according to the method described in section "7-1" of Example 7 and serially diluted to a concentration of 100 fM to 100 pM. Thereafter, the viability of HCT116 cells was analyzed according to the method described in Example 4.

### (Result)

The viability of HCT116 cells decreased depending on the concentration of the EGFR×CD3 diabody-type BsAb, and the EC₅₀ value of the EGFR×CD3 diabody-type BsAb was 8.4 pM. These results indicate that the EGFR×CD3 diabody-type BsAb expressed/secreted by the *Bifidobacterium* transformants (i.e., LH31 diabody-type BsAb) has an injury activity on cancer cells.

### [Example 10] Cytotoxic activity of DUM-126H strain culture supernatant

Then, the EGFR×CD3 diabody-type BsAb expressed/secreted by *Bifidobacterium* transformants (DUM-126H strain) was analyzed for cytotoxic activity using the culture supernatant without purification. Specifically, the viability of HCT116 cells was analyzed using the culture supernatant of DUM-126H strain prepared in Example 8 and diluted 10³-fold to 10⁶-fold according to the method described in Example 4. As the negative control, the culture supernatant of BE shuttle strain diluted 10³-fold to 10⁶-fold was analyzed in the same manner.

### (Result)

In the case of using the culture supernatant of BE shuttle strain diluted 10³-fold, the viability of HCT116 cells was 69.2%, whereas in the case of using the culture supernatant of DUM-126H strain diluted 10³-fold, the viability of HCT116 cells was 57.6% (see Figure 16). These results indicate that even in the case of using the culture supernatant of DUM-126H strain, the viability of cancer cells such as HCT116 cells can be reduced.

### [Example 11] In-vivo efficacy by mince implantation containing DUM-126H strain

Cancer-bearing Scid mice having HCT116 cells as a human large intestine cancer cell line were analyzed in vivo for the anti-tumor effect of the EGFR×CD3 diabody-type BsAb secreted by DUM-126H strain. Specifically, the analysis was performed according to the methods described in sections [11-1] to [11-4] below.

### 11-1: Measurement of tumor volume

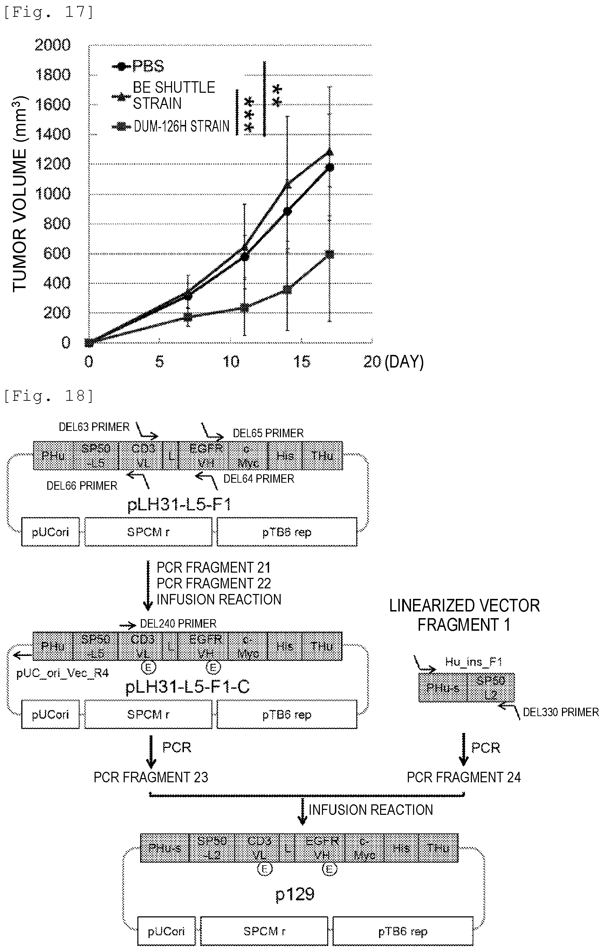

HCT116 cells cultured in McCoy's5A medium (manufactured by Sigma-Aldrich) containing 10% FBS (manufactured by Equitech-Bio Inc.) were implanted into 7 week-old female Scid mice (Japan SLC, Inc.) to produce subcutaneous cancer-bearing mice. Cancer-bearing mice having a tumor size of 447.59 to 517.62 mm³ were divided into three groups of PBS-administered group (12 mice), BE shuttle strain-administered group (14 mice), and DUM-126H strain-administered group (14 mice) (5 days before tumor mince implantation). Thereafter, 0.2 mL of PBS was administered intravenously to the tail veins of the PBS-administered group, a frozen formulation of 5.0 × 10⁸ cfu/0.2 mL of BE shuttle strain was administered intravenously to the tail veins of the BE shuttle strain-administered group, and a simple frozen formulation of 5.0 × 10⁸ cfu/0.2 mL of DUM-126H strain was administered intravenously to the tail veins of the DUM-126H strain-administered group, on day 1 (4 days before tumor mince implantation) and day 2 (3 days before tumor mince implantation). In order to promote the engraftment and growth of *Bifidobacterium* at the tumor site, 1 mL of a 10% maltose solution was intraperitoneally administered twice a day 1 to 4 days after dividing into the three groups (4 days to 1 day before tumor mince implantation), and 0.2 mL of anti-asialo GM1 antibody (manufactured by FUJIFILM Wako Pure Chemical Corporation) diluted 10-fold with PBS (-) was administered intraperitoneally 4 days later (1 day before tumor mince implantation). The anti-asialo GM1 antibody was administered intraperitoneally in the same manner to the Scid mice to be subcutaneously implanted thereafter. 5 days after dividing into the three groups (day 0 after the tumor mince implantation), tumors were extracted from the mice of each group (PBS-administered group, BE shuttle strain-administered group, and DUM-126H strain-administered group) and pooled, and the mince was diluted 1.33-fold with a 1:1 mixed solution of Matrigel (manufactured by Corning Inc.) and HBSS (manufactured by Life Technologies Corporation). After preparation, the T-LAKs cultured were mixed at 0.375 × 10⁸ cells/mL, and Teceleukin (manufactured by SHIONOGI & CO., LTD.) was added to 5000 U/mL, to prepare a mince implantation solution. 0.2 mL of the mince implantation solution was subcutaneously implanted into new 7 week-old female Scid mice, which were divided into a PBS group (14 mice), a BE shuttle strain group (15 mice), and a DUM-126H strain group (15 mice). The number of viable bacteria of *Bifidobacterium* transformants (BE shuttle strain and DUM-126H strain) contained in the tumor mince at the time of implantation was measured according to the method described in section "11-2" below. On day 0 to day 4, day 7 to day 11, and day 14 to day 16 after the tumor mince implantation, 1 mL of a 10% maltose solution was intraperitoneally administered twice a day, and on day 7, day 11, day 14, and day 17 after the tumor mince implantation, the tumor diameter in the mice of each group was measured, to calculate the tumor volume based on the formula "tumor volume = major axis × minor axis²/2" (see Figure 17). The tumor growth inhibition rate [TGI] was calculated based on the formula "TGI = [1 - average tumor volume 17 days after the tumor mince implantation (each recombinant *Bifidobacterium* strain group)/average tumor volume 17 days after the tumor mince implantation (PBS group)] × 100".

### 11-2: Measurement of the number of viable bacteria in tumor mince

To the tumor mince prepared, was added an anaerobic diluent containing a protease inhibitor (manufactured by NACALAI TESQUE, INC.), followed by homogenization, to prepare a homogenate. A portion of the homogenate was fractioned, and the EGFR×CD3 diabody-type BsAb was measured according to the method described in section "11-3" below. The remaining homogenate was appropriately diluted with the anaerobic diluent, and the resultant was then applied to a BLFS agar medium (BL agar medium containing 250 µg/mL 5-fluorouracil and 30 µg/mL spectinomycin), followed by culture at 37°C under anaerobic conditions for 3 days. The number of colonies formed on the BLFS agar medium was counted, and the number of viable bacteria of *Bifidobacterium* transformants (BE shuttle strain and DUM-126H strain) contained in the tumor before preparing the tumor mince or in the implanted tumor mince was calculated (see "the number of viable bacteria in tumor" and "the number of viable bacteria in implanted tumor mince" in Table 8, respectively).

### 11-3: Preparation of tumor sample for ELISA analysis

To the homogenate, were added 10 × Cell Lysis buffer (500 mM Tris-HCl [pH 7.5], 1500 mM sodium chloride, 5% NP-40, and 20 mM EDTA) and 50 × cOmplete (prepared by dissolving one tablet of cOmplete [EDTA-free, manufactured by Roche Diagnostics K.K., Cat#11873580001] in 1 mL of ultrapure water), followed by stirring and then standing on ice for 30 minutes. After centrifugation, the supernatant was collected and diluted with PBS (-). Thereafter, protein L-immobilized beads (manufactured by Thermo Fisher SCIENTIFIC K.K.) were added thereto, followed by overnight reaction under stirring at 4°C. The tumor supernatant solution with magnetic beads added was transferred to a new 2.0 mL tube erected on a magnetic stand, to adsorb the protein L-immobilized beads after the reaction. After the magnetic beads were magnetically adsorbed on the wall surface, the supernatant was removed, and TBS (Tris Buffered Saline) containing 1% Tween20 was added to the same 2.0 mL tube. The magnetic beads were suspended by a vortex mixer, the 2.0 mL tube was erected again on the magnetic stand to adsorb the magnetic beads on the wall surface, and then the washing solution was removed, to wash the magnetic beads. The same washing operation was performed further with TBS containing 1% Tween20 once and with TBS twice. After the final washing, the washing solution was removed, and then the magnetic beads adsorbed were suspended in 15.9 µL of 0.1 M glycine-hydrochloric acid solution (pH 2.0), followed by standing at room temperature for 10 minutes, so that the EGFR×CD3 diabody-type BsAb was eluted. The 2.0 mL tube was erected again on the magnetic stand to adsorb the magnetic beads on the wall surface, and the supernatant was collected into a new 1.5-mL tube, neutralized with 1.59 µL of 1 M Tris-HCl (pH 9.0), and used for the ELISA method described in section "11-4".

### 11-4: ELISA method

25 µL of a solution containing 2.5 µg/mL human CD3E/CD3D heterodimer recombinant protein (manufactured by ACROBiosystems) was dispensed into each well of a 96-well plate, followed by standing overnight at 4°C, for immobilization. Each well was washed with PBS-T, followed by blocking treatment with a blocking solution (Blocking One, manufactured by NACALAI TESQUE, INC.) at 25°C for 2 hours. After each well was washed with PBS-T, the tumor sample prepared in "11-3" above and standard EGFR×CD3 diabody-type BsAb for calibration curve (mutant LH31 diabody-type BsAb) purified from the culture supernatant of DUP-153 strain in Example 13, which will be described below, serially diluted to 16 ng/mL to 0.015625 ng/mL were each incubated at 25°C for 2 hours. Thereafter, each well was washed with PBS-T, followed by incubation at 25°C for 1 hour in the presence of recombinant biotinylated protein L (manufactured by Thermo Fisher SCIENTIFIC K.K.). Each well was washed with PBS-T, followed by incubation at 25°C for 30 minutes in the presence of VECTASTAIN Elite ABC reagent (avidin-biotin-labeled HRP) (manufactured by Vector Laboratories). After each well was washed with PBS-T, the color was developed at room temperature for 20 minutes in the presence of Color Solution A and Color Solution B (a solution containing TMB as a substrate of HRP) (manufactured by R&D Systems, Inc.) as detection reagents, and the color reaction was stopped with a Stop Solution (2 N sulfuric acid) (manufactured by R&D Systems, Inc.). The absorbance at 450 nm as the absorption wavelength of TMB-derived pigments and the absorbance at 570 nm as a negative control were measured using POWERSCAN HT (Gen5 2.04) (manufactured by DS Pharma Biomedical Co., Ltd.), to plot a calibration curve based on the standard EGFR×CD3 diabody-type BsAb for calibration curve. Thereafter, the concentration of the EGFR×CD3 diabody-type BsAb in the tumor mince was quantified (see "EGFR×CD3 diabody-type BsAb concentration in tumor mince" in Table 8).

**[Table 8]**

| Group | The number of viable bacteria in tumor (cfu/g tumor) | The number of viable bacteria in implanted tumor mince (cfu/mouse) | EGFR×CD3 diabody-type BsAb concentration (pM) in tumor mince |
|---|---|---|---|
| PBS group | ND | ND | ND |
| BE shuttle strain group | 7.3×10⁷ | 5.5×10⁶ | ND |
| DUM-126H strain group | 3.1×10⁸ | 2.3×10⁷ | 80.2 |

In the table, "ND" indicates that the result was below the detection sensitivity.

### (Result)

First, it was confirmed that the number of viable bacteria of *Bifidobacterium* transformants (each of BE shuttle strain and DUM-126H strain) was contained in the tumor mince used for implantation in the BE shuttle strain group and the DUM-126H strain group, and DUM-126H strain expressed/secreted the EGFR×CD3 diabody-type BsAb (i.e., LH31 diabody-type BsAb) (see Table 8).

Then, as a result of analyzing the tumor volume in each group with the tumor mince implanted, the tumor volume in the mice of the BE shuttle strain group was almost the same as the tumor volume in the mice of the PBS group as the negative control, whereas the tumor volume in the mice of the DUM-126H strain group was significantly reduced as compared with the tumor volume in the mice of the PBS group as the negative control. For example, the tumor growth inhibition rate [TGI] was 49.5% with respect to the PBS group on day 17 after the tumor mince implantation (see Figure 17). These results indicate that the EGFR×CD3 diabody-type BsAb secreted by the *Bifidobacterium* transformants (DUM-126H strain) has an anti-tumor effect.

### [Example 12] Production of Bifidobacteria (DUP-143 strain and DUP-153 strain) expressing/secreting EGFR×CD3 diabody-type BsAb

Separately from the *Bifidobacterium* transformants produced in Example 1 and Example 5, *Bifidobacteria* expressing/secreting human EGFR×human CD3 diabody-type BsAb were produced according to the methods described in sections [12-1] to [12-4] below.

### 12-1: Production of plasmid p143TLB6a

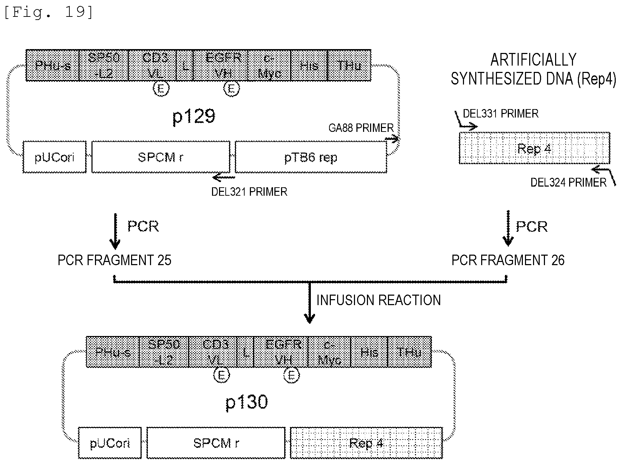

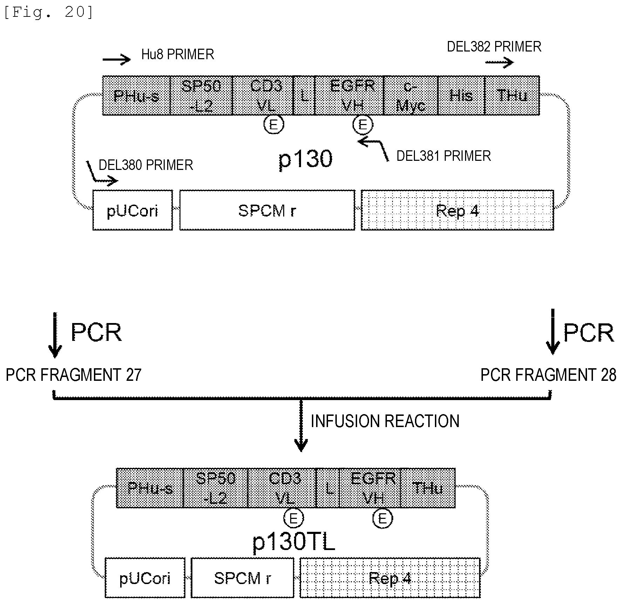

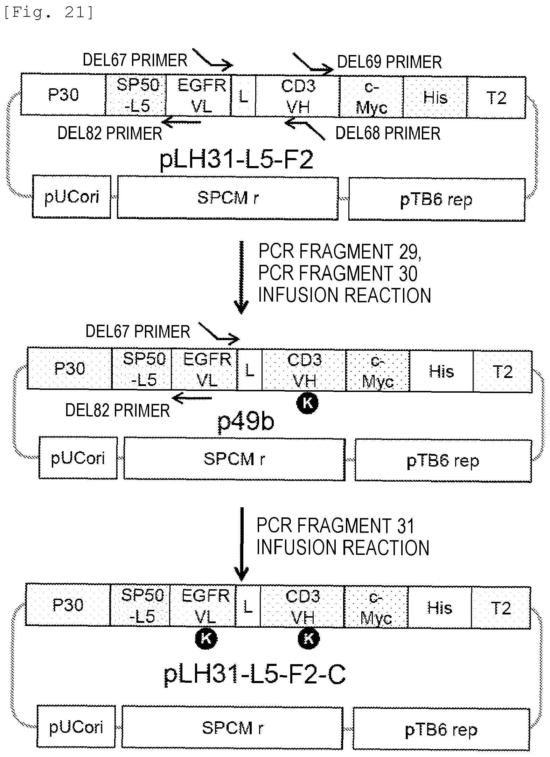

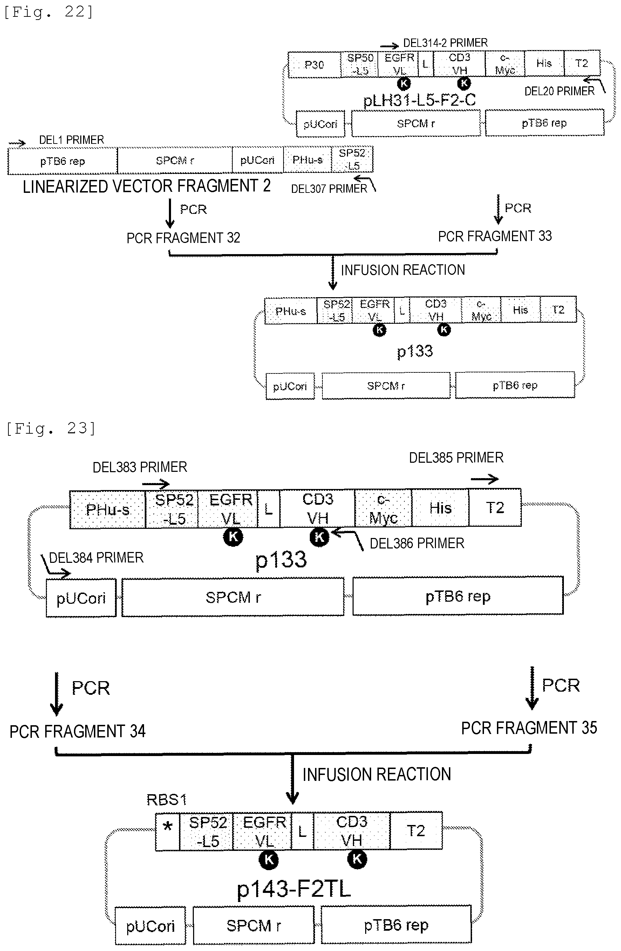

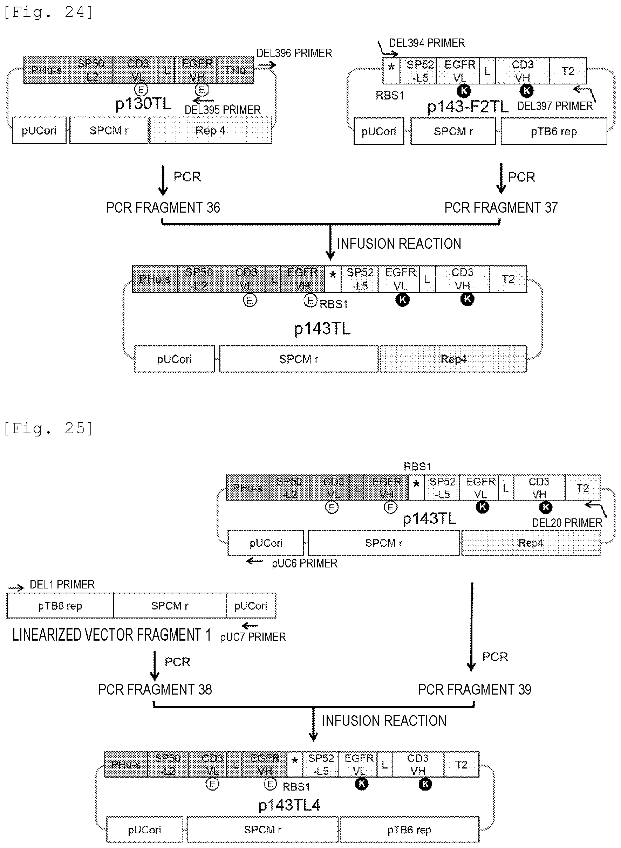

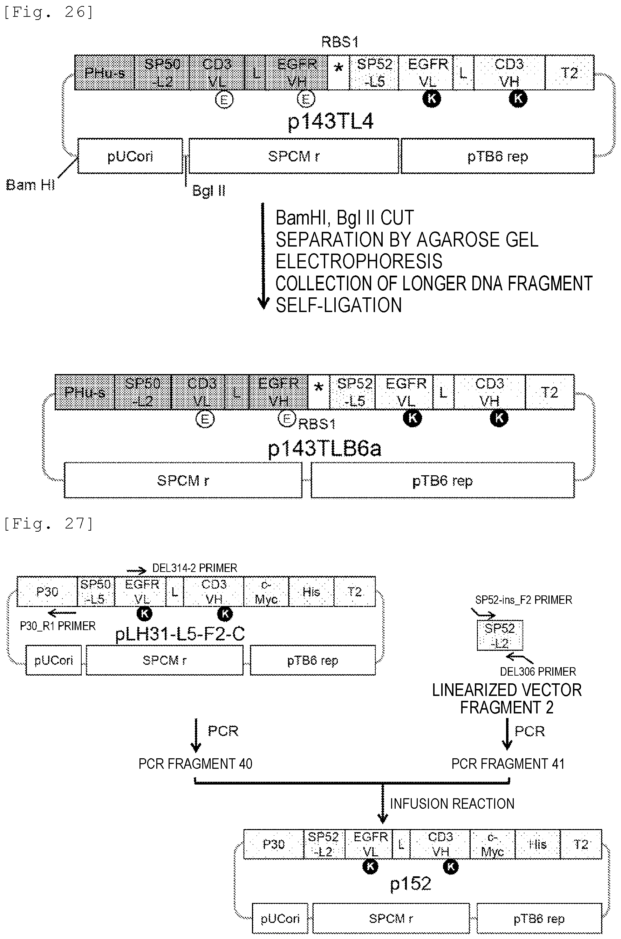

A plasmid (plasmid p143TLB6a) in which Hu promoter (PHu) was changed to Hu-s promoter (PHu-s; fragment consisting of 143 nucleotide residues on the 3' side of PHu) (corresponding to Hu1 in International Publication No. WO 2018/062225), a ribosome-binding site (RBS1 [specifically, a polynucleotide consisting of 52 nucleotide residues upstream of the translation start codon of Hup gene derived from *Bifidobacterium longum* 105-A strain]) for translating LH31 polypeptide 2 was inserted instead of P30 promoter, and the Escherichia coli origin of replication, pUCori, was removed in the plasmid pLH31-L5 (see Figure 6) produced in Example 1, the plasmid expressing: LH31 polypeptide 1 (which may be hereinafter referred to as "mutant LH31 polypeptide 1") in which SP50-L2 was added at the N-terminus instead of SP50-L5, glutamine at Kabat position 38 of the anti-human CD3 antibody VL (i.e., 38th glutamine [Q] of SEQ ID No: 5) was substituted with glutamic acid (E), and Q at Kabat position 39 of the anti-human EGFR antibody VH (i.e., 153rd Q of SEQ ID No: 5) was substituted with E; and LH31 polypeptide 2 (which may be hereinafter referred to as "mutant LH31 polypeptide 2") in which SP52-L5 was added at the N-terminus instead of P50-L5, Q at Kabat position 38 of the anti-human EGFR antibody VL (i.e., 38th Q of SEQ ID No: 6) was substituted with lysine (K), and Q at Kabat position 39 of the anti-human CD3 antibody VH (i.e., 153rd Q of SEQ ID No: 6) was substituted with K (see Figure 26) was produced according to the procedures shown in Figure 18 to Figure 26 by the same method as in Example 1. The plasmid p143TLB6a could polycistronically express mutant LH31 polypeptide 1 and mutant LH31 polypeptide 2 by insertion of RBS1 and could secrete the EGFR×human CD3 diabody-type BsAb (which may be hereinafter referred to as "mutant LH31 diabody-type BsAb") composed of mutant LH31 polypeptide 1 and mutant LH31 polypeptide 2. Table 7 shows the primer sets used for PCR.

### 12-2: Production of DUP-143 strain

The transformation into *Bifidobacterium longum* 105-A strain using the plasmid p143TLB6a was performed by the same method as in Example 1, to obtain *Bifidobacterium longum* 105-A strain (DUP-143 strain) carrying plasmid p143TLB6a.

### 12-3: Production of plasmid p153TLB6a

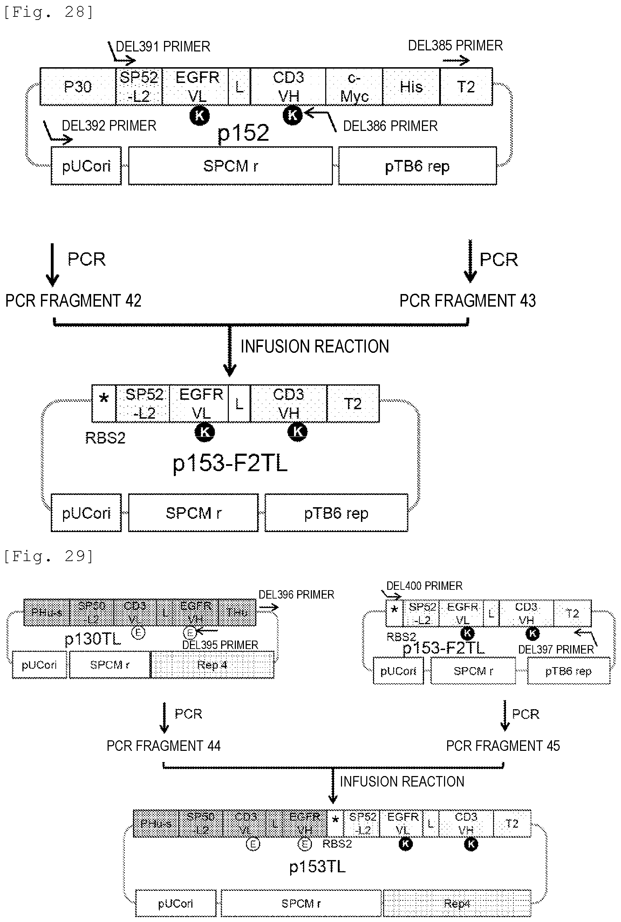

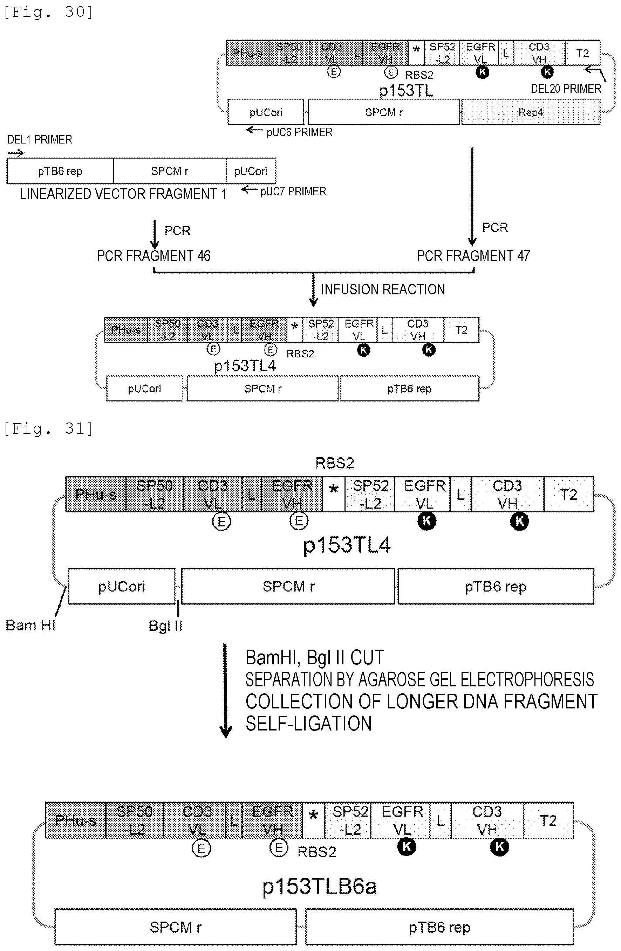

A plasmid (plasmid p153TLB6a) in which Hu promoter (PHu) was changed to Hu-s promoter, a ribosome-binding site (RBS2 [specifically, a polynucleotide consisting of 54 nucleotide residues upstream of the translation start codon of 30S ribosomal protein S16 gene derived from *Bifidobacterium longum* 105-A strain]) for translating LH31 polypeptide 2 was inserted instead of P30 promoter, and the Escherichia coli origin of replication, pUCori, was removed in the plasmid pLH31-L5 (see Figure 6) produced in Example 1, the plasmid expressing: mutant LH31 polypeptide 1 in which SP50-L2 was added at the N-terminus instead of SP50-L5 and mutant LH31 polypeptide 2 in which SP52-L2 was added at the N-terminus instead of SP50-L5 (see Figure 31) was produced according to the procedures shown in Figure 27 to Figure 31 by the same method as in Example 1. The plasmid p153TLB6a could polycistronically express mutant LH31 polypeptide 1 and mutant LH31 polypeptide 2 by insertion of RBS2 and could secrete the EGFR×human CD3 diabody-type BsAb (i.e., mutant LH31 diabody-type BsAb) composed of mutant LH31 polypeptide 1 and mutant LH31 polypeptide 2. Table 7 shows the primer sets used for PCR.

### 12-4: Production of DUP-153 strain

The transformation into *Bifidobacterium longum* 105-A strain using the plasmid p153TLB6a was performed by the same method as in Example 1, to obtain *Bifidobacterium longum* 105-A strain carrying the plasmid p153TLB6a (DUP-153 strain).

### [Example 13] Evaluation of binding activity of EGFR×CD3 diabody-type BsAbs derived from DUP-143 strain and DUP-153 strain to CD3

It was confirmed according to the methods described in sections [13-1] and [13-2] below that the EGFR×CD3 diabody-type BsAbs expressed/secreted by *Bifidobacterium* transformants (DUP-143 strain and DUP-153 strain) specifically bound to both human EGFR and human CD3.

### 13-1: Purification of EGFR×CD3 diabody-type BsAbs

The EGFR×CD3 diabody-type BsAbs secreted by *Bifidobacterium* transformants (DUP-143 strain and DUP-153 strain) were purified from the culture supernatants of DUP-143 strain and DUP-153 strain according to the method described in section "7-1" of Example 7.

### 13-2: ELISA method

The ELISA method using the EGFR×CD3 diabody-type BsAbs derived from *Bifidobacterium* transformants (DUP-143 strain and DUP-153 strain) was performed according to the method described in section "7-2" of Example 7. Further, CUM-36-1H strain-derived HER2×CD3 diabody-type BsAb purified in Example 21, which will be described below, was used as a negative control.

### (Result)

It was indicated that the corrected value of absorbance increased depending on the concentrations of DUP-143 strain- and DUP-153 strain-derived EGFR×CD3 diabody-type BsAbs (see "DUP-143" and "DUP-153" in Figure 15). Meanwhile, no increase was observed in corrected values of absorbance of the CUM-36-1H strain-derived HER2×CD3 diabody-type BsAb (see "CUM36-1H" in Figure 15) . These results indicate that the EGFR×CD3 diabody-type BsAbs (i.e., mutant LH31 diabody-type BsAb) secreted by *Bifidobacterium* transformants (DUP-143 strain and DUP-153 strain) specifically bind to both human CD3 and human EGFR.

### [Example 14] Amounts of EGFR×CD3 diabody-type BsAbs in culture supernatants of DUP-143 strain and DUP-153 strain

The amounts of the EGFR×CD3 diabody-type BsAbs in the culture supernatants of *Bifidobacterium* transformants (DUP-143 strain and DUP-153 strain) were measured according to the method described in Example 8.

### (Result)

It was confirmed that the concentration of the EGFR×CD3 diabody-type BsAb (i.e., mutant LH31 diabody-type BsAb) in the culture supernatant of DUP-143 strain was 702 ± 63 (ng/mL), and the concentration of the EGFR×CD3 diabody-type BsAb (i.e., mutant LH31 diabody-type BsAb) in the culture supernatant of DUP-153 strain was 634 ± 46 (ng/mL).

### [Example 15] Ratio of mutant LH31 polypeptide 1 to mutant LH31 polypeptide 2 in each of DUP-143 strain- and DUP-153 strain-derived mutant LH31 diabody-type BsAbs

It was confirmed that the polycistronically expressing plasmids p143TLB6a and p153TLB6a expressed mutant LH31 polypeptide 1 and mutant LH31 polypeptide 2 to the same extent.

To about 50 mL of each of the culture supernatants of DUP-143 strain and DUP-153 strain prepared in the above Example 14, was added ammonium sulfate to 80% saturation, followed by stirring overnight at 4°C for salting out. Thereafter, the mixture was centrifuged, and the precipitate was collected, dissolved in 1 mL of PBS (-), and then transferred to a spectra/pore 2 dialysis membrane (fraction molecular weight: 12 to 14 kD) (manufactured by Spectrum Laboratories Inc.), followed by dialysis in PBS (-) overnight at 4°C. The dialysis buffer was replaced on the way, and the protein solution after the dialysis was transferred to an ultrafiltration device (Amicon Ultra-0.5, nominal fraction molecular weight; 10 KDa) (manufactured by Merck KGaA) and concentrated to a 1/5 volume, to prepare a protein concentrate (n = 3) . 20 µL of a sample loading buffer was mixed with 80 µL of the protein concentrate, followed by heat treatment at 95°C for 3 minutes, to prepare a sample for electrophoresis (n = 3) . The sample for electrophoresis was electrophoresed with Mini Protean (R) TGX gel (4 to 20%) (manufactured by Bio-Rad Laboratories, Inc.) and stained with a staining solution (Oriole fluorescent gel stain) (manufactured by Bio-Rad Laboratories, Inc.).

**[Table 9]**

| Lane | Band 1 | Band 2 |
|---|---|---|
| 2 | 4, 368, 769 | 4, 504, 858 |
| 3 | 3,981,659 | 3,893,978 |
| 4 | 5,050,915 | 5,761,843 |
| 5 | 4,260,848 | 4, 452, 328 |
| 6 | 4,455,985 | 4.643,617 |
| 7 | 3, 800, 257 | 4,099,923 |

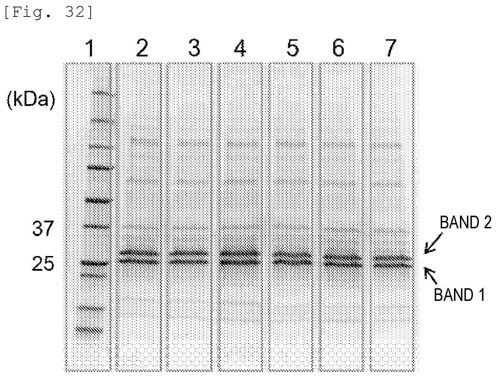

The values in the table indicate the intensities of band 1 and band 2 in Figure 32.

### (Result)

In both the DUP-143 strain and the DUP-153 strain, the band intensity derived from mutant LH31 polypeptide 1 and the band intensity derived from mutant LH31 polypeptide 2 were almost the same (see Figure 32 and Table 9) . These results indicate that the mutant LH31 polypeptide 1 and the mutant LH31 polypeptide 2 in the DUP-143 strain- and DUP-153 strain-derived mutant LH31 diabody-type BsAbs were expressed at almost the same ratio.

### [Example 16] Cytotoxic activity of DUP-143 strain and DUP-153 strain culture supernatants

The DUP-143 strain culture supernatant and the DUP-153 strain culture supernatant each diluted 10³-fold to 10⁶-fold were prepared, to analyze the cytotoxic activity according to the method described in Example 10. As cancer cells, TFK-1 cells as a human cholangiocarcinoma cell line (Institute of Development, Aging and Cancer, Tohoku University medical cell resource center/cell bank) and RKO cells as a human colon cancer cell line (obtained from ATCC [American Type Culture. Collection]) were used in addition to the HCT116 cells.

### (Result)

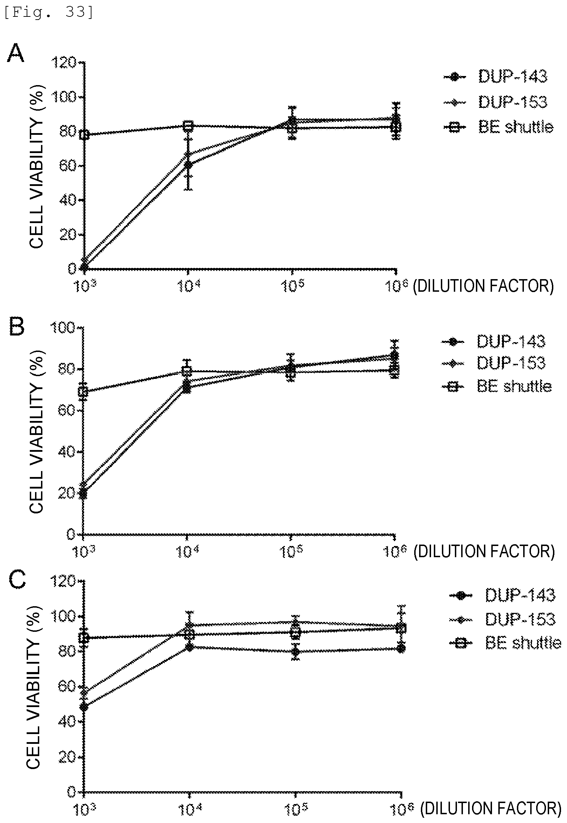

In the case of using the BE shuttle strain culture supernatant diluted 10³-fold, the viability of TFK-1 cells was 78.0%, whereas in the case of using the DUP-143 strain culture supernatant and the DUP-153 strain culture supernatant each diluted 10³-fold, the viabilities of TFK-1 cells were respectively 1.2% and 5.4% (see Figure 33A). Further, in the case of using the BE shuttle strain culture supernatant diluted 10⁴-fold, the viability of TFK-1 cells was 83.4%, whereas in the case of using the DUP-143 strain culture supernatant and the DUP-153 strain culture supernatant each diluted 10⁴-fold, the viabilities of TFK-1 cells were respectively 60.7% and 67.0% (see Figure 33A).

Further, in the case of using the BE shuttle strain culture supernatant diluted 10³-fold, the viability of HCT116 cells was 69.2%, whereas in the case of using the DUP-143 strain culture supernatant and the DUP-153 strain culture supernatant each diluted 10³-fold, the viabilities of HCT116 cells were respectively 19.9% and 24.4% (see Figure 33B). Further, in the case of using the BE shuttle strain culture supernatant diluted 10⁴-fold, the viability of HCT116 cells was 79.0%, whereas in the case of using the DUP-143 strain culture supernatant and the DUP-153 strain culture supernatant each diluted 10⁴-fold, the viabilities of HCT116 cells were respectively 71.3% and 74.3% (see Figure 33B).

Further, in the case of using the BE shuttle strain culture supernatant diluted 10³-fold, the viability of RKO cells was 87.9%, whereas in the case of using the DUP-143 strain culture supernatant and the DUP-153 strain culture supernatant each diluted 10³-fold, the viabilities of RKO cells were respectively 48.5% and 56.3% (see Figure 33C) . Further, in the case of using the BE shuttle strain culture supernatant diluted 10⁴-fold, the viability of RKO cells was 89.7%, whereas in the case of using the DUP-143 strain culture supernatant and the DUP-153 strain culture supernatant each diluted 10⁴-fold, the viabilities of RKO cells were respectively 82.6% and 95.0% (see Figure 33C).

These results indicate that the mutant LH31 diabody-type BsAbs secreted in the DUP-143 strain culture supernatant and the DUP-153 strain culture supernatant have cytotoxic activity on cancer cells such as HCT116 cells, TFK-1 cells, and RKO cells.

Further, it is indicated that the cancer cytotoxic activity of the EGFR×CD3 diabody-type BsAbs may possibly increase by substituting Q at Kabat position 38 in VL derived from anti-human CD3 antibody with E, substituting Q at Kabat position 39 in VH derived from anti-human EGFR antibody with E, substituting Q at Kabat position 38 in VL derived from anti-human EGFR antibody with K, and substituting Q at Kabat position 39 in VH derived from anti-human CD3 antibody with K, in the EGFR×CD3 diabody-type BsAbs, since the effect of reducing the viability of HCT116 cells (29% and 35%) in the case of using the DUP-143 strain (i.e., B strain carrying plasmid p143TLB6a) culture supernatant and the DUP-153 strain (i.e., B strain carrying plasmid p153TLB6a) culture supernatant increased more than that in the case of using the DUM-126H strain (i.e., B strain carrying plasmid p126) culture supernatant (83%) (see Example 10).

### [Example 17] Evaluation of cytotoxic activity of EGFR×CD3 diabody-type BsAbs purified from DUP-143 strain and DUP-153 strain culture supernatants

The EGFR×CD3 diabody-type BsAbs expressed/secreted by *Bifidobacterium* transformants (DUP-143 strain and DUP-153 strain) were purified according to the method described in section "7-1" of Example 7 and serially diluted to a concentration of 100 fM to 100 pM, to analyze the viability of HCT116 cells according to the method described in Example 4.

### (Result)

The viability of HCT116 cells decreased depending on the concentrations of the DUP-143 strain- and DUP-153 strain-derived EGFR×CD3 diabody-type BsAbs. The EC₅₀ value in the case of the DUP-143 strain-derived EGFR×CD3 diabody-type BsAb was 1.8 ± 0.9 pM, and the EC₅₀ value in the case of the DUP-153 strain-derived EGFR×CD3 diabody-type BsAb was 1.8 ± 1.1 pM. These results indicate that the EGFR×CD3 diabody-type BsAbs (i.e., mutant LH31 diabody-type BsAbs) expressed/secreted by the *Bifidobacterium* transformants have injury activity on cancer cells.

Further, the EC₅₀ values (1.8 pM) in the case of using mutant LH31 diabody-type BsAbs purified from DUP-143 strain and DUP-153 strain decreased to about 21% of the EC₅₀ value (8.4 pM) in the case of using LH31 diabody-type BsAb purified from DUM-126H strain (see Example 9). These results indicate that the EGFR×CD3 diabody-type BsAbs (mutant LH31 diabody-type BsAbs) expressed/secreted by the DUP-143 strain and the DUP-153 strain had higher cytotoxic activity on cancer cells than the EGFR×CD3 diabody-type BsAb (LH31 diabody-type BsAb) expressed/secreted by the DUM-126H strain, thereby supporting the results of Example 16.

### [Example 18] In-vivo efficacy by method of implanting mince containing DUP-143 strain and DUP-153 strain

The anti-tumor effects of the EGFR×CD3 diabody-type BsAbs secreted by the DUP-143 strain and the DUP-153 strain were analyzed in vivo using cancer-bearing Scid mice carrying cancer, HCT116 cells, as a human large intestine cancer cell line. Specifically, the analysis was performed according to the following method.

HCT116 cells cultured in McCoy's5A medium containing 10% FBS (manufactured by Equitech-Bio Inc.) were subcutaneously implanted into 9 week-old female Scid mice to produce cancer-bearing mice. The cancer-bearing mice with a tumor size of 263.83 to 476.70 mm³ were divided (5 days before tumor mince implantation) into 4 groups, a BE shuttle strain-administered group (12 mice), a DUM-126H strain-administered group (12 mice), a DUP-143 strain-administered group (12 mice), and a DUP-153 strain-administered group (12 mice). Thereafter, a frozen formulation of 5.0 × 10⁸ cfu/0.2 mL BE shuttle strain was administered intravenously to the tail veins of the BE shuttle strain-administered group, a simple frozen formulation of 5.0 × 10⁸ cfu/0.2 mL DUM-126H strain was administered intravenously to the tail veins of the DUM-126H strain-administered group, a simple frozen formulation of 5.0 × 10⁸ cfu/0.2 mL DUP-143 strain was administered intravenously to the tail veins of the DUP-143 strain-administered group, and a simple frozen formulation of 5.0 × 10⁸ cfu/0.2 mL DUP-153 strain was administered intravenously to the tail veins of DUP-153 strain-administered group, on each of day 1 (4 days before tumor mince implantation) and day 2 (3 days before tumor mince implantation) . 1 to 4 days after dividing into the 4 groups (4 days to 1 day before tumor mince implantation), 1 mL of a 10% maltose solution was intraperitoneally administered twice a day, and 0.2 mL of anti-asialo GM1 antibody diluted 10-fold with PBS (-) was administered intraperitoneally 4 days later (1 day before tumor mince implantation). Thereafter, the anti-asialo GM1 antibody was administered intraperitoneally to the Scid mice to be subjected to subcutaneous implantation in the same manner. 5 days after dividing into the 4 groups (on day 0 after tumor mince implantation), the tumors were extracted and pooled from the mice of each group (the BE shuttle strain-administered group, the DUM-126H strain-administered group, the DUP-143 strain-administered group, and the DUP-153 strain-administered group), the mince was diluted 1.33-fold with a 1:1 mixed solution of Matrigel (manufactured by Corning Inc.) and HBSS (manufactured by Life Technologies Corporation), the T-LAKs cultured were mixed to 0.375 × 10⁸ cells/mL, and Teceleukin (manufactured by SHIONOGI & CO., LTD.) was added to 5000 U/mL, to prepare a mince implantation solution. 0.2 mL of the mince implantation solution was subcutaneously implanted into new 7 week-old female Scid mice, which were divided into the BE shuttle strain group, the DUM-126H strain group, the DUP-143 strain group, and the DUP-153 strain group (12 mice for each group). The number of viable bacteria of *Bifidobacterium* transformants (BE shuttle strain, DUM-126H strain, DUP-143 strain, and DUP-153 strain) contained in the tumor before preparing tumor mince or in the tumor mince implanted was measured according to the method described in section "11-2" above (see "the number of viable bacteria in tumor" and "the number of viable bacteria in implanted tumor mince" in Table 10, respectively), and the concentration of each EGFR×CD3 diabody-type BsAb contained in the tumor mince was measured according to the methods described in sections "11-3" and "11-4" above (see "EGFR×CD3 diabody-type BsAb concentration in tumor mince" in Table 10). On day 0 to day 4, day 7 to day 11, and day 14 to day 16 after tumor mince implantation, 1 mL of a 10% maltose solution was intraperitoneally administered twice a day, and the tumor diameter of mice of each group was measured on day 7, day 11, day 14, and day 17 after tumor mince implantation, to calculate the tumor volume based on the formula "tumor volume = major axis × minor axis²/2" (see Figure 34). The tumor growth inhibition rate [TGI] was calculated based on the formula "TGI = [average tumor volume 1-17 days after tumor mince implantation (each diabody-type BsAb secretory *Bifidobacterium* strain group)/average tumor volume 17 days after tumor mince implantation (BE shuttle strain group)] × 100" .

**[Table 10]**

| Group | The number of viable bacteria in tumor (cfu/g tumor) | The number of viable bacteria in implanted tumor mince (cfu/mouse) | EGFR×CD3 diabody-type BsAb concentration (pM) in tumor mince |
|---|---|---|---|
| BE shuttle strain group | 9.9×10⁸ | 7.4×10⁷ | ND |
| DUM-126H strain group | 5.7×10⁸ | 4.3×10⁷ | 312.8 |
| DUP-143 strain group | 5.0×10⁸ | 3.8×10⁷ | 83.1 |
| DUP-153 strain group | 3.6×10⁸ | 2.7×10⁷ | 108.3 |

In the table, "ND" indicates that the result was equal to or less than the detection sensitivity.

### (Result)

First, it was confirmed that the number of viable bacteria of *Bifidobacterium* transformants (each of BE shuttle strain, DUM-126H strain, DUP-143 strain, and DUP-153 strain) was contained in the tumor mince used for implantation of each group, and the three types of *Bifidobacterium* transformants other than the negative control (DUM-126H strain, DUP-143 strain, and DUP-153 strain) expressed/secreted EGFR×CD3 diabody-type BsAbs (i.e., LH31 diabody-type BsAb and mutant LH31 diabody-type BsAbs) (see Table 10).

Thereafter, as a result of analyzing the tumor volume of each group with the tumor mince implanted, the tumor volume of the DUP-143 strain group mice and the tumor volume of the DUP-153 strain group mice significantly decreased as compared with the tumor volume of the BE shuttle strain group mice as the negative control (see Figure 34). Further, although the concentrations of the DUP-143 strain and DUP-153 strain diabody-type BsAbs contained in the tumor mince were less than the concentration of the DUM-126H strain diabody-type BsAb (see Table 10), the tumor volume reducing effect was larger when using the DUP-143 strain or the DUP-153 strain than when using the DUM-126H strain (see Figure 34). Specifically, the TGI relative to the BE shuttle strain group on day 17 after the tumor mince implantation was 43.1% for the DUM-126H strain group, whereas it was as high as 60.6% for the DUP-143 strain group and 61.5% for the DUP-153 strain. These results indicate that the EGFR×CD3 diabody-type BsAbs (mutant LH31 diabody-type BsAb) expressed/secreted by the DUP-143 strain and the DUP-153 strain have anti-tumor effects higher than that of the EGFR×CD3 diabody-type BsAb (LH31 diabody-type BsAb) expressed/secreted by the DUM-126H strain, supporting the results of Examples 16 and 17.

### [Example 19] Production of Bifidobacteria (CUM-36-1H strain and CUM-36-2H strain) expressing/secreting HER2×CD3 diabody-type BsAbs

*Bifidobacteria* expressing/secreting HER2×human CD3 diabody-type BsAbs were produced according to the methods described in sections [19-1] and [19-2] below.

### 19-1: Production of plasmids pLH36-1 and pLH36-2

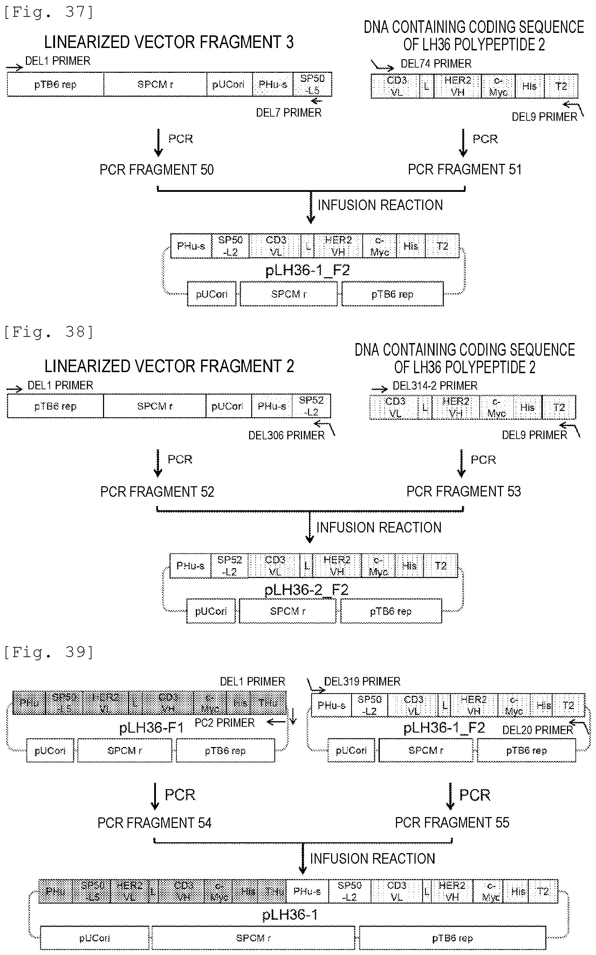

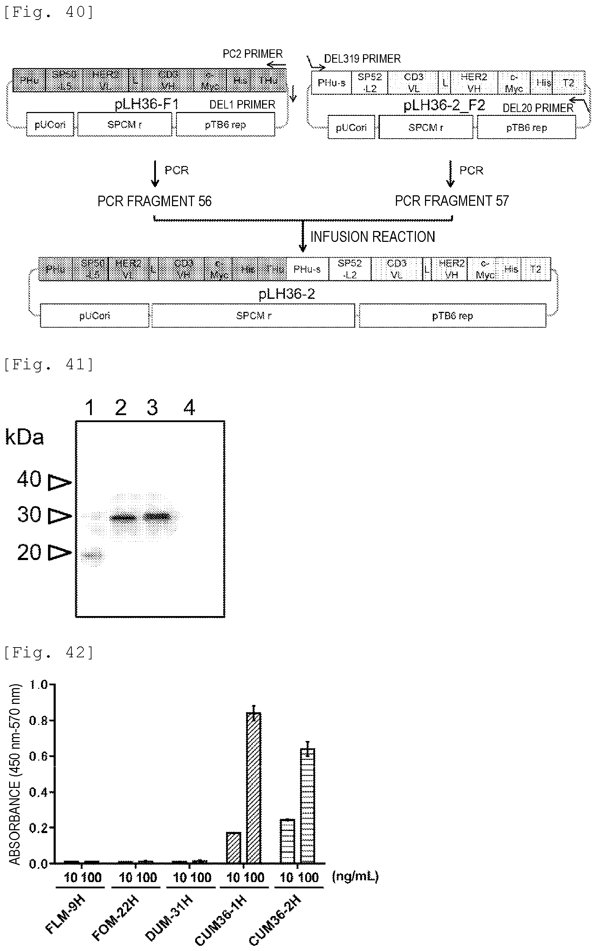

In order to produce *Bifidobacteria* expressing/secreting HER2×human CD3 diabody-type BsAbs (specifically, a diabody-type BsAb [hereinafter referred to as "LH36 diabody-type BsAb"] composed of LH36 polypeptide 1 consisting of the amino acid sequence of SEQ ID No: 14 [see Table 11] and LH36 polypeptide 2 consisting of the amino acid sequence of SEQ ID No: 15 [see Table 12]), DNA consisting of the nucleotide sequence of SEQ ID No: 16 and containing the coding sequence of LH36 polypeptide 1 (see Figure 35A) and DNA consisting of the nucleotide sequence of SEQ ID No: 17 and containing the coding sequence of LH36 polypeptide 2 (see Figure 35B) were outsourced to GenScript Japan Inc. and synthesized, to produce plasmids pLH36-1 (see Figure 39) and pLH36-2 (see Figure 40) according to the procedures shown in Figures 36 to 40. More specifically, the plasmid pLH36-1 was a plasmid expressing LH36 polypeptide 1 in which SP50-L5 (the amino acid sequence of SEQ ID No: 10) and c-Myc-His fusion tag (the amino acid sequence of SEQ ID No: 11) were respectively added to the N-terminal side and the C-terminal side and LH36 polypeptide 2 in which SP50-L2 (the amino acid sequence of SEQ ID No: 13) and c-Myc-His fusion tag (the amino acid sequence of SEQ ID No: 11) were respectively added to the N-terminal side and the C-terminal side (see Figure 39). More specifically, the plasmid pLH36-2 was a plasmid expressing LH36 polypeptide 1 in which SP50-L5 (the amino acid sequence of SEQ ID No: 10) and c-Myc-His fusion tag (the amino acid sequence of SEQ ID No: 11) were respectively added to the N-terminal side and the C-terminal side and LH36 polypeptide 2 in which SP52-L2 (i.e., the connecting peptide between secretion signal peptide SP52 [amino acid residues 1 to 35 of SEQ ID No: 18] and L2 linker peptide [amino acid residues 36 to 37 of SEQ ID No: 18]) and c-Myc-His fusion tag (the amino acid sequence of SEQ ID No: 11) were respectively added to the N-terminal side and the C-terminal side (see Figure 40). Table 7 shows the primer sets used for PCR.

**[Table 11]**

| |
|---|
| LH36 polypeptide 1 consisting of amino acid sequence of SEQ ID No: 14 |
| DIQMTQSPSSLSASVGDRVTITCKASQDVSIGVAWYQQKPGKAPKLLIYSASYRYTGVPSRFS GSGSGTDFTLTISSLQPEDFATYYCQQYYIYPYTFGQGTKVEIKA AGGGGS EVQLVESGGGL VQPGGSLRLSCAASGYSFTGYTMNWVRQAPGKGLEWVALINPYKGVSTYNQKFKDRFTISV DKSKNTAYLQMNSLRAEDTAVYYCARSGYYGDSDWYFDVWGQGTLVTVSS |

The part surrounded by a square in the table indicates VL derived from anti-HER2 antibody, the part surrounded by a double square indicates VH derived from anti-human CD3 antibody, and the peptide (AGGGGS) between the two indicates a linker peptide.

**[Table 12]**

| |
|---|
| LH36 polypeptide 2 consisting of amino acid sequence of SEQ ID No: 15 |
| DIQMTQSPSSLSASVGDRVTITCRASQDIRNYLNWYQQKPGKAPKLLIYYTSRLESGVPSRFS GSGSGTDYTLTISSLQPEDFATYYCQQGNTLPWTFGQGTKVEIKAAGGGGSEVQLVESGGG LVQPGGSLRLSCAASGFTFTDYTMDWVRQAPGKGLEWVADVNPNSGGSIYNQRFKGRFTL SVDRSKNTLYLQMNSLRAEDTAVYYCARNLGPSFYFDYWGQGTLVTVSS |

The part surrounded by a square in the table indicates VL derived from anti-human CD3 antibody, the part surrounded by a double square indicates VH derived from anti-HER2 antibody, and the peptide (AGGGGS) between the two indicates a linker peptide.

### 19-2: Production of CUM-36-1H strain and CUM-36-2H strain

The transformation into *Bifidobacterium longum* 105-A strain using the plasmids pLH36-1 and the pLH36-2 was performed by the same method as in Example 1, to obtain *Bifidobacterium longum* 105-A strain carrying the plasmid pLH36-1 (CUM-36-1H strain) and *Bifidobacterium longum* 105-A strain carrying the plasmid pLH36-2 (CUM-36-2H strain).

### [Example 20] Evaluation (WB) of expression/secretion of CUM-36-1H strain and CUM-36-2H strain

In order to confirm that *Bifidobacterium* transformants (CUM-36-1H strain and CUM-36-2H strain) expressed and secreted the HER2×CD3 diabody-type BsAbs, Western blotting method using the CUM-36-1H strain culture supernatant and the CUM-36-2H strain culture supernatant was performed according to the method described in Example 2.

### (Result)

In the CUM-36-1H strain culture supernatant and the CUM-36-2H strain culture supernatant, bands derived from the HER2×CD3 diabody-type BsAbs were observed around 30 kDa (see Figure 41). These results indicate that *Bifidobacterium* transformants (CUM-36-1H strain and CUM-36-2H strain) expressed and secreted the HER2×CD3 diabody-type BsAbs (i.e., LH36 diabody-type BsAbs).

### [Example 21] Evaluation of binding activity of CUM-36-1H strain- and CUM-36-2H strain-derived HER2×CD3 diabody-type BsAbs to HER2 and human CD3

It was confirmed according to the methods described in sections [21-1] and [21-2] below that the HER2×CD3 diabody-type BsAbs secreted by *Bifidobacterium* transformants (CUM-36-1H strain and CUM-36-2H strain) specifically bound to both HER2 and human CD3.

### 21-1: Purification of HER2×CD3 diabody-type BsAbs

The LH36 diabody-type BsAbs secreted by *Bifidobacterium* transformants (CUM-36-1H strain and CUM-36-2H strain) were purified from the CUM-36-1H strain and CUM-36-2H strain culture supernatants according to the method described in section "3-1" of Example 3.

### 21-2: ELISA method

### 21-2-1: Reaction treatment between antigen and antibody

The reaction treatment between the antibody (LH36 diabody-type BsAb) and the antigen (immobilized human CD3E/CD3D heterodimer recombinant protein) was performed according to the method described in section "3-2-1" of Example 3. Further, the EGFR×CD3 diabody-type BsAbs derived from three types of *Bifidobacterium* transformants (FLM-9H strain, FOM-22H strain, and DUM-31H strain) purified in Example 3 were used as negative controls.

### 21-2-2: Detection of antibody

25 µL of a signal enhancer HIKARI B solution (manufactured by NACALAI TESQUE, INC.) containing 0.1 µg/mL recombinant biotinylated HER2 (manufactured by ACROBiosystems) was dispensed into each well subjected to the reaction treatment between the antigen and the antibody, followed by standing at 25°C for 1 hour. The solution in each well was removed, and the well was washed with 200 µL of PBS-T 3 times. VECTASTAIN Elite ABC reagent (avidin-biotin-labeled HRP) (manufactured by Vector Laboratories) was adjusted to 4 µL/mL using a signal enhancer HIKARI B solution (manufactured by NACALAI TESQUE, INC.) containing 1% BSA, and then 25 µL thereof was dispensed into each well, followed by standing at 25°C for 30 minutes. Thereafter, the solution in each well was removed, and the well was washed with 200 µL of PBS-T 3 times. After mixing equal amounts of Color Solution A and Color Solution B (solutions containing TMB as a substrate of HRP) (manufactured by R&D Systems, Inc.) as detection reagents, 50 µL thereof was dispensed into each well, and light was blocked, followed by standing at room temperature for 20 minutes. Thereafter, 25 µL of Stop Solution (manufactured by R&D Systems, Inc.) was added thereto, to stop the color reaction. The absorbance at 450 nm as the absorption wavelength of TMB-derived pigments was measured, and the value corrected with the absorbance at 570 nm (negative control) (average ± standard deviation, see the vertical axis of Figure 42) was calculated.

### (Result)

It was indicated that the corrected value of absorbance increased depending on the concentrations of CUM-36-1H strain- and CUM-36-2H strain-derived HER2×CD3 diabody-type BsAbs (see "CUM-36-1H" and "CUM-36-2H" in Figure 42, respectively). Meanwhile, no increase was observed (see "FLM-9H", "FOM-22H", and "DUM-31H" in Figure 42) in corrected values of absorbance of the EGFR×CD3 diabody-type BsAbs derived from three types of *Bifidobacterium* transformants (FLM-9H strain, FOM-22H strain, and DUM-31H strain). These results indicate that the HER2×CD3 diabody-type BsAbs (i.e., LH36 diabody-type BsAbs) expressed/secreted by CUM-36-1H strain and CUM-36-2H strain specifically bind to both human CD3 and HER2.

### [Example 22] Cytotoxic activity of CUM-36-1H strain culture supernatant and CUM-36-2H strain culture supernatant

The CUM-36-1H strain culture supernatant and CUM-36-2H strain culture supernatant each diluted 10-fold to 10⁴-fold were prepared, and the cytotoxic activity on TFK-1 cells was analyzed according to the method described in Example 10.

### (Result)

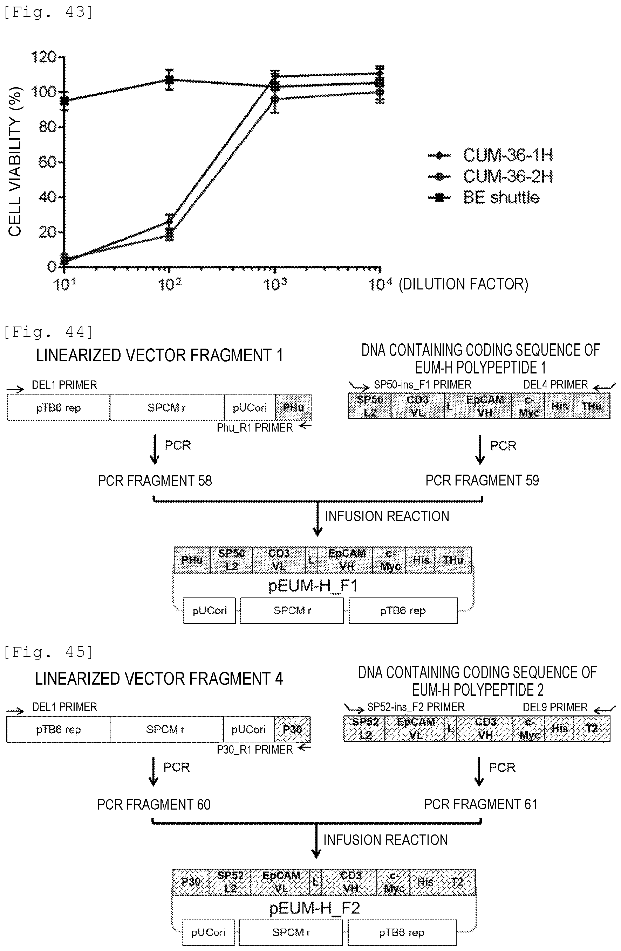

In the case of using the BE shuttle strain culture supernatant diluted 10-fold, the viability of TFK-1 cells was 95.0%, whereas in the case of using the CUM-36-1H strain culture supernatant and the CUM-36-2H strain culture supernatant each diluted 10-fold, the viability of TFK-1 cells was 3.1% and 4.7%, respectively (see Figure 43) . Further, in the case of using the BE shuttle strain culture supernatant diluted 10²-fold, the viability of TFK-1 cells was 107.2%, whereas in the case of using the CUM-36-1H strain culture supernatant and the CUM-36-2H strain culture supernatant each diluted 10²-fold, the viability of TFK-1 cells was 26.2% and 18.3%, respectively (see Figure 43) . These results indicate that the HER2×CD3 diabody-type BsAbs secreted in the CUM-36-1H strain culture supernatant and the CUM-36-2H strain culture supernatant have cytotoxic activity on cancer cells such as TFK-1 cells.

### [Example 23] Production of Bifidobacterium (EUM-H strain) expressing/secreting EpCAM×CD3 diabody-type BsAb

A *Bifidobacterium* expressing/secreting human EpCAM×human CD3 diabody-type BsAb was produced according to the methods described in sections [23-1] and [23-2] below.

### 23-1: Production of plasmid pEUM-H

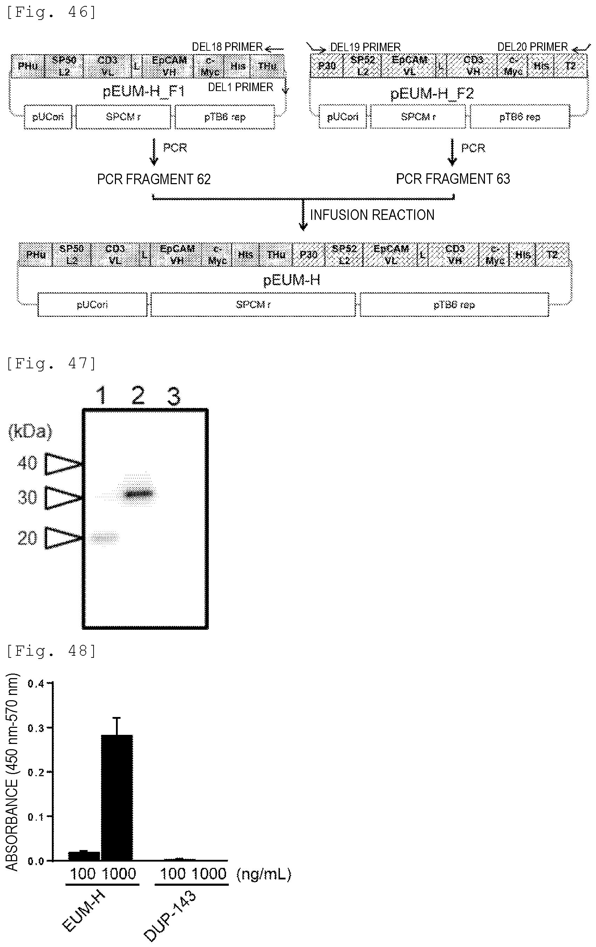

In order to produce a *Bifidobacterium* expressing/secreting a human EpCAM×human CD3 diabody-type BsAb (specifically, a diabody-type BsAb composed of EUM-H polypeptide 1 consisting of the amino acid sequence of SEQ ID No: 82 [see Table 13] and EUM-H polypeptide 2 consisting of the amino acid sequence of SEQ ID No: 83 [see Table 14] [hereinafter referred to as "EUM-H diabody-type BsAb"]), DNA consisting of the nucleotide sequence of SEQ ID No: 84 and containing the coding sequence of EUM-H polypeptide 1 (see Figure 44) and DNA consisting of the nucleotide sequence of SEQ ID No: 85 and containing the coding sequence of EUM-H polypeptide 2 (see Figure 45) were outsourced to GenScript Japan Inc. and synthesized, to produce plasmid pEUM-H according to the procedures shown in Figures 44 to 46 (see Figure 46). More specifically, the plasmid pEUM-H was a plasmid expressing EUM-H polypeptide 1 in which SP50-L2 (the amino acid sequence of SEQ ID No: 13) and c-Myc-His fusion tag (the amino acid sequence of SEQ ID No: 11) were respectively added to the N-terminal side and the C-terminal side and EUM-H polypeptide 2 in which SP52-L2 (i.e., the connecting peptide between secretion signal peptide SP52 [amino acid residues 1 to 35 of SEQ ID No: 18] and L2 linker peptide [amino acid residues 36 to 37 of SEQ ID No: 18]) and c-Myc-His fusion tag (the amino acid sequence of SEQ ID No: 11) were respectively added to the N-terminal side and the C-terminal side (see Figure 46) . Table 7 shows the primer sets used for PCR.

**[Table 13]**

| |
|---|
| EUM-H polypeptide 1 consisting of amino acid sequence of SEQ ID No: 82 |
| DIQMTQSPSSLSASVGDRVTITCRASQDIRNYLNWYQQKPGKAPKLLIYYTSRLESGVPSR FSGSGSGTDYTLTISSLQPEDFATYYCQQGNTLPWTFGQGTKVEIKAGGGGSEVQLLEQS GAELVRPGTSVKISCKASGYAFTNYWLGWVKQRPGHGLEWIGDIFPGSGNIHYNEKFKGKA TLTADKSSSTAYMQLSSLTFEDSAVYFCARLRNWDEPMDYWGQGTTVTVSS |

The part surrounded by a square in the table indicates VL derived from anti-human CD3 antibody, the part surrounded by a double square indicates VH derived from anti-human EpCAM antibody, and the peptide (AGGGGS) between the two indicates a linker peptide.

**[Table 14]**

| |
|---|
| EUM-H polypeptide 2 consisting of amino acid sequence of SEQ ID No: 83 |
| ELVMTQSPSSLTVTAGEKVTMSCKSSQSLLNSGNQKNYLTWYQQKPGQPPKLLIYWASTRES GVPDRFTGSGSGTDFTLTISSVQAEDLAVYYCQNDYSYPLTFGAGTKLEIKAAGGGGSEVQL VESGGGLVQPGGSLRLSCAASGYSFTGYTMNWVRQAPGKGLEWVALINPYKGVSTYNQKFKD RFTISVDKSKNTAYLQMNSLRAEDTAVYYCARSGYYGDSDWYFDVWGQGTLVTVSS |

The part shown by an underline in the table indicates VL derived from anti-human EpCAM antibody, the part shown by a double underline indicates VH derived from anti-human CD3 antibody, and the peptide (AAGGGGS) between the two indicates a linker peptide.

### 23-2: Production of EUM-H strain

The transformation into *Bifidobacterium longum* 105-A strain using the plasmid pEUM-H was performed by the same method as in Example 1, to obtain *Bifidobacterium longum* 105-A strain carrying the plasmid pEUM-H (EUM-H strain).

### [Example 24] Evaluation (WB) of expression/secretion of EUM-H strain

In order to confirm that the *Bifidobacterium* transformants (EUM-H strain) expressed and secreted the EpCAM×CD3 diabody-type BsAb, Western blotting method using the EUM-H strain culture supernatant was performed according to the method described in Example 2.

### (Result)

In the EUM-H strain culture supernatant, a band derived from the EpCAM×CD3 diabody-type BsAb was observed around 30 kDa (see Figure 47). This result indicates that *Bifidobacterium* transformants (EUM-H strain) express and secrete the EpCAM×CD3 diabody-type BsAb (i.e., EUM-H diabody-type BsAb).

### [Example 25] Evaluation of binding activity of EUM-H strain-derived EpCAM×CD3 diabody-type BsAb to human EpCAM and human CD3

It was confirmed according to the methods described in sections [25-1] and [25-2] below that the EpCAM×CD3 diabody-type BsAb secreted by *Bifidobacterium* transformants (EUM-H strain) specifically bound to both human EpCAM and human CD3.

### 25-1: Purification of EpCAM×CD3 diabody-type BsAb

The EUM-H diabody-type BsAb secreted by *Bifidobacterium* transformants (EUM-H strain) was purified from the EUM-H strain culture supernatant according to the method described in section "3-1" of Example 3.

### 25-2: ELISA method

### 25-2-1: Reaction treatment between antigen and antibody

25 µL of a solution containing 2.5 µg/mL human CD3E/CD3D heterodimer recombinant protein (available from ACROBiosystems) was dispensed into each well of a 96-well plate, followed by standing overnight at 4°C for immobilization. The solution after immobilization was removed, and each well was washed with 200 µL of PBS-T 3 times. Thereafter, 200 µL of a blocking solution (Blocking One, manufactured by NACALAI TESQUE, INC.) diluted 5-fold with distilled water was dispensed, followed by standing at 25°C for 2 hours for blocking treatment. The blocking solution in each well was removed, and the well was washed with 200 µL of PBS-T 3 times. 100 (ng/mL) or 1000 (ng/mL) EpCAM×CD3 diabody-type BsAb and 100 (ng/mL) or 1000 (ng/mL) EGFR×CD3 diabody-type BsAb as a negative control were each diluted 50-fold with a signal enhancer HIKARI A solution (manufactured by NACALAI TESQUE, INC.) containing 1% BSA, and 25 µL of each solution was dispensed into each well after blocking, followed by standing at 25°C for 2 hours for reaction treatment between the antibody (EpCAM×CD3 diabody-type BsAb) and the antigen (immobilized human CD3E/CD3D heterodimer recombinant protein). Thereafter, the solution in each well was removed, and the well was washed with 200 µL of PBS-T 3 times.

### 25-2-2: Detection of antibody

25 µL of a signal enhancer HIKARI B solution (manufactured by NACALAI TESQUE, INC.) containing 0.1 µg/mL recombinant biotinylated EpCAM (manufactured by ACROBiosystems) was dispensed into each well subjected to the reaction treatment between the antigen and the antibody, followed by standing at 25°C for 1 hour. The solution in each well was removed, and the well was washed with 200 µL of PBS-T 3 times. VECTASTAIN Elite ABC reagent (avidin-biotin-labeled HRP) (manufactured by Vector Laboratories) was adjusted to 4 µL/mL using a signal enhancer HIKARI B solution (manufactured by NACALAI TESQUE, INC.) containing 1% BSA, and 25 µL thereof was dispensed into each well, followed by standing at 25°C for 1 hour. Thereafter, the solution in each well was removed, and the well was washed with 200 µL of PBS-T 3 times. After mixing equal amounts of Color Solution A and Color Solution B (solutions containing TMB as a substrate of HRP) (manufactured by R&D Systems, Inc.) as detection reagents, 50 µL thereof was dispensed into each well, and light was blocked, followed by standing at room temperature for 20 minutes. Thereafter, 25 µL of Stop Solution (manufactured by R&D Systems, Inc.) was added thereto, to stop the color reaction. The absorbance at 450 nm as the absorption wavelength of TMB-derived pigments was measured, and the value corrected with the absorbance at 570 nm (negative control) (average ± standard deviation, see the vertical axis of Figure 48) was calculated.

### (Result)

It was indicated that the corrected value of absorbance increased depending on the concentration of EUM-H strain-derived EpCAM×CD3 diabody-type BsAb (see "EUM-H" in Figure 48). Meanwhile, no increase was observed in corrected value of absorbance of DUP-143 strain-derived EGFR×CD3 diabody-type BsAb (see "DUP-143" in Figure 48) . These results indicate that the EpCAM×CD3 diabody-type BsAb secreted by *Bifidobacterium* transformants (EUM-H strain) specifically binds to both human CD3 and EpCAM.

### [Example 26] Cytotoxic activity of diabody purified from EUM-H strain culture supernatant

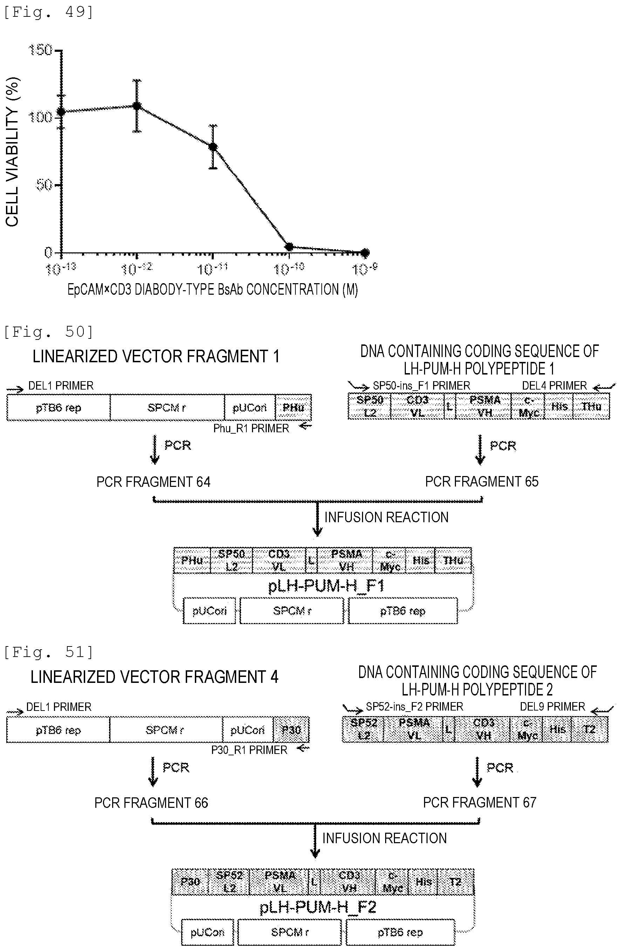

The method was the same as in Example 4. However, a diabody purified from the EUM-H strain culture supernatant serially diluted to a concentration of 100 fM to 1 nM was used as a diabody to be added to the cells. Figure 49 shows the results.

### (Result)

As shown in Figure 49, the diabody purified from the EUM-H strain culture supernatant exhibited cytotoxic activity on HCT116 cells as an EpCAM-positive human colon cancer cell line, and the EC₅₀ value was 17.3 pM. These results indicate that the EpCAM×CD3 diabody-type BsAb secreted in the EUM-H strain culture supernatant has cytotoxic activity on cancer cells such as HCT116 cells.

### [Example 27] Production of Bifidobacteria (LH-PUM-H strain and HL-PUM-H strain) expressing/secreting PSMA×CD3 diabody-type BsAbs

*Bifidobacteria* expressing/secreting human PSMA×human CD3 diabody-type BsAbs were produced according to the methods described in sections [27-1] and [27-2] below.

### 27-1: Production of plasmid pLH-PUM-H and pHL-PUM-H

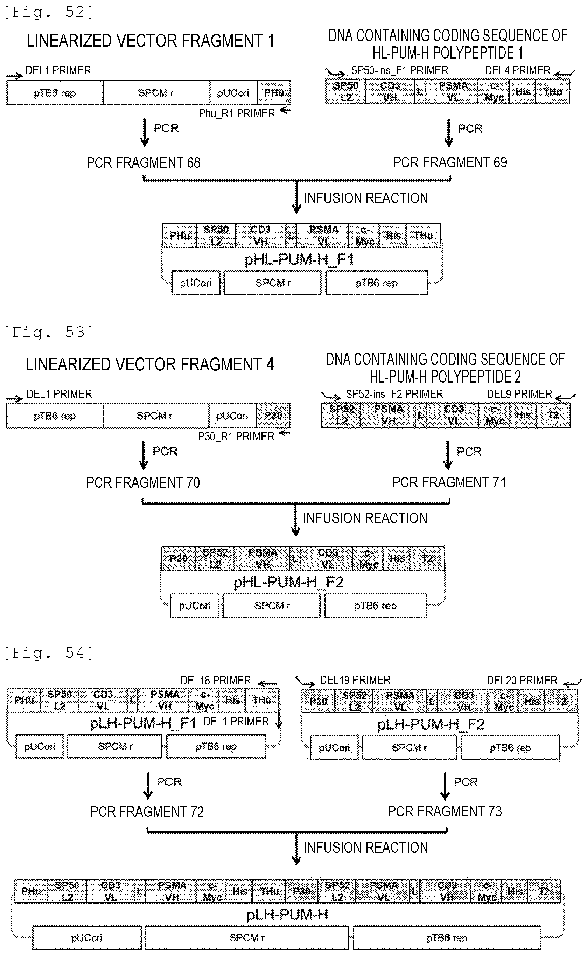

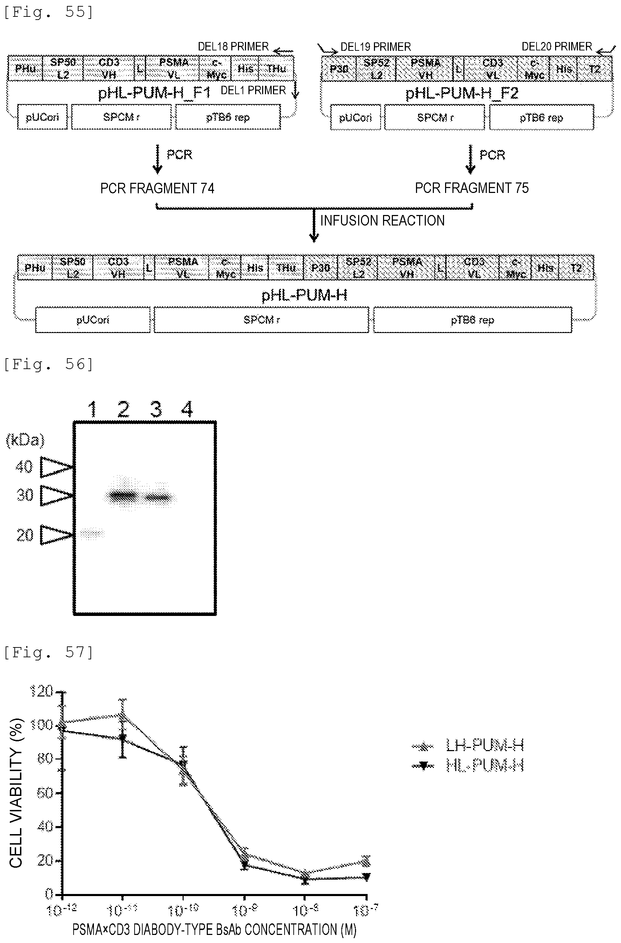

In order to produce *Bifidobacteria* expressing/secreting the human PSMA×human CD3 diabody-type BsAbs (specifically, a *Bifidobacterium* expressing/secreting a diabody-type BsAb [hereinafter referred to as "LH-PUM-H diabody-type BsAb"] composed of LH-PUM-H polypeptide 1 consisting of the amino acid sequence of SEQ ID No: 86 [see Table 15] and LH-PUM-H polypeptide 2 consisting of the amino acid sequence of SEQ ID No: 87 [see Table 16] and a diabody-type BsAb [hereinafter referred to as "HL-PUM-H diabody-type BsAb"] composed of HL-PUM-H polypeptide 1 consisting of the amino acid sequence of SEQ ID No: 88 [see Table 17] and HL-PUM-H polypeptide 2 consisting of the amino acid sequence of SEQ ID No: 89 [see Table 18]), DNA consisting of the nucleotide sequence of SEQ ID No: 90 and containing the coding sequence of LH-PUM-H polypeptide 1 (see Figure 50) and DNA consisting of the nucleotide sequence of SEQ ID No: 91 and containing the coding sequence of LH-PUM-H polypeptide 2 (see Figure 51), or DNA consisting of the nucleotide sequence of SEQ ID No: 92 and containing the coding sequence of HL-PUM-H polypeptide 1 (see Figure 52) and DNA consisting of the nucleotide sequence of SEQ ID No: 93 and containing the coding sequence of HL-PUM-H polypeptide 2 (see Figure 53) were outsourced to GenScript Japan Inc. and synthesized, to produce plasmids pLH-PUM-H (see Figure 54) and pHL-PUM-H (see Figure 55) according to the procedures shown in Figures 50 to 55. More specifically, plasmid pLH-PUM-H was a plasmid expressing LH-PUM-H polypeptide 1 in which SP50-L2 (the amino acid sequence of SEQ ID No: 13) and c-Myc-His fusion tag (the amino acid sequence of SEQ ID No: 11) were respectively added to the N-terminal side and the C-terminal side and LH-PUM-H polypeptide 2 in which SP52-L2 (i.e., the connecting peptide between secretion signal peptide SP52 [amino acid residues 1 to 35 of SEQ ID No: 18] and L2 linker peptide [amino acid residues 36 to 37 of SEQ ID No: 18]) and c-Myc-His fusion tag (the amino acid sequence of SEQ ID No: 11) were respectively added to the N-terminal side and the C-terminal side (see Figure 54). More specifically, plasmid pHL-PUM-H was a plasmid expressing HL-PUM-H polypeptide 1 in which SP50-L2 (the amino acid sequence of SEQ ID No: 13) and c-Myc-His fusion tag (the amino acid sequence of SEQ ID No: 11) were respectively added to the N-terminal side and the C-terminal side and HL-PUM-H polypeptide 2 in which SP52-L2 (i.e., the connecting peptide between secretion signal peptide SP52 [amino acid residues 1 to 35 of SEQ ID No: 18] and L2 linker peptide [amino acid residues 36 to 37 of SEQ ID No: 18]) and c-Myc-His fusion tag (the amino acid sequence of SEQ ID No: 11) were respectively added to the N-terminal side and the C-terminal side (see Figure 55). Table 7 shows the primer sets used for PCR.

**[Table 15]**

| |
|---|
| LH-PUM-H polypeptide 1 consisting of amino acid sequence of SEQ ID No: 86 |
| DIQMTQSPSSLSASVGDRVTITCRASQDIRNYLNWYQQKPGKAPKLLIYYTSRLESGVPSR FSGSGSGTDYTLTISSLQPEDFATYYCQQGNTLPWTFGQGTKVEIKAGGGGSQVQLVESG GGLVKPGESLRLSCAASGFTFSDYYMYWVRQAPGKGLEWVAIISDGGYYTYYSDIIKGRFT ISRDNAKNSLYLQMNSLKAEDTAVYYCARGFPLLRHGAMDYWGQGTLVTVSS |

The part surrounded by a square in the table indicates VL derived from anti-human CD3 antibody, the part surrounded by a double square indicates VH derived from anti-human PSMA antibody, and the peptide (AGGGGS) between the two indicates a linker peptide.

**[Table 16]**

| |
|---|
| LH-PUM-H polypeptide 2 consisting of amino acid sequence of SEQ ID No: 87 |
| DIQMTQSPSSLSASVGDRVTITCKASQNVDTNVAWYQQKPGQAPKSLIYSASYRYSDVPSRF SGSASGTDFTLTISSVQSEDFATYYCQQYDSYPYTFGGGTKLEIKAAGGGGSEVQLVESGGG LVQPGGSLRLSCAASGYSFTGYTMNWVRQAPGKGLEWVALINPYKGVSTYNQKFKDRFTISV DKSKNTAYLQMNSLRAEDTAVYYCARSGYYGDSDWYFDVWGQGTLVTVSS |

The part shown by an underline in the table indicates VL derived from anti-human PSMA antibody, the part shown by a double underline indicates VH derived from anti-human CD3 antibody, and the peptide (AAGGGGS) between the two indicates a linker peptide.

**[Table 17]**

| |
|---|
| HL-PUM-H polypeptide 1 consisting of amino acid sequence of SEQ ID No: 88 |
| EVQLVESGGGLVQPGGSLRLSCAASGYSFTGYTMNWVRQAPGKGLEWVALINPYKGVSTYN QKFKDRFTISVDKSKNTAYLQMNSLRAEDTAVYYCARSGYYGDSDWYFDVWGQGTLVTVSS AAGGGGSDIQMTQSPSSLSASVGDRVTITCKASQNVDTNVAWYQQKPGQAPKSLIYSASYR YSDVPSRFSGSASGTDFTLTISSVQSEDFATYYCQQYDSYPYTFGGGTKLEIK |

The part surrounded by a square in the table indicates VH derived from anti-human CD3 antibody, the part surrounded by a double square indicates was VL derived from anti-human PSMA antibody, and the peptide (AAGGGGS) between the two indicates a linker peptide.

**[Table 18]**

| |
|---|
| HL-PUM-H polypeptide 2 consisting of amino acid sequence of SEQ ID No: 89 |
| QVQLVESGGGLVKPGESLRLSCAASGFTFSDYYMYWVRQAPGKGLEWVAIISDGGYYTYYSD IIKGRFTISRDNAKNSLYLQMNSLKAEDTAVYYCARGFPLLRHGAMDYWGQGTLVTVSSAAG GGGSDIQMTQSPSSLSASVGDRVTITCRASQDIRNYLNWYQQKPGKAPKLLIYYTSRLESGV PSRFSGSGSGTDYTLTISSLQPEDFATYYCQQGNTLPWTFGQGTKVEIK |

The part shown by an underline in the table indicates VH derived from anti-human PSMA antibody, the part shown by a double underline indicates VL derived from anti-human CD3 antibody, and the peptide (AAGGGGS) between the two indicates a linker peptide.

### 27-2: Production of LH-PUM-H strain and HL-PUM-H strain

The transformation into *Bifidobacterium longum* 105-A strain using the plasmids pLH-PUM-H and pHL-PUM-H was performed by the same method as in Example 1, to obtain *Bifidobacterium longum* 105-A strain carrying the plasmids pLH-PUM-H and pHL-PUM-H (LH-PUM-H strain and HL-PUM-H strain).

### [Example 28] Evaluation (WB) of expression/secretion of LH-PUM-H strain and HL-PUM-H strain

In order to confirm that *Bifidobacterium* transformants (LH-PUM-H strain and HL-PUM-H strain) expressed and secreted the PSMA×CD3 diabody-type BsAbs, Western blotting method using the LH-PUM-H strain and HL-PUM-H strain culture supernatants was performed according to the method described in Example 2.

### (Result)

In the LH-PUM-H strain and HL-PUM-H strain culture supernatants, bands derived from the PSMA×CD3 diabody-type BsAbs were observed around 30 kDa (see Figure 56). These results indicate that *Bifidobacterium* transformants (LH-PUM-H strain and HL-PUM-H strain) expressed and secreted the PSMA×CD3 diabody-type BsAbs (i.e., LH-PUM-H diabody-type BsAb and HL-PUM-H diabody-type BsAb).

### [Example 29] Cytotoxic activity of diabody purified from LH-PUM-H strain and HL-PUM-H strain culture supernatants

The method was the same as in Example 4. However, human PSMA-positive 22Rv1 cells were used as the cells, and diabodies purified from the LH-PUM-H strain and HL-PUM-H strain culture supernatants (purified by the same method as in "3-1" of Example 3) serially diluted to a concentration of 1 pM to 100 nM were used as diabodies to be added to the cells. Figure 57 shows the results.

### (Result)

As shown in Figure 57, the diabodies purified from the LH-PUM-H strain and HL-PUM-H strain culture supernatants exhibited cytotoxic activity on 22Rv1 cells as a PSMA-positive human prostate cancer cell line, and the EC₅₀ values were 155.0 pM and 232.6 pM, respectively. These results indicate that the PSMA×CD3 diabody-type BsAbs secreted in the LH-PUM-H strain and HL-PUM-H strain culture supernatants have cytotoxic activity on cancer cells such as 22Rv1 cells.

### [Example 30] Evaluation of binding activity of DUP-143 strain-derived EGFR×CD3 diabody-type BsAb to human HER3 30-1: Purification of EGFR×CD3 diabody-type BsAb

The EGFR×CD3 diabody-type BsAb secreted by *Bifidobacterium* transformants (DUP-143 strain) was purified by the same method as in "7-1" of Example 7.

### 30-2: ELISA method

### 30-2-1: Reaction treatment between antigen and antibody

25 µL of a solution containing 1.0 µg/mL human HER3 recombinant protein (manufactured by Sino Biological) was dispensed into each well of a 96-well plate, followed by standing overnight at 4°C for immobilization. The solution after immobilization was removed, and each well was washed with 200 µL of PBS-T 3 times. Thereafter, 200 µL of a blocking solution (Blocking One, manufactured by NACALAI TESQUE, INC.) diluted 5-fold with distilled water (manufactured by Otsuka Pharmaceutical Co., Ltd.) was dispensed, followed by standing at 25°C for 2 hours for blocking treatment. The blocking solution in each well was removed, and the well was washed with 200 µL of PBS-T 3 times. A solution containing 0 (ng/mL), 0.1 (ng/mL), 1 (ng/mL), 10 (ng/mL), or 100 (ng/mL) EGFR×CD3 diabody-type BsAb was prepared with a signal enhancer HIKARI A solution (manufactured by NACALAI TESQUE, INC.) containing 1% BSA, and 25 µL of the solution was dispensed into each well after blocking. It was allowed to stand at 25°C for 2 hours for the reaction treatment between the EGFR×CD3 diabody-type BsAb and the immobilized human HER3 recombinant protein. Thereafter, the solution in each well was removed, and the well was washed with 200 µL of PBS-T 3 times.

### 30-2-2: Detection of antibody

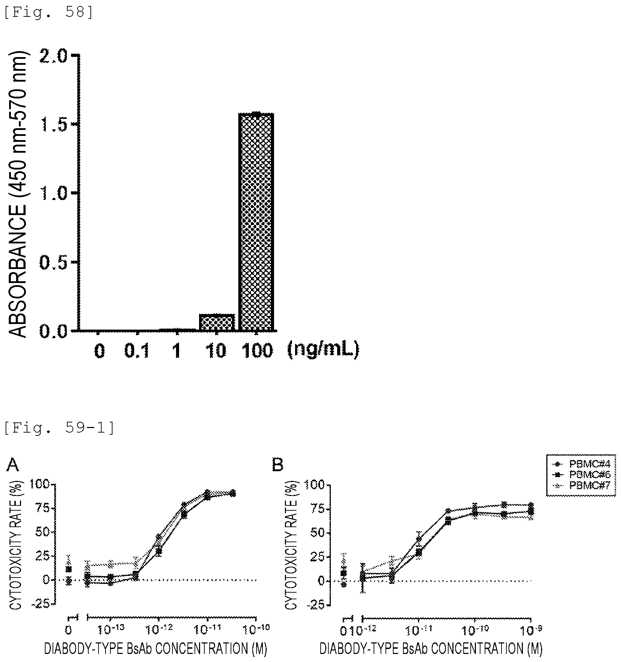

The recombinant biotinylated protein L (manufactured by Thermo Fisher SCIENTIFIC K.K.) was prepared into 0.05 µg/mL with a signal enhancer HIKARI B solution (manufactured by NACALAI TESQUE, INC.) containing 1% BSA, 25 µL thereof was dispensed into each well subjected to the reaction treatment between the antigen and the antibody, followed by standing at 25°C for 1 hour. The solution in each well was removed, and the well was washed with 200 µL of PBS-T 3 times. VECTASTAIN Elite ABC reagent (avidin-biotin-labeled HRP) (manufactured by Vector Laboratories) was adjusted into 4.0 µL/mL using a signal enhancer HIKARI B solution (manufactured by NACALAI TESQUE, INC.) containing 1% BSA, and 25 µL thereof was dispensed into each well, followed by standing at 25°C for 30 minutes. Thereafter, the solution in each well was removed, and the well was washed with 200 µL of PBS-T 3 times. After mixing equal amounts of Color Solution A and Color Solution B (solutions containing TMB as a substrate of HRP) (manufactured by R&D Systems, Inc.) as detection reagents, 50 µL thereof was dispensed into each well, and light was blocked, followed by standing at room temperature for 20 minutes. Thereafter, 25 µL of Stop Solution (manufactured by R&D Systems, Inc.) was added thereto, to stop the color reaction. The absorbance at 450 nm as the absorption wavelength of TMB-derived pigments was measured, and the value corrected with the absorbance at 570 nm (negative control) (average ± standard deviation, see the vertical axis of Figure 58) was calculated.

### (Result)

It was indicated that the corrected value of absorbance increased depending on the concentration of the DUM-143 strain-derived EGFR×CD3 diabody-type BsAb (see

Figure 58). This result indicates that the EGFR×CD3 diabody-type BsAb (i.e., LH31 diabody-type BsAb) secreted by the *Bifidobacterium* transformants (DUP-143 strain) also specifically binds to human HER3 in addition to human EGFR.

### [Example 31] Evaluation of cytotoxic activity of DUP-143 strain-derived EGFR×CD3 diabody-type BsAb

Using a total of 6 types of cancer cell lines having different EGFR expression levels and different HER3 expression levels, specifically, one human epidermoid cancer cell line (A-431), one human non-small cell lung cancer cell line (NCI-H1975), and four human colon cancer cell lines (HCT116, HT-29, RKO, and SW620), the cytotoxic activity of the DUP-143 strain-derived EGFR×CD3 diabody-type BsAb was evaluated.

31-1: Measurement of expression levels of EGFR and HER3 in various cancer cell lines

The expression levels of EGFR and HER3 in the 6 types of cancer cell lines were each quantified using an anti-human EGFR antibody (clone AY13) (manufactured by BioLegend, Inc.) and an anti-human erbB3/HER3 antibody (clone 1B4C3) (manufactured by BioLegend, Inc.), and QIFIKIT (manufactured by Agilent Technologies, Inc.) according to the protocol attached to the kit. BD FACSCantoII was used for measurement, and a flow cytometry analysis software (Kaluza Ver2.1) (manufactured by BECKMAN COULTER) was used for analysis.

### 31-2: Method for measuring cytotoxic activity

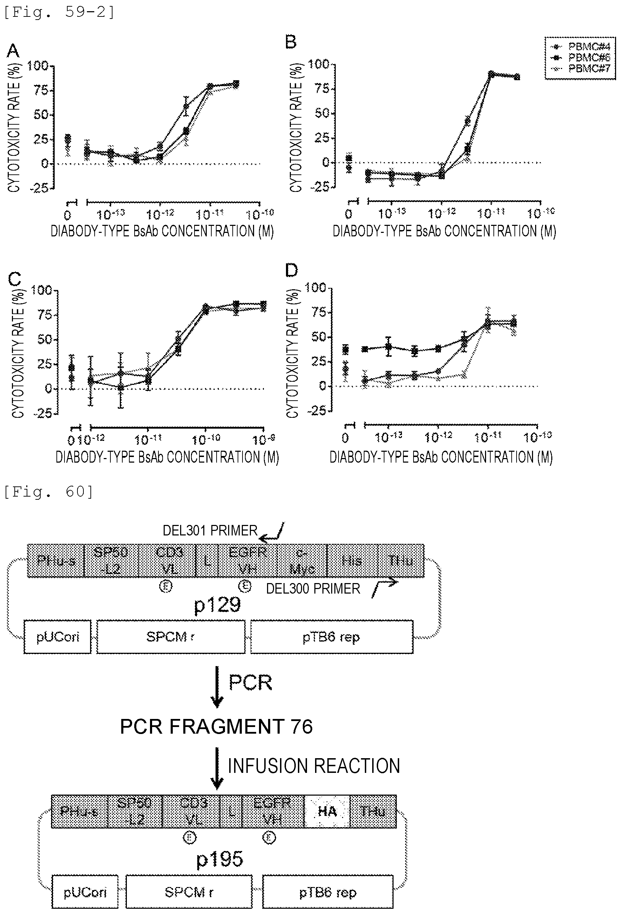

The one human epidermoid cancer cell line and the four human colon cancer cell lines were each seeded on a 96-well plate at 1 × 10⁴ cells/well, and the one human non-small cell lung cancer cell line was seeded on a 96-well plate at 5 × 10³ cells/well. After human PBMCs ("PBMC#4", "PBMC#6", and "PBMC#7" in Figure 59-1 and Figure 59-2) separated from 3 donors as effector cells were mixed at a ratio to each of the various cancer cell lines of 20:1, the DUP-143 strain-derived EGFR×CD3 diabody-type BsAb serially diluted to a concentration of 30 fM to 30 pM was added to each of A-431, HCT116, HT-29, and SW620 (the horizontal axes in Figure 59-1 and Figure 59-2), and the DUP-143 strain-derived EGFR×CD3 diabody-type BsAb serially diluted to a concentration of 1 pM to 1 nM was added to each of NCI-H1975 and RKO (the horizontal axes in Figure 59-1 and Figure 59-2), to give a total amount of the medium of 200 µL. Thereafter, they were cultured in an incubator set to 37°C and 5%CO₂, each well was washed 72 hours later with an RPMI-1640 medium 3 times, and 120 µL of an RPMI-1640 medium containing CellTiter 96 Aqueous One Solution Cell Proliferation Assay (manufactured by Promega) reagent was added to each well. The absorbance at 490 nm was measured, and the absorbance at 660 nm was measured as a control wavelength. The cytotoxic activity was calculated, taking the cytotoxic activity with only each cancer cell line in the well as 0% and the cytotoxic activity in the blank well as 100% (n = 3 wells). The test was conducted twice.

### (Result)

Table 19 below shows the expression levels of EGFR and HER3 in each cancer cell line.

**[Table 19]**

| Cell line | Origin | EGFR | HER3 |
|---|---|---|---|
| A-431 | Epidermal cancer | 703858 | 8622 |
| NCI-H1975 | Non-small cell lung cancer | 78228 | 5868 |
| HT-29 | Colon cancer | 30465 | 6311 |
| HCT116 | Colon cancer | 27772 | 2620 |
| RKO | Colon cancer | 4320 | <2000 |
| SW620 | Colon cancer | <2000 | 4030 |

### (Result)

While the expression of either or both of EGFR and HER3 was observed in all the 6 types of cancer cell lines, the expression levels of EGFR and HER3 in the human epidermoid cancer cell line (A-431) were highest, which were respectively 703858 (site/cells) and 8622 (site/cells) (see Table 19). As a result of measuring the cytotoxic activity of the DUP-143 strain-derived EGFR×CD3 diabody-type BsAb on the 6 types of cancer cell lines, it was indicated that the cell injury rates of the 6 types of cancer cell lines increased depending on the concentration of the DUP-143 strain-derived EGFR×CD3 diabody-type BsAb (see Figure 59-1 and Figure 59-2). These results indicate that the DUP-143 strain-derived EGFR×CD3 diabody-type BsAb has cytotoxic activity on the cancer cells expressing EGFR or HER3. The EC50 values of the DUP-143 strain-derived EGFR×CD3 diabody-type BsAb were 1.6 pM in A-431, 9.6 pM in NCI-H1975, 3.6 pM in HT-29, 4.2 pM in HCT116, 38.4 pM in RKO, and 3.2 pM in SW620.

### [Example 32] Production of Bifidobacterium (DUP-199HAF strain) expressing/secreting mutant LH31 diabody-type BsAb

Separately from the *Bifidobacterium* transformants produced in Example 12, a *Bifidobacterium* expressing/secreting mutant LH31 diabody-type BsAb was produced according to the methods described in sections [32-1] to [32-2] below.

### 32-1: Production of plasmid p199

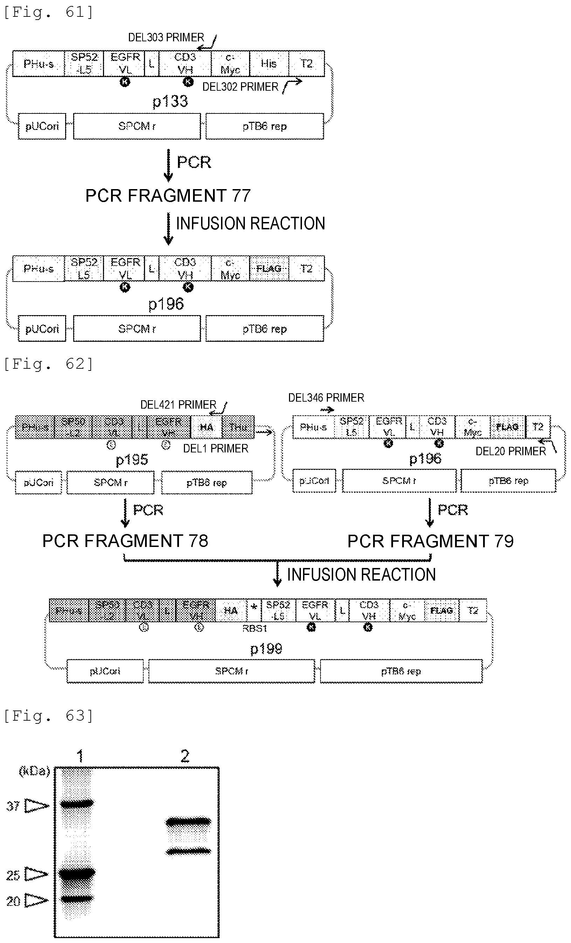

A plasmid (plasmid p199) expressing mutant LH31 polypeptide 1 in which HA tag (polypeptide consisting of the amino acid sequence of SEQ ID No: 102) was fused at the C-terminus and mutant LH31 polypeptide 2 in which c-Myc tag (polypeptide consisting of the amino acid sequence of SEQ ID No: 103) and FLAG tag (polypeptide consisting of the amino acid sequence of SEQ ID No: 104) were fused at the C-terminus (see Figure 62) was produced by the same method as in Example 1 according to the procedures shown in Figures 60 to 62. Table 7 shows the primer sets used for PCR.

### 32-2: Production of DUP-199HAF strain

The transformation into *Bifidobacterium longum* 105-A strain using the plasmid p199 was performed by the same method as in Example 1, to obtain *Bifidobacterium longum* 105-A strain carrying the plasmid p199 (DUP-199HAF strain).

### [Example 33] Evaluation (SDS-PAGE) of expression/secretion of DUP-199HAF strain

It was confirmed according to the methods described in sections "33-1" and "33-2" below that the *Bifidobacterium* transformants (DUP-199HAF strain) expressed and secreted the mutant LH31 diabody-type BsAb.

### 33-1: Purification of mutant LH31 diabody-type BsAb

The mutant LH31 diabody-type BsAb secreted by the *Bifidobacterium* transformants (DUP-199HAF strain) was purified from the DUP-199HAF strain culture supernatant according to the method described in section "7-1" of Example 7.

### 33-2: Evaluation (SDS-PAGE) of expression/secretion of mutant LH31 diabody-type BsAb

In order to confirm that the *Bifidobacterium* transformants (DUP-199HAF strain) expressed and secreted mutant LH31 diabody-type BsAb, SDS-PAGE using the solution purified from the DUP-199HAF strain culture supernatant was performed according to the method described in Example 15, followed by fluorescence staining.

### (Result)

In the DUP-199HAF strain culture supernatant, two bands derived from mutant LH31 polypeptide 1 and mutant LH31 polypeptide 2 constituting the mutant LH31 diabody-type BsAb were observed around 30 kDa (see Figure 63). These results indicate that the DUP-199HAF strain expressed and secreted the mutant LH31 diabody-type BsAb.

### [Example 34] Tissue distribution of Bifidobacterium and mutant LH31 diabody-type BsAb by administration of DUP-199HAF strain to mouse tail vein

In order to confirm the tissue distribution of *Bifidobacterium* by administration of DUP-199HAF strain to mouse tail vein and the transition of the concentration of the mutant LH31 diabody-type BsAb in the tumor and the blood, the DUP-199HAF strain was intravenously administered to cancer-bearing SCID mice with HCT116 cells as a human large intestine cancer cell line implanted, and the number of viable bacteria of the DUP-199HAF strain in each tissue and the concentration of the EGFR×CD3 diabody-type BsAb secreted from the DUP-199HAF strain in the tumor and the blood plasma were analyzed over time.

### 34-1: Administration of DUP-199HAF strain to cancer-bearing mice

HCT116 cells cultured in a McCoy's5A medium (manufactured by Sigma-Aldrich) containing 10% FBS (manufactured by Equitech-Bio Inc.) were subcutaneously implanted into 8 week-old female SCID mice (manufactured by Japan SLC, Inc.), to produce cancer-bearing mice. A simple frozen formulation of DUP-199HAF strain was administered into the tail veins of the cancer-bearing mice with a tumor size of 101.54 to 251.60 mm³ at a dose of 5.0 × 10⁸ cfu/0.2 mL. On day 1, day 3, day 5, day 7, day 10, day 12, day 14, day 17, day 19, and day 21 after the administration, tumor and non-tumor tissues (brain, heart, lung, kidney, spleen, and liver) were extracted each from 5 of the cancer-bearing mice. Further, the blood plasma was fractionated by collecting and centrifuging the blood. 1 mL of a 10% maltose solution was intraperitoneally administered at a frequency of twice a day from day 0 (date of administration) to the day before the extraction with 5 administrations and 2 rests.

### 34-2: Measurement of the number of viable bacteria of DUP-199HAF strain

An anaerobic diluent containing a protease inhibitor (manufactured by NACALAI TESQUE, INC.) was added to each of the tumor and non-tumor tissues (brain, heart, lung, kidney, spleen, and liver), followed by homogenization, to prepare a tissue homogenate. The tissue homogenate prepared and the blood appropriately diluted with the anaerobic diluent were applied to a BLFS agar medium (BL agar medium containing 250 µg/mL 5-fluorouracil and 30 µg/mL spectinomycin), followed by culture at 37°C under anaerobic conditions for 3 days. The number of colonies formed on the BLFS agar medium was counted, to calculate the number of viable bacteria of the DUP-199HAF strain (see Figure 64). A part of the tissue homogenate was collected and used for the method described in section "34-3" below.

### 34-3: Preparation of tumor sample for ELISA analysis

To the tumor homogenate prepared in "34-2" above, were added 10 × Cell Lysis buffer (500 mM Tris-HCl [pH 7.5], 1500 mM sodium chloride, 5% NP-40, and 20 mM EDTA) and 50 × cOmplete (prepared by dissolving one tablet of cOmplete [EDTA-free, manufactured by Roche Diagnostics K.K., Cat#11873580001] in 1 mL of ultrapure water), followed by stirring, and then the supernatant after centrifugation was collected, to prepare a tumor sample for ELISA analysis.

### 34-4: ELISA method

25 µL of each solution containing 5 µg/mL (for blood plasma) or 2 µg/mL (for tumor) human CD3E/CD3D heterodimer recombinant protein (manufactured by ACROBiosystems) was dispensed into each well of a 96-well plate, followed by standing overnight at 4°C for immobilization. Each well was washed with PBS-T, followed by blocking treatment at 25°C for 2 hours with a blocking solution (Blocking One, manufactured by NACALAI TESQUE, INC.) diluted 5-fold with distilled water (Otsuka Pharmaceutical Co., Ltd.). Each well was washed with PBS-T, and the standard EGFR×CD3 diabody-type BsAb for calibration curve (mutant LH31 diabody-type BsAb) purified from the DUP-199HAF strain culture supernatant and serially diluted from 4 ng/mL to 0.01 ng/mL (for blood plasma) and from 64 ng/mL to 0.015625 ng/mL (for tumor) respectively in the tumor sample for ELISA analysis prepared in "34-3" above and in the blood plasma prepared in "34-1" above was incubated at 25°C for 2 hours. Thereafter, each well was washed with PBS-T, followed by incubation at 25°C for 1 hour in the presence of anti-HA biotin (manufactured by Roche Diagnostics K.K.). Each well was washed with PBS-T, followed by incubation at 25°C for 30 minutes in the presence of VECTASTAIN Elite ABC reagent (avidin-biotin-labeled HRP) (manufactured by Vector Laboratories). Each well was washed with PBS-T, color was developed at room temperature for 20 minutes in the presence of Color Solution A and Color Solution B (solutions containing TMB as a substrate of HRP) (manufactured by R&D Systems, Inc.) as detection reagents, and the color reaction was stopped with a Stop Solution (2N sulfuric acid) (manufactured by R&D Systems, Inc.). The absorbance at 450 nm as the absorption wavelength of TMB-derived pigments and the absorbance at 570 nm as a negative control were measured using POWERSCAN HT (Gen5 2.04) (manufactured by DS Pharma Biomedical Co., Ltd.), to plot a calibration curve based on the standard EGFR×CD3 diabody-type BsAb for calibration curve. Thereafter, the concentrations of the EGFR×CD3 diabody-type BsAb in the tumor and the blood plasma were quantified (see Figure 65).

### (Result)

When the DUP-199HAF strain was administered to the HCT116 cancer-bearing SCID mice, the number of viable bacteria of the DUP-199HAF strain gradually increased in the tumor, to reach the maximum (central value: 4.8 × 10⁷ cfu/g of tumor) on day 5 after the administration, and the number of viable bacteria (central value) observed was 10⁶ cfu/g of tumor or more until day 19 after the administration (see Figure 64). Meanwhile, although viable bacteria of the DUP-199HAF strain were detected in some of the non-tumor tissues (spleen, liver, kidney, and heart) on day 1 after the administration, the detection level considerably decreased thereafter, and it was less than the detection limit from day 5 after the administration (see Figure 64). These results indicate that *Bifidobacterium* transformants (DUP-199HAF strain) specifically engraft in the tumor.

Further, the concentration of the DUP-199HAF strain-derived EGFR×CD3 diabody-type BsAb in the tumor reached the maximum (an average: 34588.4 pg/g of tumor) on day 5 after the administration of the DUP-199HAF strain, and the EGFR×CD3 diabody-type BsAb was observed in the tumor at the quantitation limit (2500 pg/g of tumor) or more as an average until day 17 after the administration (see Figure 65). These results indicate that the *Bifidobacterium* transformants (DUP-199HAF strain) that had engrafted in the tumor expressed/secreted the EGFR×CD3 diabody-type BsAb. The concentration of the DUP-199HAF strain-derived EGFR×CD3 diabody-type BsAb in the blood plasma was less than the quantitation limit (250 pg/mL) as an average at any time after the administration.

### [Example 35] In-vivo anti-tumor effect by DUP-143 strain

In order to confirm the in-vivo anti-tumor effect by the DUP-143 strain, HCT116 cells as a human large intestine cancer cell line were implanted, and in-vivo analysis was conducted using severe immunodeficient mice (NOG mice) with human PBMCs transplanted. Specifically, analysis was performed according to the method described below.

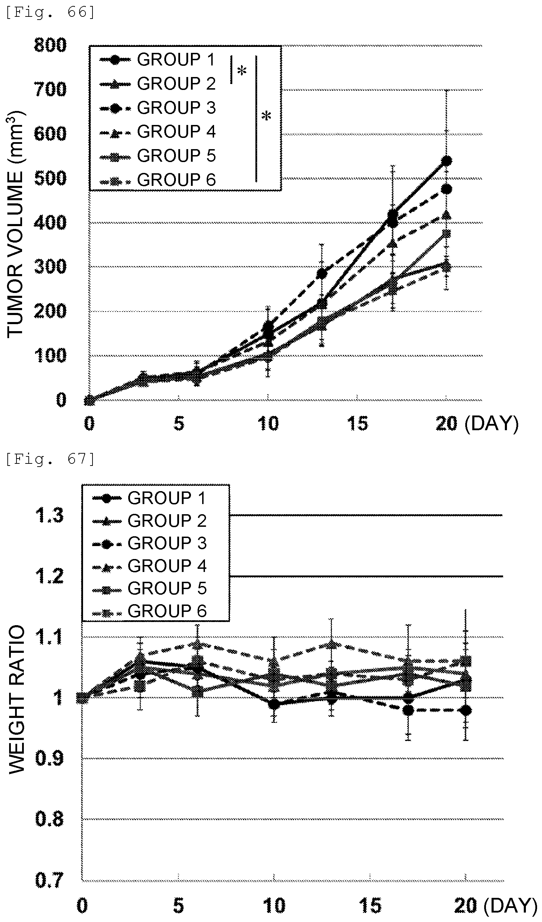

HCT116 cells were cultured in a McCoy's5A medium (manufactured by Sigma-Aldrich) containing 10% FBS (manufactured by Equitech-Bio Inc.) and subcutaneously implanted into 7 week-old female NOG mice (manufactured by In-Vivo Science Inc.), to produce cancer-bearing mice. On day 3 and day 9 after the implantation, PBMCs were intraperitoneally transplanted, and a simple frozen formulation of the DUP-143 strain or a frozen formulation of the BE shuttle strain was administered into the tail veins at 5.0 × 10⁸ cfu/0.2 mL from day 3 to day 18 after the implantation (see Figure 66). The configuration of each group (groups 1 to 6) was as follows.
Group 1: The DUP-143 strain was administered with no PBMCs transplanted (on day 7, day 10, day 14, and day 17 after the implantation).
Group 2: The DUP-143 strain was administered (on day 7, day 10, day 14, and day 17 after the implantation) with PBMCs transplanted (on day 3 after the implantation).
Group 3: The BE shuttle strain was administered (on day 7, day 10, day 14, and day 17 after the implantation) with PBMCs transplanted (on day 3 and day 9 after the implantation).
Group 4: The DUP-143 strain was administered (on day 7, day 10, day 14, and day 17 after the implantation) with PBMCs transplanted (on day 3 and day 9 after the implantation).
Group 5: The DUP-143 strain was administered (on day 3, day 6, day 10, day 13, and day 17 after the implantation) with PBMCs transplanted (on day 3 and day 9 after the implantation).
Group 6: The DUP-143 strain was administered (on day 7, day 9, day 11, day 14, day 16, and day 18 after the implantation) with PBMCs transplanted (on day 3 and day 9 after the implantation).

After the administration of the frozen formulation, 1 mL of a 10% maltose solution was intraperitoneally administered twice a day with 5 administrations and 2 rests. On day 3, day 6, day 10, day 13, day 17, and day 20 after the administration, the body weight and the tumor diameter in the mice of each group were measured. The tumor volume was calculated based on the formula "tumor volume = major axis × minor axis²/2" (see Figure 66). The tumor growth inhibition rate [TGI] was calculated based on the formula "TGI = [1 - average tumor volume on day 20 after the implantation (groups 2 to 6)/average tumor volume on day 20 after the implantation (group 1)] × 100". The body weight ratio was calculated based on the formula "body weight ratio = body weight at each time/body weight on date of implantation" (see Figure 67).

### (Result)

As a result of analyzing the tumor volume in the mice of each group, the tumor volume in the mice of the BE shuttle strain-administered (on day 7, day 10, day 14, and day 17 after the implantation) group with PBMCs transplanted (on day 3 and day 9 after the implantation) (group 3) was almost the same as the tumor volume in the mice of the DUP-143 strain-administered (on day 7, day 10, day 14, and day 17 after the implantation) group with no PBMCs transplanted (group 1) as a negative control (see Figure 66). Meanwhile, the tumor volume in the mice of the DUP-143 strain-administered (on day 7, day 10, day 14, and day 17 after the implantation) group with PBMCs transplanted (on day 3 after the implantation) (group 2) and the tumor volume in the mice of the DUP-143 strain-administered (on day 7, day 9, day 11, day 14, day 16, and day 18 after the implantation) group with PBMCs transplanted (on day 3 and day 9 after the implantation) (group 6) significantly decreased as compared with the tumor volume in the mice of group 1 as a negative control, and the tumor growth inhibition rates [TGI] to that of the mice of group 1 as the negative control were respectively 43.0% and 45.0% on day 20 after the implantation, for example (see Figure 66). Meanwhile, no weight loss was observed in any group (see Figure 67) . These results indicate that the EGFR×CD3 diabody-type BsAb secreted by the *Bifidobacterium* transformants (DUP-143 strain) has an anti-tumor effect.

### Industrial Applicability

The present invention contributes to prevention or treatment of solid tumors (generally, solid cancers).

## Claims

1. A bacterium of the genus *Bifidobacterium* expressing:
a first polypeptide comprising a light-chain variable region (VL) that binds to human CD3 and a heavy-chain variable region (VH) that binds to a human tumor cell surface antigen; and
a second polypeptide comprising VL that binds to a human tumor cell surface antigen and VH that binds to human CD3,
and secreting a diabody-type bispecific antibody composed of the first polypeptide and the second polypeptide.

2. The bacterium of the genus *Bifidobacterium* according to claim 1, wherein the first polypeptide comprises the VL that binds to human CD3 and the VH that binds to the human tumor cell surface antigen in this order from the amino terminus to the carboxyl terminus, and the second polypeptide comprises the VL that binds to the human tumor cell surface antigen and the VH that binds to human CD3 in this order from the amino terminus to the carboxyl terminus.

3. The bacterium of the genus *Bifidobacterium* according to claim 1 or 2, wherein a linker peptide each consisting of 4 to 9 amino acids is connected between the VL and the VH of the first polypeptide and between the VL and the VH of the second polypeptide, respectively.

4. The bacterium of the genus *Bifidobacterium* according to any one of claims 1 to 3, wherein a secretion signal peptide-linker peptide conjugate containing any of amino acid sequences (i) to (iii) below is connected to the amino terminus of each of the first polypeptide and the second polypeptide:
(i) an amino acid sequence having a sequence identity of at least 80% with the amino acid sequence shown in SEQ ID No: 10;
(ii) an amino acid sequence having a sequence identity of at least 80% with the amino acid sequence shown in SEQ ID No: 13; and
(iii) an amino acid sequence having a sequence identity of at least 80% with the amino acid sequence shown in SEQ ID No: 18.

5. The bacterium of the genus *Bifidobacterium* according to any one of claims 1 to 4, wherein
the amino acid residue at Kabat position 38 in the VL of the first polypeptide is glutamic acid, the amino acid residue at Kabat position 39 in the VH of the first polypeptide is glutamic acid, the amino acid residue at Kabat position 38 in the VL of the second polypeptide is lysine, and the amino acid residue at Kabat position 39 in the VH of the second polypeptide is lysine; or
the amino acid residue at Kabat position 38 in the VL of the first polypeptide is lysine, the amino acid residue at Kabat position 39 in the VH of the first polypeptide is lysine, the amino acid residue at Kabat position 38 in the VL of the second polypeptide is glutamic acid, and the amino acid residue at Kabat position 39 in the VH of the second polypeptide is glutamic acid.

6. The bacterium of the genus *Bifidobacterium* according to any one of claims 1 to 5, wherein the human tumor cell surface antigen is an antigen belonging to a human EGFR (Epidermal Growth Factor Receptor) family, a human EpCAM (Epithelial cell adhesion molecule), or a human PSMA (Prostate Specific Membrane Antigen).

7. The bacterium of the genus *Bifidobacterium* according to any one of claims 1 to 6, wherein the antigen belonging to a human EGFR family is a human EGFR, a human HER2, or a human HER3.

8. The bacterium of the genus *Bifidobacterium* according to any one of claims 1 to 7, wherein the first polypeptide and the second polypeptide are expressed polycistronically.

9. The bacterium of the genus *Bifidobacterium* according to any one of claims 1 to 8, wherein the bacterium is *Bifidobacterium longum.*

10. An agent for preventing or treating solid tumors, comprising the bacterium of the genus *Bifidobacterium* according to any one of claims 1 to 9 as an active component.

11. The agent according to claim 10, wherein the agent is used in combination with an agent for promoting engraftment and/or growth of a bacterium of the genus *Bifidobacterium* in solid tumors.

12. The agent according to claim 11, wherein the agent for promoting engraftment and/or growth of a bacterium of the genus *Bifidobacterium* in solid tumors is maltose.
